# EUROPEAN PATENT APPLICATION

(11) **EP 4 317 184 A1**
(43) Date of publication of application: **07.02.2024**
(21) Application number: 22791107.0
(22) Date of filing: 21.04.2022
(51) Int. Cl.: C07K 16/18, C07K 16/46, A61K 38/17

(54) **BISPECIFIC MULTIFUNCTIONAL FUSION POLYPEPTIDE**

(30) Priority: 22.04.2021 CN 202110436970; 30.07.2021 CN 202110871320; 24.09.2021 CN 202111121937; 10.03.2022 CN 202210240917
(71) Applicant: Guangdong Fapon Biopharma Inc., Dongguan, Guangdong 523000 (CN)
(72) Inventor: LU, Di, Dongguan, Guangdong 523000 (CN); HUO, Yongting, Dongguan, Guangdong 523000 (CN); LU, Lisheng, Dongguan, Guangdong 523000 (CN)
(74) Representative: Berggren Oy
(86) International application number: PCT/CN2022/088198
(87) International publication number: WO 2022/223001

(57) **Abstract**

The present application relates to the technical field of biomedicines, and particularly to a bispecific fusion polypeptide. The bispecific fusion polypeptide comprises an antigen-binding moiety. The antigen-binding moiety comprises a first antigen-binding moiety comprising a first polypeptide comprising a first heavy chain variable domain VH1 of a first antibody from the N terminus to the C terminus, which is operably linked to a first conjugate fragment; and a second polypeptide comprising a first light chain variable domain VL1 of the first antibody from the N terminus to the C terminus, which is operably linked to a second conjugate fragment, wherein the first conjugate fragment and the second conjugate fragment are capable of specifically binding; and the first conjugate fragment is a receptor and the second conjugate fragment is a ligand; or the first conjugate fragment is a ligand and the second conjugate fragment is a receptor.

## Description

### Cross-Reference to Related Application

The present disclosure claims the priority of a total of 4 Chinese patent applications with Application No. "202110436970.6", entitled "antibody or functional fragment thereof" filed on April 22, 2021, Application No. "202110871320.4", entitled "antibody or functional fragment thereof" filed on July 30, 2021, Application No. "202111121937.0", entitled "multifunctional bispecific fusion polypeptide" filed on September 24, 2021, and Application No. "202210240917.3", entitled "multifunctional bispecific fusion polypeptide" filed on March 10, 2022 submitted to China Patent Office, and the disclosure of which is incorporated by reference herein in its entirety.

### Technical Field

The present disclosure relates to the technical field of biomedicines, and particularly to a bispecific fusion polypeptide and/or multifunctional fusion polypeptide comprising a ligand and a receptor.

### Background

In order to broaden clinical efficacies of protein drugs, global biotechnology science researchers are struggling to develop biomacromolecule drugs with different structures and types. Bispecific antibodies (BsAb) are currently the most clinically popular novel biomacromolecule drug structures. The BsAb are antibodies may bind to two different antigens or different epitopes of an antigen simultaneously, and may exert biological functions that cannot be achieved by monoclonal antibodies through a specific mode of action.

With the progress of recombinant protein expression and genetic engineering techniques, the forms of the BsAb are increasingly diversified. Until now, more than 20 forms of BsAb have been developed into a technical platform.

During an assembly of IgG-type bispecific antibodies, 10 possible combinations of two natural heavy chains and two natural light chains may be randomly generated, of which only one is a target bispecific antibody product. The biochemical characteristics of 10 different bispecific antibody products are similar. It is extremely difficult to separate out the target bispecific antibody, such that the yield and the purity of the target bispecific antibody are low, the cost is increased, and the efficacy is affected.

A core value of a bispecific antibody technical platform is to solve problems of mismatching of heavy chains and mismatching of light chain and heavy chain. Main technical platforms for solving the mismatching of heavy chains are: Knob-into-Holes (KiH), ART-Ig, a strand-exchange engineered domain (SEED) technology, and XmAb.

The main technical platforms for solving the mismatching of heavy chain and light chain are:
1. Common light chain technology: the technology was developed by Genentech in 2006. Antibodies found from a phage-display screening directed against multiple antigens often share the same VL domain, reflecting a very limited size of a light chain library in a phage library. The use of a common light chain strategy simplifies an antibody engineering and a purification process in an industrial production. Advantages: a problem of LC/HC and HC/HC mismatching is solved. Disadvantages: it is very difficult to find VL commonly shared by antibodies in a phage-display screening, Patent Application WO2008027236.
2. CrossMab technology: the technology was developed by Roche in 2007 and solves a light chain problem by exchanging heavy chain and light chain domain sequences of a Fab fragment, which is generally achieved in three ways: exchanging VH and VL, exchanging CH1 and CL or exchanging VH and VL and CH1 and CL. Advantages: a problem of mismatching of light and heavy chain may be solved by combining a KiH structure, and affinities of original antigens are reserved. Disadvantages: the expression level is relatively low, Patent Application WO2009080251.

The technologies for solving a problem of mismatching of heavy chains are relatively mature. The technologies for solving a problem of mismatching of light chain and heavy chain still need to be improved. Therefore, the present disclosure is specifically provided.

### Summary

The present disclosure aims to solve one technical problem existing in the related art to some extent.

The inventor provides a concept for developing a novel bispecific antibody. The mismatching of heavy chain and light chain is avoided or reduced by replacing CH1 and CL in the antibody or a functional fragment thereof with a specific affinity for a ligand and a receptor; and further, the replacement may be simultaneously or independently selected from CH2, CH3, and optionally CH4, thereby facilitating formation of a heavy chain heterodimer. In another aspect, the bispecific antibody provided in the present disclosure is a multifunctional fusion protein that may exert a dual-target specificity and also exert a conductive biological activity of a ligand and a receptor.

In one aspect, the present disclosure provides a bispecific fusion polypeptide, comprising a first antigen-binding moiety which comprises:
a first polypeptide comprising a first heavy chain variable domain VH1 of a first antibody from the N terminus to the C terminus, which is operably linked to a first conjugate fragment; and
a second polypeptide comprising a first light chain variable domain VL1 of the first antibody from the N terminus to the C terminus, which is operably linked to a second conjugate fragment, wherein,
the first conjugate fragment and the second conjugate fragment are capable of specifically binding; and
the first conjugate fragment is a receptor and the second conjugate fragment is a ligand; or the first conjugate fragment is a ligand and the second conjugate fragment is a receptor.
In some embodiments, the bispecific fusion polypeptide further comprises a second antigen-binding moiety, wherein the second antigen-binding moiety is different from the first antigen-binding moiety, and
the second antigen-binding moiety comprises:
   a third polypeptide comprising a second heavy chain variable domain VH2 of a second antibody from the N terminus to the C terminus, which is operably linked to a third conjugate fragment; and
   a fourth polypeptide comprising a second light chain variable domain VL2 of the second antibody from the N terminus to the C terminus, which is operably linked to a fourth conjugate fragment, wherein,
   the third conjugate fragment and the fourth conjugate fragment are capable of specifically binding; the third conjugate fragment is a receptor and the fourth conjugate fragment is a ligand; or the third conjugate fragment is a ligand and the fourth conjugate fragment is a receptor; and
   the third conjugate fragment and/or the fourth conjugate fragment, and the first conjugate fragment and/or the second conjugate fragment are selected from different receptors and ligands.
   In some embodiments, the bispecific fusion polypeptide further comprises a second antigen-binding moiety, wherein the second antigen-binding moiety is different from the first antigen-binding moiety, and
   the second antigen-binding moiety comprises:
      a third polypeptide comprising a second heavy chain variable domain VH2 of a second antibody from the N terminus to the C terminus, which is operably linked to an antibody heavy chain constant region CH1; and
      a fourth polypeptide comprising a second light chain variable domain VL2 of the second antibody from the N terminus to the C terminus, which is operably linked to an antibody light chain constant region CL.

In some embodiments, the receptor only comprises an active site that recognizes and binds to a ligand but does not comprise a functionally active site that generates a response reaction.

In some embodiments, at least one non-natural interchain bond is comprised between the receptor and the ligand, and the non-natural interchain bond is capable of enhancing a specific binding force between the receptor and the ligand. In some embodiments, the non-natural interchain bond is formed between a first mutant residue comprised by the receptor and a second mutant residue comprised by the ligand. In some embodiments, at least one of the first mutant residue and the second mutant residue is a cysteine residue. In some embodiments, the non-natural interchain bond is a disulfide bond.

In some embodiments, at least one natural glycosylation site is not present in the receptor and/or the ligand.

In some embodiments, the receptor and the ligand thereof are selected from an interleukin and a receptor thereof.

In some embodiments, the interleukin and the receptor thereof have a lift-type steric conformantion and are selected from IL15/IL15R, IL2/IL2R, IL4/IL-4Rα+Rγ, IL-6/IL-6R, IL-11/IL-11R, IL-13/IL-13R1, IL-20/IL20Rα+IL20Rβ, and/or IL24/IL20Rα+IL20Rβ.

In some embodiments, the interleukin and the receptor thereof have a pincer-type steric conformantion and are selected from IL7/IL7R, IL21/IL21R, and IL23A/IL12B. In some embodiments, the ligand and the receptor are selected from IL15 and IL15Rα.

In some embodiments, the E at position 90 of the IL15 is mutated into C and the P at position 67 of the IL15Rα is mutated into C.

In some embodiments, a non-natural disulfide bond is present between the first heavy chain variable domain VH1 and the first light chain variable domain VL1, and preferably, the first heavy chain variable domain VH1 and the first light chain variable domain VL1 comprise any one of the following mutation combinations:

| Combination | VH | VL |
|---|---|---|
| 1 | 37C | 95C |
| 2 | 44C | 100C |
| 3 | 44C | 101C |
| 4 | 44C | 105C |
| 5 | 45C | 87C |
| 6 | 45C | 98C |
| 7 | 100C | 50C |
| 8 | 100bC | 49C |
| 9 | 98C | 46C |
| 10 | 101C | 46C |
| 11 | 105C | 43C |
| 12 | 106C | 57C |
| 13 | 108C | 43C |

In some embodiments, the D at position 61 of the IL15 is mutated into N, the E at position 64 is mutated into Q, and/or the N at position 65 is mutated into D.

In some embodiments, at least one N-glycosylation site of the IL15 is not present; preferably, the N-glycosylation site is selected from N71, N79, and/or N112; and preferably, the IL15 comprises the following amino acid mutations: N71Q, N79Q, and/or N112Q.

In some embodiments, at least one O-glycosylation site of the IL15Rα is not present; preferably, the O-glycosylation site is selected from T2, T81, and/or T86; and preferably, the IL15Rα comprises the following amino acid mutations: T2A, T81A, and/or T86A.

In some embodiments, the ligand and the receptor are selected from IL2 and IL2Rα.

In some embodiments, the S at position 75 of the IL2 is mutated into C and the N terminus of the IL2Rα is extended by two or three amino acids.

Preferably, the N terminus of the IL2Rα is extended by two amino acids, the second extended amino acid is cysteine, and the first extended amino acid is any one of a non-polar fatty acid amino acid, an aromatic amino acid, an amino acid with an uncharged R group, an amino acid with a positively charged R group or an amino acid with a negatively charged R group.

Preferably, the N terminus of the IL2Rα is extended by three amino acids, the second extended amino acid is cysteine, and the first and third extended amino acids are any one of a non-polar fatty acid amino acid, an aromatic amino acid, an amino acid with an uncharged R group, an amino acid with a positively charged R group or an amino acid with a negatively charged R group.

In some embodiments, the bispecific fusion polypeptide comprises an antibody Fc constant region. In some embodiments, the antibody Fc constant region is a heterodimer. In some embodiments, the antibody Fc constant region is the heterodimer associated by KiH, a hydrophobic interaction, an electrostatic interaction, a hydrophilic interaction, and/or increased flexibility. In some embodiments, the antibody Fc constant region comprises CH2, CH3, and optionally CH4, and the CH2, the CH3, and/or the optionally CH4 are replaced by the receptor and the ligand thereof.

In some embodiments, the first antigen-binding moiety and the second antigen-binding moiety bind to different antigens or bind to different epitopes of the same antigen. In some embodiments, the first antigen-binding moiety targets an immune cell and the second antigen-binding moiety targets a tumor cell. In some embodiments, the first antigen-binding moiety and the second antigen-binding moiety both target a tumor cell. In some embodiments, the first antigen-binding moiety and the second antigen-binding moiety both target an immune cell. In some embodiments, the first antigen-binding moiety and the second antigen-binding moiety bind to link a T cell and a tumor antigen. In some embodiments, the first antigen-binding moiety and the second antigen-binding moiety bind to link an NK cell and a tumor antigen. In some embodiments, the first antigen-binding moiety and the second antigen-binding moiety bind to synergistically inhibit a signaling pathway. In some embodiments, the first antigen-binding moiety and the second antigen-binding moiety bind to form a protein complex.

The present disclosure further relates to an isolated nucleic acid, encoding the bispecific fusion polypeptide.

The present disclosure further relates a vector containing the nucleic acid.

The present disclosure further relates to a host cell containing the nucleic acid or the vector.

The present invention further relates to a method for preparing a bispecific fusion polypeptide, comprising:
transforming a host cell with the vector;
culturing the transformed host cell; and
collecting the bispecific fusion polypeptide expressed in the host cell.

The present disclosure further relates to a pharmaceutical composition, comprising the bispecific fusion polypeptide, a pharmaceutically acceptable carrier, an excipient, or a stabilizer.

The present disclosure further relates to use of the bispecific fusion polypeptide in preparing a drug for treating a disease.

### Brief Description of the Drawings

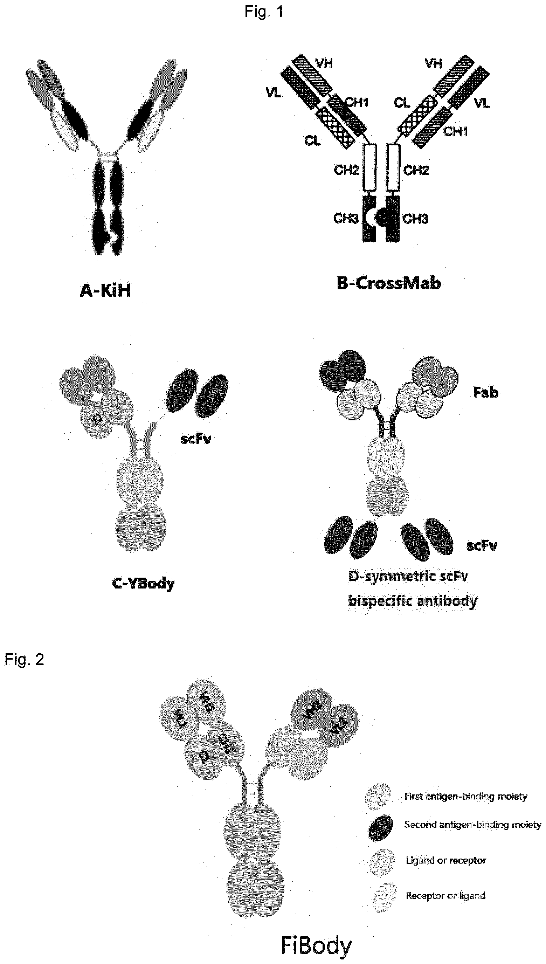

FIG. 1 shows 4 classical bispecific antibody platforms: FIG. 1A is a KiH heterodimeric Fc modification technology; FIG. 1B is a CrossMab bispecific antibody technology; FIG. 1C is a YBody bispecific antibody technology of Wuhan YZY Med (asymmetric scFv bispecific antibody); and FIG. 1D is a symmetric scFv bispecific antibody;
FIG. 2 is a novel bispecific antibody FiBody provided by the present disclosure, wherein CH1 and CL of one side Fab are replaced by a ligand and a receptor with a specific affinity;

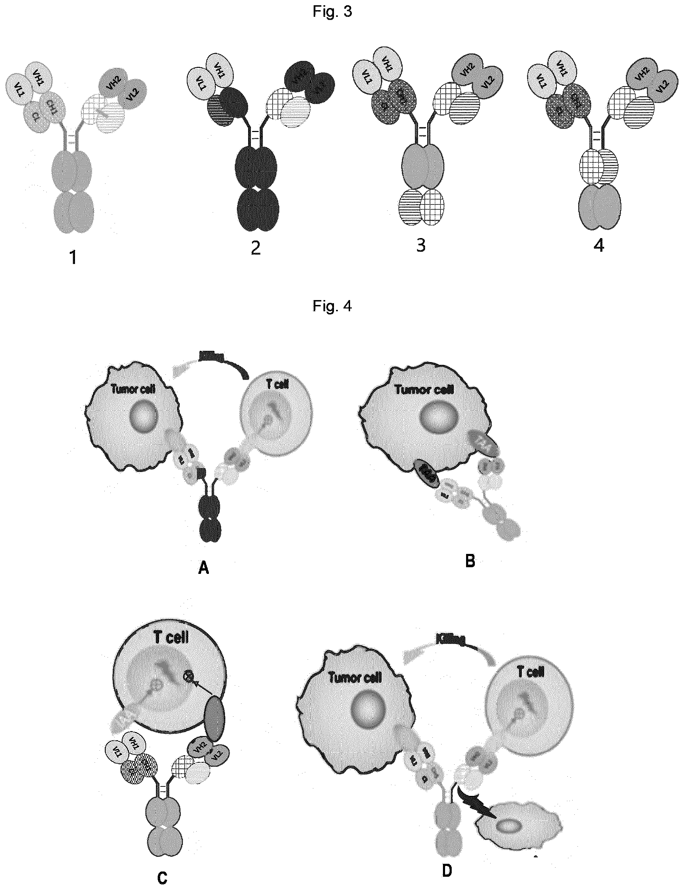

FIG. 3 exemplarily shows 4 feasible solutions of FiBody: FIG. 3-1 shows a modified ligand and receptor having a non-naturally occurring interchain bond between the ligand and the receptor; FIG. 3-2 shows that CH1 and CL of both Fabs are replaced by a receptor and a ligand, and both sides are selected from different ligands and receptors; FIG. 3-3 shows that in addition to that CH1 and CL of an Fab on one side of the antibody are replaced by a ligand and a receptor, a CH3 fragment in an Fc dimer is also replaced by a ligand and a receptor; and FIG. 3-4 shows that in addition to that CH1 and CL of an Fab on one side are replaced by a ligand and a receptor, CH2 in the Fc dimer is also replaced by a ligand and a receptor; and other feasible modification modes are various;
FIG. 4 shows an example that when the bispecific antibody of the present disclosure is used to treat a tumor, a targeted binding of an antigen-binding moiety of the bispecific antibody comprises 3 exemplary types: FIG. 4-A shows that a first antigen-binding moiety targets a T cell and a second antigen-binding moiety targets a tumor cell; FIG. 4-B shows both a first antigen-binding moiety and a second antigen-binding moiety target a tumor cell; FIG. 4-C shows both a first antigen-binding moiety and a second antigen-binding moiety target a T cell; and FIG. 4-D exemplarily shows that the bispecific antibody of the present disclosure may optionally be a trifunctional fusion protein, is also capable of activating a ligand and receptor pathway and eliciting a biological activity of a ligand and a receptor in addition to exerting binding of different antigens;
FIG. 5 shows steric conformantions of interleukins and receptors thereof, which may be classified into four types: A: lift type, B: bowknot type, C: ballplayer type, and D: pincer type;
FIG. 6 is an example of four steric conformantions of interleukins and receptors thereof, wherein A lift type is IL2/IL2R, B bowknot type is IL22/IL22R, C ballplayer type is IL18/IL18R, and D pincer type is IL21/IL21R;
FIG. 7 is a FiBody design based on IL15 (ligand) and IL15RA (receptor) in the example of the present disclosure, wherein a second antigen-binding region VH is linked to IL15RA and a second antigen-binding region VL is linked to IL15;
FIG. 8 is a structural schematic diagram of modification and optimization of a disulfide bond;
FIG. 9 is a three-dimensional structural schematic diagram of an interaction of IL15/IL15RA with an IL2/15Rβ/γC complex;
FIG. 10 shows a structural schematic diagram of a mismatched molecule R1042/R1124 in the example of the present disclosure;
FIG. 11 shows an HPLC-SEC detection result of a sample R0951 in the example of the present disclosure;
FIG. 12 shows an HPLC-SEC detection result of a sample R1042 in the example of the present disclosure;
FIG. 13 shows an HPLC-SEC detection result of a sample R0809 in the example of the present disclosure;
FIG. 14 shows an HPLC-SEC detection result of a sample R1110 in the example of the present disclosure;
FIG. 15 shows an HPLC-SEC detection result of a sample R1262 in the example of the present disclosure;
FIG. 16 shows binding activities of TIGIT ends of bispecific antibodies (R0950, R0951, R0952, R0954, R0955, and R0960) and CHO-Tigit cells detected by an FCM method in the example of the present disclosure;
FIG. 17 shows binding activities of TIGIT ends of bispecific antibodies (R1123/R1119/R1120/R1124) and CHO-Tigit cells detected by an FCM method in the example of the present disclosure;
FIG. 18 shows binding activities of TIGIT ends of bispecific antibodies (R1042/R1043) and CHO-Tigit cells detected by an FCM method in the example of the present disclosure;
FIG. 19 shows a binding activity of a TIGIT ends of a bispecific antibody (R0810) and CHO-Tigit cells detected by an FCM method in the example of the present disclosure;
FIG. 20 shows a binding activity of a TIGIT end of a bispecific antibody (R1262) and CHO-Tigit cells detected by an FCM method in the example of the present disclosure, wherein hlgG1 is a subtype control antibody;
FIG. 21 shows binding activities of PD-L1 ends of bispecific antibodies (R0950, R0951, R0952, R0954, R0955, and R0960) and CHO-PD-L1 cells detected by an FCM method in the example of the present disclosure;
FIG. 22 shows binding activities of PD-L1 ends of bispecific antibodies (R1072, R1115-R1120, and R1123-R1124) and CHO-PD-L1 cells detected by an FCM method in the example of the present disclosure;
FIG. 23 shows binding activities of PD-L1 ends of bispecific antibodies (R0950, R1042, and R1043) and CHO-PD-L1 cells detected by an FCM method in the example of the present disclosure;
FIG. 24 shows binding activities of PD-L1 ends of bispecific antibodies (R1072 and R1081-R1086) and CHO-PD-L1 cells detected by an FCM method in the example of the present disclosure;
FIG. 25 shows binding activities of PD-L1 ends of bispecific antibodies (R1072 and R1109-R1111) and CHO-PD-L1 cells detected by an FCM method in the example of the present disclosure;
FIG. 26 shows a binding activity of a PD-L1 end of a bispecific antibody (R1262) and CHO-PD-L1 cells detected by an FCM method in the example of the present disclosure;
FIG. 27 shows a detection result of blocking of R1262 on a binding force between a ligand and a target region in the example of the present disclosure;
FIG. 28 shows binding activities of sample receptor and ligand complexes (IL15/IL15R) (R0950, R0951, R0952, R0954, R0955, and R0960) in the example of the present disclosure;
FIG. 29 shows binding activities of samples (R1042 and R1043) receptor and ligand complexes (IL15/IL15R) in the example of the present disclosure;
FIG. 30 shows gel electrophoresis detection results of disulfide bond-modified optimized samples (R1072, R1081, R1082, R0954, and R1084-R1086) in the example of the present disclosure;
FIG. 31 shows gel electrophoresis detection results of FiBody samples prepared in example 9 of the present disclosure (disulfide bond-modified IL2/IL2Rα complexes 1 and 2, and an IL2/IL2Rα complex 3 constructed correspondingly in example 8) and R1115 in example 2, wherein the complexes 1 and 2 are disulfide bond-modified IL2/IL2Rα complexes, the complex 3 is the prior reported IL2/IL2Rα complex, and a complex 4 is a disulfide bond-unmodified IL2/IL2Rα complex (sample R1115);
FIG. 32 shows gel electrophoresis detection results of purified disulfide bond-modified IL2/IL2Rα complexes 5, 6, 7, and 8 and a complex 1 prepared in example 14 of the present disclosure, wherein a disulfide bond-unmodified IL2/IL2Rα complex 4 is used as a control; and
FIG. 33 shows gel electrophoresis detection results of purified disulfide bond-modified IL2/IL2Rα complexes 9, 10, 11, 12, and 13 and the complex 1 prepared in example 14 of the present disclosure, wherein the disulfide bond-unmodified IL2/IL2Rα complex 4 is used as a control.

### Detailed Description of the Embodiments

A reference will now be made in detail to embodiments of the present disclosure, wherein one or more examples of which are described below. Each example is provided by an explanation, not a limitation, of the present disclosure. Actually, it would have been obvious to a person skilled in the art to make various changes and modifications to the present disclosure without departing from the scope or spirit of the present disclosure. For example, features illustrated or described as a part of one embodiment, may be used on another embodiment to produce a still further embodiment. All references, including publications, patents, and patent applications, cited in the present disclosure are incorporated herein by reference in their entirety.

Unless otherwise defined, all technical and scientific terms used herein have the same meaning as commonly understood by a person skilled in the technical field of the present disclosure. The terms used in the description of the present disclosure herein are only for the purpose of describing specific examples and are not intended to limit the present disclosure. As used herein, the term "and/or" includes any and all combinations of one or more related items listed.

### Terms:

The term "antigen-binding moiety" or "antigen-binding domain" means a portion of an antigen-binding molecule that confers a binding specificity to an antigenic determinant. In some embodiments, the "antigen-binding moiety" is an antibody functional fragment.

The term "amino acid" means one of 20 naturally occurring amino acids encoded by DNA and RNA.

Single-letter abbreviations of non-polar fatty acid amino acids are glycine (G), alanine (a), valine (V), leucine (L), isoleucine (I), and methionine (M) respectively; single-letter abbreviations of aromatic amino acids are phenylalanine (F), tryptophan (W), and tyrosine (Y) respectively; single-letter abbreviations of amino acids with an uncharged R group are serine (S), threonine (T), cysteine (C), proline (P), aspartic acid (N), and glutamine (Q) respectively; single-letter abbreviations of amino acids with a positively charged R group are lysine (K), arginine (R), and histidine (H) respectively; and single-letter abbreviations of amino acids with a negatively charged R group are aspartic acid (D) and glutamic acid (E).

The term "wild-type or WT" means an amino acid sequence or a nucleotide sequence found in nature, including an allelic variation. A WT protein has an amino acid sequence or a nucleotide sequence that has not been intentionally modified.

The term "antibody" encompasses any immunoglobulin, monoclonal antibody, polyclonal antibody, polyspecific antibody, bispecific (bivalent) antibody, or bispecific fusion polypeptide that may bind to a specific antigen. A natural and intact antibody comprises two heavy chains and two light chains. Each heavy chain consists of a variable region ("HCVR") and first, second, and third constant regions (CH1, CH2, and CH3 respectively), and each light chain consists of a variable region ("LCVR") and a constant region (CL). Mammalian heavy chains may be divided into α, δ, ε, γ, and µ, and mammalian light chains into λ or κ.

The antibody is "Y" shaped, and a backbone consists of the second (CH2), the third (CH3), and optionally the fourth constant regions (CH4) of the two heavy chains, which are bound by disulfide bonds. Each arm of the "Y" structure comprises a variable region (VH) and a first constant region (CH1) of one of the heavy chains, which bind to a variable region (VL) and a constant region (CL) of one of the light chains. The variable regions of the light and heavy chains are responsible for antigen binding. The variable region of each chain contains three hypervariable regions, wherein complementarity determining regions (CDRs) of the light (L) chain include LCDR1, LCDR2, and LCDR3, and the CDRs of the heavy (H) chain include HCDR1, HCDR2, and HCDR3. The three CDRs are separated by flanking continuous portions called framework regions (FRs) that are more highly conserved than the CDRs and form a scaffold-supported hypervariable loop. The HCVR and the LCVR each comprise 4 FRs. The CDRs and the FRs are arranged from an amino terminus to a carboxy terminus in the following order: FR1, CDR1, FR2, CDR2, FR3, CDR3, and FR4.

The constant regions of the heavy chain and the light chain are not involved in antigen binding, but have multiple effector functions. Antibodies may be divided into several kinds depending on an amino acid sequence of a heavy chain constant region. Depending on whether containing α, δ, ε, γ, and µ heavy chains, the antibodies may be divided into five major classes or isoforms respectively: IgA, IgD, IgE, IgG, and IgM. Several main antibody classes may further be divided into subclasses, such as IgG1(γ1 heavy chain), IgG2(γ2 heavy chain), IgG3(γ3 heavy chain), IgG4(y4 heavy chain), IgA1(α1 heavy chain), or IgA2 (α2 heavy chain).

The hypervariable regions typically comprise about 24-34 (LCDR1; "L" represents the light chain), 50-56 (LCDR2), and 89-97 (LCDR3) amino acid residues in the light chain variable region, and about 31-35 (HCDR1; "H" represents the heavy chain), 50-65 (HCDR2), and 95-102 (HCDR3) amino acid residues in the heavy chain variable region; Kabat et al., SEQUENCES OF PROTEINS OF IMMUNOLOGICAL INTEREST), 5th edition, Public Health Service, National Institutes of Health, Bethesda, Md. (1991) and/or those residues that form hyper-mutated loops (e.g., residues 26-32 (LCDR1), 50-52 (LCDR2), and 91-96 (LCDR3) in the light chain variable region, and 26-32 (HCDR1), 53-55 (HCDR2), and 96-101 (HCDR3) in the heavy chain variable region); and Chothia and Lesk (1987), J. Mol. Biol., 196: 901-917).

In some embodiments, the antibody is a bispecific antibody (BiAb). The term "bispecific" herein refers to two different antigens, or even when both are the same antigen, each of which has a binding specificity directed to different epitopes. The epitopes may be derived from different antigens or the same antigen. The terms "bispecific fusion polypeptide" and "bispecific antibody" herein refer to all prepared products having a full-length antibody or a fragment with an antigen-binding site. The antibody may be a human antibody, a non-human antibody (e.g., a murine antibody), a humanized antibody, or a chimeric antibody (e.g., a human-mouse chimeric antibody or a chimera of different subtypes of antibodies). In some cases, a variant of the antibody is a conservative modification or a conservative replacement or substitution on a sequence of the antibody provided herein. The "conservative modification" or the "conservative replacement or substitution" refer to the replacement of an amino acid in a protein with other amino acids having similar characteristics (e.g., charge, side chain size, hydrophobicity/hydrophilicity, backbone conformation, rigidity, etc.), such that changes may be made frequently without changing a biological activity of the protein. It is known to a person skilled in the art that, in general, a single amino acid replacement in a non-essential region of a polypeptide does not substantially change a biological activity (see, for example, Watson et al. (1987), Molecular Biology of the Gene, The Benjamin/Cummings Pub. Co., page 224, (the fourth edition)). In addition, replacement of structurally or functionally similar amino acids is unlikely to destroy a biological activity. A person skilled in the art would have been able to determine suitable variants of the antigen-binding molecules set forth herein with well-known technologies. With regard to nucleotide and amino acid sequences, the term "identity" indicates a degree of identity between two nucleic acid or two amino acid sequences when optimally aligned and compared with appropriate insertions or deletions.

The term "Fab" is a Fab fragment not containing or containing a small portion of a residual Fc fragment in an immunoglobulin, e.g., the Fab fragment comprises variable regions of a heavy chain and a light chain, and a first constant domain of all or a portion. For simplicity, the term "Fab" hereinafter may also refer to a fragment such as F(ab)2.

The term "Fc" or "Fc region" or "Fc domain" means the constant region of an antibody, in some cases excluding all or a portion of a first constant region immunoglobulin domain (e.g., CH1) or all or a portion of a portion thereof, and in some cases further excluding all or a portion of a hinge. Therefore, Fc may refer to all or a portion of the last two constant region immunoglobulin domains (e.g., CH2 and CH3) of IgA, IgD, and IgG, the last three constant region immunoglobulin domains of IgE and IgM, and optionally a flexible hinge N terminus of these domains. With regard to IgA and IgM, the Fc may comprise a J chain. With regard to IgG, the Fc domain comprises the immunoglobulin domains CH2 and CH3 (Cy2 and Cγ3) and a lower hinge region located between CH1 (Cγ1) and CH2 (Cy2). Although a boundary of the Fc region may vary, the Fc region of a human IgG heavy chain is generally defined to include residues E216, C226, or A231 of its carboxy terminus, wherein numbering is performed according to an EU index in Kabat. In some embodiments, as described more comprehensively below, the Fc region is subjected to an amino acid modification, e.g., the Fc is a heterodimer.

The "modification" herein refers to an amino acid substitution, insertion and/or deletion in a polypeptide sequence or an alteration in a moiety chemically linked to a protein. The term "amino acid modification" herein refers to an amino acid substitution, insertion and/or deletion in a polypeptide sequence. For clarity, unless otherwise indicated, the amino acid modification is always amino acids encoded by DNA, e.g., 20 amino acids with codons in DNA and RNA.

The "epitope" means herein a determinant that interacts with a specific antigen-binding domain, e.g., a variable region (referred to as a paratope) of an antibody molecule. The epitope is a group of molecules, such as amino acids or sugar side chains, and generally has specific structural characteristics and specific charge characteristics. A single molecule may have more than one epitope. The epitope may comprise amino acid residues directly involved in binding (also referred to as an immunodominant component of the epitope) and other amino acid residues not directly involved in binding, such as amino acid residues effectively blocked by a specific antigen-binding peptide. In other words, the amino acid residues are within a cover area of the specific antigen-binding peptide. The epitope may be either conformational or linear. The conformational epitope is produced by a spatial juxtaposition of amino acids from different segments of a linear polypeptide chain. The linear epitope may be produced by adjacent amino acid residues in a polypeptide chain. The conformational and non-conformational epitopes may be distinguished by a loss of binding to the former, but not the latter, in the presence of a denaturing solvent. The epitope typically comprises at least 3, and more typically at least 5 or 8-10 amino acids in a unique spatial conformation. An antigen-binding molecule that recognizes the same epitope may be verified in a simple immunoassay, demonstrating an ability of one antigen-binding molecule to block binding of another antigen-binding molecule to a target antigen. As outlined below, the present disclosure comprises antigen-binding molecules and antigen-binding domains listed herein, and further comprises antigen-binding molecules and antigen-binding domains that compete for binding with the epitopes to which the listed antigen-binding molecules or antigen-binding domains bind.

The terms "specific binding", "selective binding", "selectively binds to", and "specifically binds to" refer to a biological binding process that a directed ligand that may be competitively blocked by a corresponding substance interacts with a specific structural site *in vitro* or *in vivo,* for example, binding between an antigen and an antibody or between a receptor and a ligand.

A strength or an affinity of specific binding may be expressed according to a dissociation constant (KD) of an interaction, wherein a smaller KD represents a greater affinity and a greater KD represents a lower affinity, such as the KD is expressed by an antigen-binding force of at least about 10⁻⁴ M, at least about 10⁻⁵ M, at least about 10⁻⁶ M, at least about 10⁻⁷ M, at least about 10⁻⁸ M, at least about 10⁻⁹ M, at least about ¹0-¹⁰ M, at least about 10⁻¹¹ M, at least about 10⁻¹² M, or greater. A binding characteristic may be determined by methods well-known in the art, such as a biolayer interferometry and a method based on a surface plasmon resonance. In one such method, an association and dissociation rate of an antigen-binding site/an antigen or a receptor/ligand complex needs to be measured, wherein the rate depends on a concentration of a complex partner, an affinity of an interaction, and a geometric parameter that affects the rate equally in both directions. Therefore, the association rate (ka) and the dissociation rate (KD) may be determined, and a ratio of kd/ka is equal to the dissociation constant KD (Nature, 361:186-187 (1993) and Davies et al. (1990), Annual RevBiochem, 59:439-473).

The term "immune cell" comprises a cell that is involved in an immune system of protecting the body against both infectious diseases and foreign substances. The immune cells may include, for example, neutrophils, eosinophils, basophils, lymphocytes, such as B cells, T cells, and monocytes. The T cells may include, for example, CD4+, CD8+, T helper cells, cytotoxic T cells, γδT cells, regulatory T cells, suppressor T cells, and natural killer cells.

The term "multifunctional fusion polypeptide" means a non-naturally occurring binding molecule designed to target two or more antigens. The "multifunctional fusion polypeptide" described herein is typically a genetically engineered fusion protein designed to bring two different desired biological functions into a single binding molecule. For example, the multifunctional fusion polypeptide may be a multifunctional binding molecule.

The term "FiBody" is a bispecific fusion polypeptide or a multifunctional fusion protein obtained by substituting a portion or all of a constant region of a bispecific antibody with a specific affinity of a ligand and a receptor thereof.

The "YBody" technology mentioned in the present disclosure was developed by Wuhan YZY Med in 2012. The technology is that based on the "Knob-into-Holes" technology, one of a formed heterodimer is a normal heavy chain, the other is an N terminal of an Fc functional region linked to scFv, and an asymmetric bispecific antibody is formed.

The term "about" or "approximately" refers to quantification, level, value, quantity, frequency, percentage, dimension, size, amount, weight, or length that is 30%, 25%, 20%, 25%, 10%, 9%, 8%, 7%, 6%, 5%, 4%, 3%, 2%, or 1% different from reference quantification, level, value, quantity, frequency, percentage, dimension, size, amount, weight, or length. In particular embodiments, when the term "about" or "approximately" precedes a numerical value, it indicates to a range of the value plus or minus 15%, 10%, 5%, or 1%.

Unless otherwise specified in the context, the words "include", "comprise", and "contain" will be understood to mean comprising the stated step or element or a group of steps or elements but not excluding any other step or element or a group of steps or elements. The "consisting of" means including and being limited to what is followed by the phrase "consisting of". Therefore, the phrase "consisting of" means that the listed elements are required or necessary and that no other elements may be present. The "essentially consisting of" means including any elements listed thereafter, and being limited to other elements that contribute to or do not interfere with activities or functions of the listed elements as detailed in the present disclosure. Therefore, the phrase "essentially consisting of" means that the listed elements are required or necessary, but other elements are optionally and may be present or not present depending on whether they affect activities or functions of the listed elements.

"One embodiment", "embodiment", "specific embodiment", "related embodiment", "certain embodiment", "another embodiment" or "further embodiment" or a combination thereof mentioned in the whole text of the present disclosure mean that the described specific feature, structure, or characteristic related in the embodiment are included in at least one embodiment of the present disclosure. Therefore, the foregoing phrases appearing in various places throughout the description are not necessarily all referring to the same embodiment. Furthermore, the specific feature, structure, or characteristics may be combined in any suitable manner in one or more embodiments.

The term "optionally" is used for a descriptive purpose only and not to be construed as indicating or implying a relative importance. Therefore, a feature defined as "optionally" may explicitly or implicitly include or exclude the feature.

The terms "first" and "second" in the description and in the claims are used for distinguishing similar elements and not necessarily for describing a sequence or a time order. It should be understood that such used terms are interchangeable under an appropriate circumstance and that the embodiments of the present disclosure described herein are capable of being operated in other sequences different from the description or illustration herein.

### Bispecific fusion polypeptide

The present disclosure provides a novel bispecific fusion polypeptide comprising a ligand (or a fragment thereof) and a receptor thereof (or a fragment thereof). The ligand (or the fragment thereof) and the receptor thereof (or the fragment thereof) independently replace CH1 and CL of Fab on one side of an antibody respectively. Specifically, the bispecific fusion polypeptide comprises a first antigen-binding moiety. The first antigen-binding moiety comprises: a first polypeptide comprising a first heavy chain variable domain VH1 of a first antibody from the N terminus to the C terminus, which is operably linked to a first conjugate fragment; and
a second polypeptide comprising a first light chain variable domain VL1 of the first antibody from the N terminus to the C terminus, which is operably linked to a second conjugate fragment, wherein
the first conjugate fragment is a receptor and the second conjugate fragment is a ligand; or the first conjugate fragment is a ligand and the second conjugate fragment is a receptor.

The bispecific fusion polypeptide further comprises a second antigen-binding moiety which is different from the first antigen-binding moiety. A polypeptide fusion method for the second antigen-binding moiety is optionally selected from:
1. CH1 and CL of Fab on the other side of the antibody are replaced by another ligand (or a fragment thereof) and a receptor thereof (or a fragment thereof), i.e.,
   the second antigen-binding moiety comprises: a third polypeptide comprising a second heavy chain variable domain VH2 of a second antibody from the N terminus to the C terminus, which is operably linked to a third conjugate fragment; and
   a fourth polypeptide comprising a second light chain variable domain VL2 of the second antibody from the N terminus to the C terminus, which is operably linked to a fourth conjugate fragment, wherein,
   the third conjugate fragment is a receptor and the fourth conjugate fragment is a ligand; or the third conjugate fragment is a ligand and the fourth conjugate fragment is a receptor; and
   the third conjugate fragment and/or the fourth conjugate fragment, and the first conjugate fragment and/or the second conjugate fragment are selected from different receptors and ligands.
2. Original CH1 and CL of Fab on the other side are retained, i.e.,
   the second antigen-binding moiety comprises a third polypeptide comprising a second heavy chain variable domain VH2 of a second antibody from the N terminus to the C terminus, which is operably linked to an antibody heavy chain constant region CH1; and
   a fourth polypeptide comprising a second light chain variable domain VL2 of the second antibody from the N terminus to the C terminus, which is operably linked to an antibody light chain constant region CL.

The present disclosure uses the unique specific binding force of a ligand and a receptor thereof, and creatively operably links the ligand and the receptor thereof to the antigen-binding region (antibody variable region). The linking comprises linking to one antigen-binding region, and the other antigen-binding region is still linked to CH1 and CL; or both antigen-binding regions are linked to the ligand and the receptor, and only the two antigen-binding regions are linked to different ligands and receptors, thereby avoiding mismatching of different antigen-binding regions.

In some embodiments, the bispecific fusion polypeptide provided by the present disclosure is a multifunctional fusion polypeptide comprising an antibody Fab. The Fab comprises two different antigen-binding moieties, a first antigen-binding moiety and a second antigen-binding moiety. CH1 and CL of one side of the Fab are independently substituted with a ligand and a receptor thereof, and CH1 and CL of the other side of the Fab are unsubstituted, and the receptor comprises both an active site that recognizes and binds to the ligand and a functionally active site that generates a response reaction; and the light chain of the first antigen-binding moiety is not mismatched to the heavy chain of the second antigen-binding moiety. In some embodiments, CH1 and CL of the other side of the Fab are independently substituted with a second ligand and a receptor thereof, and the first ligand and a receptor thereof are different from the second ligand and a receptor thereof.

The multifunctional fusion protein may exert a dual-target specificity and also exert a conductive biological activity of a ligand and a receptor. For example, in a specific embodiment, the ligand and the receptor thereof are IL15 and IL15Rα. The multifunctional fusion polypeptide has a dual-target targeting effect, and the IL15Rα may further present the IL-15 to an IL-2/15Rβγ dimer to form a ternary complex, activate JAK and STAT signaling pathways, promote proliferation and activation of target cells, increase secretion levels of IFN-γ and TNF-α; JAK/STAT and Ras/MAPK enhance proliferation signals; and up-regulation of Bcl-2 and Bcl-XL (anti-apoptotic proteins), and down-regulation of Bim and Puma (pro-apoptotic proteins) attenuate apoptotic signals.

### i. Ligand and receptor

The "receptor" is a substance capable of recognizing and binding to a bioactive molecule on a cell membrane or in a cell, and a bioactive substance capable of binding to the receptor is collectively called "ligand".

The receptors are classified into two classes, cell surface receptors and intracellular receptors, according to their location in the cells. The receptor itself contains at least two active sites: one is an active site that recognizes and binds to a ligand; and the other is a functionally active site responsible for generating a response reaction, this site can only generate a response reaction after binding to a ligand to form a binary complex and be allosteric, thereby initiating a series of biochemical reactions that ultimately lead to a biological effect of a target cell.

The receptors are typically glycoproteins. Binding between the wild-type receptors and the ligands is not mediated by covalent bonds, but rather is primarily through ionic bonds, hydrogen bonds, Van der Waals forces, and hydrophobic interactions. When the receptors bind to the ligands, the receptors have the characteristics of saturation, high affinity, specificity, etc.

The receptors and ligands, in coordination with each other, have an affinity for relative specific binding, and an optional biological effect. In some embodiments, the receptor only comprises an active site that recognizes and binds to a ligand but does not comprise a functionally active site that generates a response reaction. (e.g., function that activates a biological effect of a downstream signaling pathway). In some embodiments, the receptor and/or ligand is a natural receptor-ligand structure. The receptor comprises both an active site for recognizing a binding ligand and a functionally active site responsible for generating a response and may exert a corresponding biological function. The bispecific fusion protein is a multifunctional fusion protein, has a dual specificity, and also may exert functions of a ligand and a receptor.

In some embodiments, the receptor and/or ligand is modified from a natural sequence. The modification includes but is not limited to: truncation, insertion and/or mutation. The purposes of these modifications include but are not limited to: increasing or decreasing a binding force of a ligand and a receptor; enhancing, reducing or eliminating biological functions of the ligand and the receptor; increasing, decreasing or eliminating glycosylation sites in receptor and or ligand proteins; and reducing or eliminating toxicity of a receptor and a ligand.

In some embodiments, an amino acid sequence of the receptor and/or the ligand each independently consists of 10-1,000 amino acids. In some embodiments, the amino acid sequence of the receptor and/or the ligand each independently consists of 20-800 amino acids. In some embodiments, the amino acid sequence of the receptor and/or the ligand each independently consists of 30-600 amino acids. In some embodiments, the amino acid sequence of the receptor and/or the ligand each independently consists of 40-400 amino acids. In some embodiments, the amino acid sequence of the receptor and/or the ligand each independently consists of 50-300 amino acids. In some embodiments, the amino acid sequence of the receptor and/or the ligand each independently consists of 55-260 amino acids. For example, the amino acid sequence of the receptor and/or the ligand may also be independently selected from 20, 30, 40, 50, 60, 70, 80, 90, 100, 150, 200, 300, 400, 500, 600, 700, 800, and 900 amino acids.

In some embodiments, the molecular weight of the receptor and/or the ligand is each independently selected from 1 KD-100 KD. In some embodiments, the molecular weight of the receptor and/or the ligand is each independently selected from 2 KD-80 KD. In some embodiments, the molecular weight of the receptor and/or the ligand is each independently selected from 3 KD-70 KD. In some embodiments, the molecular weight of the receptor and/or the ligand is each independently selected from 4 KD-60 KD. In some embodiments, the molecular weight of the receptor and/or the ligand is each independently selected from 4 KD-50 KD. In some embodiments, the molecular weight of the receptor and/or the ligand is each independently selected from 4 KD-40 KD. In some embodiments, the molecular weight of the receptor and/or the ligand is each independently selected from 5 KD-30 KD. For example, the molecular weight of the receptor and/or the ligand may be independently selected from 1 KD, 2 KD, 3 KD, 4 KD, 4.5 KD, 5 KD, 6 KD, 7 KD, 8 KD, 9 KD, 10 KD, 11 KD, 15 KD, 18 KD, 20 KD, 25 KD, 30 KD, 35 KD, 40 KD, 45 KD, 50 KD, 60 KD, 70 KD, 80 KD, 90 KD, and 100 KD.

A binding mode of the receptor (or a fragment thereof) and a corresponding ligand thereof (or a fragment thereof) may be covalent binding, non-covalent interaction, or a combination thereof. Examples of non-covalent bonds include, but are not limited to, hydrogen bonds, hydrophobic bonds, ionic bonds, and Van der Waals bonds. In some embodiments, the ligand and/or the receptor of an antibody may be modified to increase an affinity when the affinity between inserted or replaced conjugate fragments is lower than expected (e.g., two variable regions in an antigen-binding moiety cannot be brought closer to function to specifically recognize an antigen, or a heavy chain mismatching between 2 or more heavy chain constant regions cannot be prevented, or a mismatching between the antigen-binding moieties cannot be prevented to realize a specific VL-VH portion combination). In some embodiments, at least one non-natural interchain bond is comprised between the receptor and the ligand, and the non-natural interchain bond is capable of enhancing the specific binding force between the receptor and the ligand. In some embodiments, the non-natural interchain bond is formed between a first mutant residue comprised by the receptor and a second mutant residue comprised by the ligand. In some embodiments, at least one of the first mutant residue and the second mutant residue is a cysteine residue. In some embodiments, the non-natural interchain bond is a disulfide bond.

In some embodiments, at least one natural glycosylation site is not present in the receptor and/or the ligand.

In some embodiments, the receptor and the ligand thereof are selected from an interleukin and a receptor thereof.

The inventor studies steric conformantions of a large number of interleukins and receptors thereof, and finds that the steric conformantions of the interleukins or IFN molecules may be classified into four types: A-lift type, B-bowknot type, C-ballplayer type, and D-pincer type as shown in Table 3:

**Table 3**

| Name of interleukin | type | Ligand | Ligand size | Receptor | Receptor size |
|---|---|---|---|---|---|
| Interleukin1α | | IL-1α | 160a.a. | IL-1R | 310a.a. |
| Interleukin1β | C | IL-1β | 155a.a. | IL1R1+IL1R2 | 310a.a. |
| Interleukin 2 | A | IL-2 | 133a.a. | IL-2Rα+IL2Rβ+Rγ | 220 a.a. |
| Interleukin 3 | C | IL-3 | 132a.a. | IL-3R | 286a.a. |
| Interleukin 4 | A | IL-4 | 128 a.a. | IL-4Rα+Rγ | 206a.a |
| Interleukin 5 | D | IL-5 | 115a.a. | IL-5R | 321a.a. |
| Interleukin 6 | A | IL-6 | 182a.a. | IL-6R | 345a.a. |
| Interleukin 7 | D | IL-7 | 152a.a. | IL-7R | 218a.a. |
| Interleukin 8 | | IL-8/CXCL8 | 77a.a. | IL-8RA/ IL-8RB | 350a.a./ 360a.a. |
| Interleukin 9 | | IL-9 | 136a.a. | IL-9R | 230a.a. |
| Interleukin 10 | B | IL-10 | 160a.a. | IL-10R1 | 224a.a. |
| Interleukin 11 | A | IL-11 | 178a.a. | IL-11R | 346a.a. |
| Interleukin 12 | | IL-12α/p35 | 197a.a. | IL-12β | 306a.a. |
| Interleukin 12 | | IL-12β/p40 | 306a.a. | IL23A+IL23R+IL12RB1 | |
| Interleukin 13 | A | IL-13 | 122a.a. | IL-13R1 | 321a.a. |
| Interleukin 15 | A | IL-15 | 114a.a. | IL-15Rα | 175a.a. |
| Interleukin 17 | | IL-17/CTLA8 | 132a.a. | IL-17R+IL-17RA | 287a.a. |
| Interleukin 18 | C | IL-18 | 157a.a. | IL18R1 | 164a.a. |
| Interleukin 19 | | IL-19 | 177 a.a. | IL20Rα+IL20Rβ | |
| Interleukin 20 | A | IL-20 | 152a.a. | IL20Rα+IL20Rβ | |
| Interleukin 21 | D | IL-21 | 138a.a. | IL21R | 213 a.a. |
| Interleukin 23 | D | IL-23A/p19 | 170a.a. | IL12B | 306a.a. |
| Interleukin 24 | A | IL24 | 155a.a. | IL20Rα+IL20Rβ | |
| Interleukin 27 | | IL-27A/p28 | 215a.a. | IL-27B | 209 a.a. |

In some embodiments, the ligand and the receptor thereof are selected from type A interleukins and receptors thereof, such as IL15/IL15R, IL2/IL2R, IL4/IL-4Rα+Rγ, IL-6/IL-6R, IL-11/IL-11R, IL-13/IL-13R1, IL-20/IL20Rα+IL20Rβ, and/or IL24/IL20Rα+IL20Rβ.

In some embodiments, the ligand and the receptor thereof are selected from type D interleukins and receptors thereof, such as IL7/IL7R, IL21/IL21R, and IL23A/IL12B.

In some embodiments, the interleukins and receptors thereof have amino acid sequences shown in the following table:

| Cvtokine or receptor thereof | Sequence |
|---|---|
| IL2 (human, amino acid sequence in mature form) -133a.a. |  (SEQ ID NO.21) |
| IL2Rα (human, amino acid sequence in mature form of IL2Rα extracellular domain)-219a.a. | ELCDDDPPEIPHATFKAMAYKEGTMLNCECKRGFRRIKSGSLYMLCTGNSSHSSW DNQCQCTSSATRNTTKQVTPQPEEQKERKTTEMQSPMQPVDQASLPGHCREPPP WENEATERIYHFVVGQMVYYQCVQGYRALHRGPAESVCKMTHGKTRWTQPQLIC TGEMETSQFPGEEKPQASPEGRPESETSCLVTTTDFQIQTEMAATMETSIFTTEYQ (SEQ ID NO.22) |
| IL2Rα (human, amino acid sequence in mature fully truncated form of IL2Rα extracellular domain)-166 a.a. | ELCDDDPPEIPHATFKAMAYKEGTMLNCECKRGFRRIKSGSLYMLCTGNSSHSSW DNQCQCTSSATRNTTKQVTPQPEEQKERKTTEMQSPMQPVDQASLPGHCREPPP WENEATERIYHFVVGQMVYYQCVQGYRALHRGPAESVCKMTHGKTRWTQPQLIC TGE (SEQ ID NO.23) |
| IL2Rα (human, amino acid sequence in mature half truncated form of IL2Rα extracellular domain)-191a.a. | ELCDDDPPEIPHATFKAMAYKEGTMLNCECKRELCDDDPPEIPHATFKAMAYKEGT MLNCECKRGFRRIKSGSLYMLCTGNSSHSSWDNQCQCTSSATRNTTKQVTPQPE EQKERKTTEMQSPMQPVDQASLPGHCREPPPWENEATERIYHFVVGQMVYYQCV QGYRALHRGPAESVCKMTHGKTRWTQPQLICTGEMETSQFPGEEKPQASPEGRP ESETS (SEQ ID NO.24) |
| IL-15 (human, amino acid sequence in mature form)-114a.a. | NWVNVISDLKKIEDLIQSMHIDATLYTESDVHPSCKVTAMKCFLLELQVISLESGDASI HDTVENLIILANNSLSSNGNVTESGCKECEELEEKNIKEFLQSFVHIVQMFINTS (SEQ ID NO.25) |
| IL-15Rα (human, amino acid sequence in extracellular region)-175a.a . | ITCPPPMSVEHADIWVKSYSLYSRERYICNSGFKRKAGTSSLTECVLNKATNVAHWT TPSLKCIRDPALVHQRPAPPSTVTTAGVTPQPESLSPSGKEPAASSPSSNNTAATTA AIVPGSQLMPSKSPSTGTTEISSHESSHGTPSQTTAKNWELTASASHQPPGVYPQG HSDTT (SEQ ID NO.26) |
| IL-15Rasushi (human, sushi domain amino acid sequence)-77a.a . | ITCPPPMSVEHADIWVKSYSLYSRERYICNSGFKRKAGTSSLTECVLNKATNVAHWT TPSLKCIRDPALVHQRPAPP (SEQ ID NO.27) |
| IL-15Rasushi (human, sushi domain amino acid sequence)-65a.a. | ITCPPPMSVEHADIWVKSYSLYSRERYICNSGFKRKAGTSSLTECVLNKATNVAHWT TPSLKCIR (SEQ ID NO.28) |
| IL-15Rasushi (human, sushi domain amino acid sequence)-73a.a. | ITCPPPMSVEHADIWVKSYSLYSRERYICNSGFKRKAGTSSLTECVLNKATNVAHWT TPSLKCIRDPALVHQR (SEQ ID NO.29) |
| IL-15Rasushi (human, sushi domain amino acid sequence)-86a.a. | ITCPPPMSVEHADIWVKSYSLYSRERYICNSGFKRKAGTSSLTECVLNKATNVAHWT TPSLKCIRDPALVHQRPAPPSTVTTAGVT (SEQ ID NO.30) |
| IL-15Rasushi (human, sushi domain amino acid sequence)-102a.a. | ITCPPPMSVEHADIWVKSYSLYSRERYICNSGFKRKAGTSSLTECVLNKATNVAHWT TPSLKCIRDPALVHQRPAPPSTVTTAGVTPQPESLSPSGKEPAAS (SEQ ID NO.31) |

In some embodiments, the receptor-ligand combination is selected from IL15 and IL15Rα. In the present disclosure, the "IL15Rα" and "IL15RA" are interchangeable.

In some embodiments, the IL15 and the IL15Rα comprise at least one non-natural interchain bond. In some embodiments, the non-natural interchain bond is a disulfide bond, and the IL15 and/or the IL15Rα comprise at least one amino acid mutation to cysteine. In some embodiments, the mutation is located at a contact interface of the IL-15 and the IL15Rα. In some embodiments, the cysteine mutation of the IL15 is selected from E90C, and the cysteine mutation of the IL15Rα is selected from P67C. A position of the amino acid of the IL15 refers to SEQ ID NO.26 and a position of the amino acid of the IL15Rα refers to SEQ ID NO. 27.

In some embodiments, at least one natural glycosylation site is not present in the IL15 or the IL15Rα. In some embodiments, the glycosylation site is an N-glycosylation site. In some embodiments, the IL15 comprises at least one amino acid mutation: N71Q, N79Q or N112Q. In some embodiments, the glycosylation site is an O-glycosylation site. In some embodiments, the IL15Rα comprises at least one amino acid mutation: T2A, T81A, and/or T86A.

In some embodiments, the receptor-ligand combination is selected from IL2 and IL2Rα.

In some embodiments, the IL2 and the IL2Rα comprise at least one non-natural interchain bond. In some embodiments, the non-natural interchain bond is a disulfide bond, and the IL2 and/or the IL2Rα comprise at least one amino acid mutation to cysteine. In some embodiments, S at a 75th site of the IL2 is mutated to C, and a N terminus of the IL2Rα is extended by two or three amino acids.

In some embodiments, when the N terminus of the IL2Rα is extended by two amino acids, the extended second amino acid is cysteine, and the extended first amino acid is any one of a non-polar fatty acid amino acid, an aromatic amino acid, an amino acid with an uncharged R group, an amino acid with a positively charged R group or an amino acid with a negatively charged R group.

In some embodiments, when the N terminus of the IL2Rα is extended by three amino acids, the extended second amino acid is cysteine, and the extended first and third amino acids are any one of a non-polar fatty acid amino acid, an aromatic amino acid, an amino acid with an uncharged R group, an amino acid with a positively charged R group or an amino acid with a negatively charged R group. A position of the amino acid of the IL2 refers to SEQ ID NO.21 and a position of the amino acid of the IL2Rα refers to SEQ ID NO. 22 or SEQ ID NO. 23.

Insertion or replacement positions of the cooperated receptor (or a fragment thereof) and ligand (or a fragment thereof) may be located, for example:
the receptor or a fragment thereof is inserted into or replaces a CL region, and the ligand or a fragment thereof is inserted into or replaces a CH1 region; or
the receptor or a fragment thereof is inserted into or replaces a CH1 region, and the ligand or a fragment thereof is inserted into or replaces a CL region.

### ii. Antigen-binding moiety

The present disclosure provides a bispecific fusion polypeptide comprising a first antigen-binding moiety and a second antigen-binding moiety and having two antigen specificities. The first antigen-binding moiety and the second antigen-binding moiety are different, the first antigen-binding moiety and the second antigen-binding moiety may bind to different antigens, or the first antigen-binding moiety and the second antigen-binding moiety may bind to different epitopes of the same antigen.

In some embodiments, a target of the bispecific fusion protein is a tumor. In some embodiments, binding targets of the first antigen-binding moiety and the second antigen-binding moiety are both expressed in a tumor cell. In some embodiments, a binding target of the first antigen-binding moiety is in a tumor cell and a binding target of the second antigen-binding moiety is in an immune cell. In some embodiments, binding targets of the first antigen-binding moiety and the second antigen-binding moiety are both in an immune cell.

T cell redirected killing is a desirable mechanism of action in many therapeutic field. In preclinical and clinical trials, various bispecific antibody forms were involved in T-cell redirection (May C et al., (2012) Biochem Pharmacol, 84 (9): 1105-1112; Frenkel SR, and baeuuerle PA, (2013) CURR OPIN Chemical Biology, vol. 17 (3): pages 385-92). All T cell retargeting bispecific antibodies or fragments thereof have been engineered to have at least two antigen-binding sites, one site binds to a surface antigen on a target cell and the other site binds to a T cell surface antigen. Among the T cell surface antigens, an ε subunit of human CD3 derived from a TCR protein complex is most often targeted as a target for redirected T cell killing.

Tumor-associated antigens that may be targeted include but are not limited to: α-fetoprotein (AFP), α-actinin-4, A3, an antigen specific for an A33 antibody, ART-4, B7, Ba 733, BAGE, a BrE3-antigen, CA125, CAMEL, CAP-1, a carbonic anhydrase IX, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD44, CD45, CD46, CD52, CD54, CD55, CD59, CD64, CD66a-e, CD67, CD70, CD70L, CD74, CD79a, CD80, CD83, CD95, CD126, CD132, CD133, CD138, CD147, CD154, CDC27, CDK-4/m, CDKN2A, CTLA-4, CXCR4, CXCR7, CXCL12, HIF-1α, a colon specific antigen p (CSAp), CEA (CEACAM5), CEACAM6, c-Met, DAM, EGFR, EGFRvlll, EGP-1 (TROP-2), EGP-2, ELF2-M, Ep-CAM, a fibroblast growth factor (FGF), Flt-1, Flt-3, a folate receptor, a G250 antigen, Claudin18.2, GAGE, gp100, GRO-β, HLA-DR, HM1.24, human chorionic BCMA gonadotropin (HCG) and subunits thereof, HER2/neu, HMGB-1, a hypoxia-inducible factor (HIF-1), HSP70-2M, HST-2, la, IGF-1R, IFN-γ, IFN-α, IFN-β, IFN-λ, IL-4R, IL-6R, IL-13R, IL-15R, IL-17R, IL-18R, IL-2, IL-6, IL-8, IL-12, IL-15, IL-17, IL-18, IL-23, IL-25, insulin-like growth factor-1 (IGF-1), a KC4-antigen, a KS-1 antigen, KS1-4, Le-Y, LDR/FUT, a macrophage migration inhibitory factor (MIF), MAGE, MAGE-3, MART-1, MART-2, NY-ESO-1, TRAG-3, mCRP, MCP-1, MIP-1A, MIP-1B, MIF, MUC1 MUC2, MUC3, MUC4, MUC5ac, MUC13, MUC16, MUM-1/2, MUM-3, NCA66, NCA95, NCA90, a PAM4 antigen, a pancreatic cancer mucin, a PD-1 receptor, a placental growth factor, p53, PLAGL2, a prostatic acid phosphatase, PSA, PRAME, PSMA, P1GF, ILGF, ILGF-1R, IL-6, IL-25, RS5, RANTES, T101, SAGE, S100, survivin, survivin-2B, TAC, TAG-72, tenascin, a TRAIL receptor, TNF-α, a Tn antigen, a Thomson-Friedenreich antigen, tumor necrosis antigen, VEGFR, ED-B fibronectin, WT-1, a 17-1A-antigen, complement factors C3, C3a, C3b, C5a, and C5, angiogenic markers, bcl-2, bcl-6, Kras, oncogene markers, and oncogene products (see, for example, Sensi et al., Clin Cancer Res 2006, 12: 5023-32; Pamiani et al., Jlmmonol 2007, 178:1975-79; Novellino et al., Cancer Immunol Immunother 2005, 54:187-207).

Although antibodies or other binding molecules specific for effector T cells preferably bind to a CD3 antigen, other antigens expressed on the effector T cells are known and may be targeted by T-cell redirected complexes. Exemplary T-cell antigens include, but are not limited to, CD2, CD3, CD4, CD5, CD6, CD8, CD25, CD28, CD30, CD40, CD40L, CD44, CD45, CD69, and CD90.

Immune checkpoints are inhibitory pathways in an immune system and are critical to maintaining self-tolerance and regulating duration and magnitude of a physiological immune response in a peripheral tissue to minimize collateral tissue damage. In some embodiments, the targets of the first antigen-binding moiety and the second antigen-binding moiety are immune checkpoints or ligands thereof. The immune checkpoint molecules include but are not limited to: TIGIT, PD-1, TIM-3, LAG3, GTLA4, BTLA, BTN1A1, VISTA, LAIR, CD96, PVRIG, LlLRA3, LlLRA4,LlLRB1, LILRB2, LILRB3, LLRB4, NKG-2A, CD47, CD200R1, CD300, Dectin-1, ICOS, NKp30, CD28, CD28H, CRTAM, DNAM-1, 4-1-BB, BAFF, CD27, CD30, CD40, DR3, GITR, HVEM, LIGHT, OX40, TACI, 2B4, CD2, CD48, CD229, SLAM, SLAMF5, GRAAC, TIM1, TIM4, CD7, DPPIV.

In some embodiments, the target of the first antigen-binding part is PD-1, and the target of the second antigen-binding part is PD-L1. In some embodiments, the target of the first antigen-binding moiety is PD-1, and the target of the second antigen-binding moiety is TIGIT. In some embodiments, the target of the first antigen-binding moiety is PD-1, and the target of the second antigen-binding moiety is GTLA4. In some embodiments, the target of the first antigen-binding moiety is PD-1, and the target of the second antigen-binding moiety is LAG3. In some embodiments, the target of the first antigen-binding moiety is PD-1, and the target of the second antigen-binding moiety is TIM-3. In some embodiments, the target of the first antigen-binding moiety is PD-1, and the target of the second antigen-binding moiety is CD47. In some embodiments, the target of the first antigen-binding moiety is PD-1, and the target of the second antigen-binding moiety is GTLA4. In some embodiments, the target of the first antigen-binding moiety is PD-1, and the target of the second antigen-binding moiety is 4-1-BB. In some embodiments, the target of the first antigen-binding moiety is PD-L1, and the target of the second antigen-binding moiety is 4-1-BB. In some embodiments, the target of the first antigen-binding moiety is PD-L1, and the target of the second antigen-binding moiety is TIGIT.

In some embodiments, the first antigen-binding moiety targets a tumor-associated antigen, and the second antigen-binding moiety targets an immune checkpoint. In some embodiments, the first antigen-binding moiety targets HER2, and the second antigen-binding moiety targets PD-1. In some embodiments, the first antigen-binding moiety targets VEGF, and the second antigen-binding moiety targets PD-L1. In some embodiments, the first antigen-binding moiety targets Claudin18.2, and the second antigen-binding moiety targets PD-L1. In some embodiments, the first antigen-binding moiety targets HER2, and the second antigen-binding moiety targets CTLA-4. In some embodiments, the first antigen-binding moiety targets CD20, and the second antigen-binding moiety targets CD47. In some embodiments, the first antigen-binding moiety targets HER2, and the second antigen-binding moiety targets CD47.

In some embodiments, the first antigen-binding moiety and the second antigen-binding moiety target tumor heterogeneity simultaneously. Exemplary common targets for tumors include but are not limited to HGF and VEGF, IGF-1R and VEGF, Her2 and VEGF, CD19 and CD3, CD20 and CD3, Her2 and CD3, CD19 and FcγIIIRa, CD20 and FcγRIIIa, and Her2 and FcγRIIIa. The bispecific fusion polypeptide of the present disclosure may bind to VEGF and phosphatidylserine; VEGF and ErbB3; VEGF and PLGF; VEGF and ROBO4; VEGF and BSG2; VEGF and CDCP1; VEGF and ANPEP; VEGF and c-MET; HER-2 and ERB3; HER-2 and BSG2; HER-2 and CDCP1; HER-2 and ANPEP; EGFR and CD64; EGFR and BSG2; EGFR and CDCP1; EGFR and ANPEP; IGF1R and PDGFR; IGF1R and VEGF; IGF1R and CD20; CD20 and CD74; CD20 and CD30; CD20 and DR4; CD20 and VEGFR2; CD20 and CD52; CD20 and CD4; HGF and c-MET; HGF and NRP1; HGF and phosphatidylserine; ErbB3 and IGF1R; ErbB3 and IGF1,2; c-Met and Her-2; c-Met and NRP1; c-Met and IGF1R; IGF1,2 and PDGFR; IGF1,2 and CD20; IGF1,2 and IGF1R; IGF2 and EGFR; IGF2 and HER2; IGF2 and CD20; IGF2 and VEGF; IGF2 and IGF1R; IGF1 and IGF2; PDGFRa and VEGFR2; PDGFRa and PLGF; PDGFRa and VEGF; PDGFRa and c-Met; PDGFRa and EGFR; PDGFRb and VEGFR2; PDGFRb and c-Met; PDGFRb and EGFR; RON and c-Met; RON and MTSP1; RON and MSP; RON and CDCP1; VGFR1 and PLGF; VGFR1 and RON; VGFR1 and EGFR; VEGFR2 and PLGF; VEGFR2 and NRP1; VEGFR2 and RON; VEGFR2 and DLL4; VEGFR2 and EGFR; VEGFR2 and ROBO4; VEGFR2 and CD55; LPA and S1P; EPHB2 and RON; CTLA4 and VEGF; CD3 and EPCAM; CD40 and IL6; CD40 and IGF; CD40 and CD56; CD40 and CD70; CD40 and VEGFR1; CD40 and DR5; CD40 and DR4; CD40 and APRIL; CD40 and BCMA; CD40 and RANKL; CD28 and MAPG; CD80 and CD40; CD80 and CD30; CD80 and CD33; CD80 and CD74; CD80 and CD2; CD80 and CD3; CD80 and CD19; CD80 and CD4; CD80 and CD52; CD80 and VEGF; CD80 and DR5; CD80 and VEGFR2; CD22 and CD20; CD22 and CD80; CD22 and CD40; CD22 and CD23; CD22 and CD33; CD22 and CD74; CD22 and CD19; CD22 and DR5; CD22 and DR4; CD22 and VEGF; CD22 and CD52; CD30 and CD20; CD30 and CD22; CD30 and CD23; CD30 and CD40; CD30 and VEGF; CD30 and CD74; CD30 and CD19; CD30 and DR5; CD30 and DR4; CD30 and VEGFR2; CD30 and CD52; CD30 and CD4; CD138 and RANKL; CD33 and FTL3; CD33 and VEGF; CD33 and VEGFR2; CD33 and CD44; CD33 and DR4; CD33 and DR5; DR4 and CD137; DR4 and IGF1,2; DR4 and IGF1R; DR4 and DR5; DR5 and CD40; DR5 and CD137; DR5 and CD20; DR5 and EGFR; DR5 and IGF1,2; DR5 and IGFR; DR5 and HER-2, and EGFR and DLL4. Other target combinations include one or more members of the EGF/erb-2/erb-3 family.

In addition, exemplary common targets for autoimmune and inflammatory diseases include but are not limited to IL-1 and TNFα, IL-6 and TNFα, IL-6 and IL-1, IgE and IL-13, IL-1 and IL-13, IL-4 and IL-13, IL-5 and IL-13, IL-9 and IL-13, CD19 and FcγRIIb, and CD79 and FcγRIIb.

Exemplary targets for treating inflammatory diseases include but are not limited to: TNF and IL-17A; TNF and RANKL; TNF and VEGF; TNF and SOST; TNF and DKK; TNF and αVβ3; TNF and NGF; TNF and IL-23p19; TNF and IL-6; TNF and SOST; TNF and IL-6R; TNF and CD-20; IgE and IL-13; IL-13 and IL23p19; IgE and IL-4; IgE and IL-9; IgE and IL-9; IgE and IL-13; IL-13 and IL-9; IL-13 and IL-4; IL-13 and IL-9; IL-13 and IL-9; IL-13 and IL-4; IL-13 and IL-23p19; IL-13 and IL-9; IL-6R and VEGF; IL-6R and IL-17A; IL-6R and RANKL; IL-17A and IL-1β; IL-1β and RANKL; IL-1β and VEGF; RANKL and CD-20; IL-1α And IL-1β; IL-1α and IL-1β.

Targets involved in rheumatoid arthritis (RA) include but are not limited to: TNF and IL-18; TNF and IL-12; TNF and IL-23; TNF and IL-1β; TNF and MIF; TNF and IL-17; and TNF and IL-15.

Targets for treating systemic lupus erythematosus (SLE) include but are not limited to: CD20, CD22, CD19, CD28, CD4, CD24, CD37, CD38, CD40, CD69, CD72, CD74, CD79A, CD79B, CD80, CD81, CD83, CD86, IL-4, IL-6, IL10, IL2, IL4, IL11, TNFRSF5, TNFRSF6, TNFRSF8, C5, TNFRSF7, TNFSF5, TNFSF6, TNFSF7, BLR1, HDAC4, HDAC5, HDAC7A, HDAC9, ICOSL, IGBP1, MS4A1, RGSI, SLA2, IFNB1, AICDA, BLNK, GALNAC4S-6S T, INHA, INHBA, KLF6, DPP4, FCER2, R2, ILIR2, ITGA2, ITGA3, MS4A1, ST6GALI, CDIC, CHSTIO, HLA-A, HLA-DRA, NT5E, CTLA4, B7.1, B7.2, BlyS, BAFF, IFN-α, and TNF-α.

Targets for treating multiple sclerosis (MS) include but are not limited to: IL-12, TWEAK, IL-23, CXCL13, CD40, CD40L, IL-18, VEGF, VLA-4, TNF, CD45RB, CD200, IFNγ, GM-CSF, FGF, C5, CD52, and CCR2.

Targets for treating sepsis include but are not limited to: TNF, IL-1, MIF, IL-6, IL-8, IL-18, IL-12, IL-10, IL-23, FasL, LPS, a Toll-like receptor, TLR-4, a tissue factor, MIP-2, ADORA2A, IL-1B, CASP1, CASP4, NFκB1, PROC, TNFRSFIA, CSF3, CCR3, ILIRN, MIF, NFκB1, PTAFR, TLR2, TLR4, GPR44, HMOX1, midkine, IRAK1, NFκB2, SERPINA1, SERPINE1, and TREM1.

To form the bispecific fusion protein of the present disclosure, antibodies may be prepared against any combination of these antigens; that is, each of these antigens may be optionally and independently included or not included by a polyspecific antibody of the present disclosure.

In some embodiments, the first antigen-binding moiety and the second antigen-binding moiety target different epitopes of the same antigen.

In some embodiments, at least one of the two antigen-binding fragments may further comprise a secretory signal sequence.

The secretory signal sequence refers to a sequence that induces secretion of an expressed protein or peptide by being linked to an N terminus of a coding sequence located outside a cell membrane or outside a cell, and may be a peptide sequence consisting of about 18-30 amino acids. All proteins that may be transported to the outside of the cell membrane have different signal sequences, which are cleaved by signal peptidases on the cell membrane. In general, for foreign proteins that are not naturally expressed by the host cell, the secretory signal sequences, or modified sequences that secrete the proteins into cell periplasm or a culture medium may be used.

### iii. Heterodimeric Fc fusion protein

In some embodiments, a heavy chain constant region Fc is comprised and the constant region Fc is a heterodimer (heterodimeric Fc fusion protein).

The Fc includes but is not limited to the following combinations:

CH2;

CH2+CH3;

and

CH2+CH3+CH4.

A mutation is introduced into the Fc constant region to avoid mismatching of heavy chains.

In some embodiments, the mutation introduced into the Fc constant region is based on a KiH technique (Knob-into-Holes), wherein an amino acid mutation is introduced into one heavy chain of the Fc constant region, the volume of the introduced amino acid is greater than the volume of an original amino acid residue, forming a protuberant "Knob"-like structure (Knob), and another mutation is introduced into another chain region of the Fc constant region, wherein the volume of the introduced amino acid is less than the volume of an original amino acid residue, forming a concave, a "Hole"-like structure (Hole), such that the convex heavy chain is are more likely to pair with the concave heavy chain, and mismatching of the heavy chains is avoided. This technology was developed by Genentech and described in patent application WO1996027011, which is incorporated herein in its entirety.

In some embodiments, the mutation introduction of the Fc constant region is based on electrostatic interactions. For example, an ART-lg technology was developed by Chugai, Roche, specifically alters charges of a Fc constant region domain, facilitates pairing of heterologous heavy chains, and is equivalent to a charged-version KiH technique and described in patent application WO2006106905, which is incorporated herein in its entirety.

In some embodiments, the mutation introduction of the Fc constant region is based on a SEED technology. A SEED heterodimerization is another design strategy based on a spatial mutation and the strategy utilizes a complementarity of alternating sequences derived from IgG and IgA CH3 domains (also known as AG SEED CH3 and GA SEED CH3). IgG and IgA CH3 derivatives produce complementary sequences. Therefore, when two complementary heavy chain heterodimers are assembled, assembly of homodimers lacking complementarity is excluded. This technology is described in patent application WO2007110205, which is incorporated herein in its entirety.

In some embodiments, the mutation introduction of the Fc constant region is based on an isoelectric point change, which facilitates improving a heterodimer formation rate and maintaining stability of the Fc region. This technology is described in WO2014145806, which is incorporated herein in its entirety.

In some embodiments, the Fc constant region is a heterodimer associated based on hydrophilic interactions or increased flexibility.

In some embodiments, the Fc constant region is a heterodimer associated based on any combination of the above technologies, e.g., in some embodiments, the Fc constant region is mutated based on a combination of the KIH and the electrostatic interactions. For example, an XmAb bispecific platform method may improve thermostability of the bispecific antibody by binding electrostatic interactions, CH3 domain conformations, and hydrogen bonds. In particular, this strategy mutates and exchanges an Fc side chain of natural IgG1 into S364K and K370S heterodimers to form hydrogen bonds between the two heterodimers, followed by L368D/K370S replacement to drive an interaction of a salt bridge to promote heterodimer formation, patent application WO2014145907, which is incorporated herein in its entirety.

In some embodiments, all or a portion of the CH2, CH3, or CH4 region is inserted into or replaced with a receptor and a ligand thereof.

In some embodiments, the inserted or replaced regions are independently located in the CH2, CH3, or CH4 region, or any location between adjacent regions (e.g., CH1-CH2 junction, CH2-CH3 junction, and CH3-CH4 junction).

In some embodiments, when any two constant regions (such as any one of CL-CH1, CH2-CH2, CH3-CH3, or CH4-CH4 regions) mentioned above are inserted or replaced, an affinity between two conjugate fragments that cooperate with each other in the replacement regions is K_{D} < 1×10⁻³ (M), such as x × 10⁻⁴ (M), x × 10⁻⁵ (M), x × 10⁻⁶ (M), x × 10⁻⁷ (M), x × 10⁻⁸ (M), x × 10⁻⁹ (M), x × 10⁻¹⁰ (M), and x × 10⁻¹¹ (M); and the value of x may be selected from 1-9, such as 2, 3, 4, 5, 6, 7, and 8.

In some embodiments, an N terminus and/or a C terminus of the conjugate fragment is linked to the antigen-binding fragment by a linker peptide.

In some embodiments, the number of amino acids in the linker peptide ranges from 1-30; and may be 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30, preferably 5-20.

In some embodiments, the amino acids of the linker peptide are nonsense polypeptides that do not have additional functions (e.g., protein localization, cleavage sites, etc.) other than linking.

In some embodiments, the linker peptide is a flexible linker peptide.

In some embodiments, an amino acid sequence of the linker peptide is selected from one or more of Gly, Ser, Pro, Ala, and Glu.

In some embodiments, the amino acid sequence of the linker peptide is selected from (GGGGS)n, (GGGS)n, (GGS)n, (GS)n, or (G)n, wherein n is selected from 1, 2, 3, 4, 5, or 6.

The linker peptide is generally flexible and may reduce steric hindrance between the fusion protein and a target protein, thereby facilitating proper folding of the protein.

In further embodiments, the linker peptide is a rigid linker peptide; i.e. a relatively non-flexible peptide linker. The rigid linker peptide does not require a complete lack of flexibility, but is less flexible than the flexible linker peptide such as a glycine-rich peptide linker. Due to its relative lack of flexibility, the rigid linker peptide reduces a movement of two protein domains (in the present case, a stabilizer protein and a thermostable reverse transcriptase) linked together by the rigid linker peptide. A linker peptide providing an ordered chain (e.g., an α helical structure) may provide a rigid linker peptide. For example, arginine, leucine, glutamic acid, glutamine, and methionine all exhibit a relatively high inclination for a helical linker structure. However, a non-helical linker containing many proline residues may also exhibit a significant rigidity. Examples of the rigid linker peptide include polylysine and poly-DL-alanine polylysine. A further description of a rigid peptide linker is provided by Wriggers et al., Biopolymers, 80, pages 736-46 (2005). In addition, the rigid linker peptide is described in a linker database described by George et al., Protein Engineering, 15, pages 871-79 (2003). Preferably, the rigid linker peptide is also a non-cleavable linker peptide, i.e. a non-cleavable rigid linker peptide.

### Isolated nucleic acid

The present disclosure further relates to an isolated nucleic acid encoding a bispecific fusion polypeptide or a multifunctional fusion protein as described above.

The term "isolated nucleic acid" herein refers to a deoxyribonucleic acid or ribonucleic acid polymer in a single-stranded or double-stranded form. The isolated nucleic acid includes a RNA genomic sequence, DNA (gDNA and cDNA) or a RNA sequence transcribed from DNA. Unless otherwise specified, the polypeptide further includes natural polynucleotide, sugar, or base changed analogs. According to one aspect of the present disclosure, the polynucleotide is a light-chain polynucleotide.

The isolated nucleic acid includes a nucleotide sequence encoding an amino acid sequence of a protein complex and also includes a nucleotide sequence complementary thereto. The complementary sequence includes a fully complementary sequence and a substantially complementary sequence, which refers to a sequence that hybridizes under a stringent condition known in the art to the nucleotide sequence encoding the amino acid sequence of the protein complex.

Furthermore, the nucleotide sequence encoding the amino acid sequence of the protein complex may be altered or mutated. Such alterations include additions, deletions, or non-conservative or conservative substitutions. A polynucleotide encoding the amino acid sequence of the protein complex may be interpreted to include a nucleotide sequence that is substantially identical to the isolated nucleic acid. The substantial identity aligns the nucleotide sequence with an additional random sequence to maximize their correspondence. When the aligned sequences are analyzed with an algorithm common in the field, the sequence may show a homology greater than 80%, greater than 90%, or greater than 95%.

### Vector

The present disclosure further relates a vector containing the nucleic acid.

The term "vector" refers to a nucleic acid delivery vehicle into which a polynucleotide may be inserted. When a vector is capable of expressing a protein encoded by an inserted polynucleotide, the vector is referred to as an expression vector. The vector may be introduced into a host cell by transformation, transduction, or transfection such that a genetic material element it carries is expressed in the host cell. The vectors are well known to a person skilled in the art, and include but are not limited to: plasmid; phagemid; cosmid; artificial chromosomes such as yeast artificial vhromosome (YAC), bacterial artificial chromosome (BAC), or P1-derived artificial chromosome (PAC); and phage such as λ phage or M13 phage, animal viruses, etc. The animal viruses that may be used as a vector include, but are not limited to, retroviruses (including lentiviruses), adenoviruses, adeno-associated viruses, herpes viruses (e.g., herpes simplex viruses), pox viruses, baculoviruses, papilloma viruses, and papovaviruses (e.g., SV40). The vector may contain a selectable marker (e.g., a tag to facilitate enrichment, such as his tag; or a tag to facilitate detection, such as GFP), and an origin of replication compatible with a cell type prescribed by the cloning vector. The expression vector contains regulatory elements necessary to affect expressions in the prescribed target cells, such as enhancers, promoters, internal ribosome entry sites (IRES), and other expression control elements (e.g., transcription termination signals, or polyadenylation signals and poly-U sequences, etc.). The vector may be a cloning vector or an expression vector. When the antibody or the fragment is expressed or prepared, a prokaryotic expression vector and a eukaryotic expression vector are usually involved, PET series and pGEX series are usually used as the prokaryotic expression vector, and pcDNA3.1, pcDNA3.4, pcDNA4, pEGFP-N1, pEGFP-N1, pSV2, etc. are usually used as the eukaryotic expression vector.

In the present disclosure, the vector may be a composition, for example a mixture of various plasmids, and different plasmids carrying a portion of an antibody or a fragment thereof.

### Host cell

The present disclosure further relates to a host cell containing the nucleic acid or the vector.

Various cultured host cells that may be used include, for example, prokaryotic cells, eukaryotic cells, bacterial cells (such as *Escherichia coli* or *Bacillus stearothermophilus),* fungal cells (such as *Saccharomyces cerevisiae* or *Pichia pastoris*), insect cells (such as *Lepidoptera* insect cells including *Spodoptera frugiperda* cells), or mammalian cells (such as Chinese Hamster Ovary (CHO) cells, NS0 cells, baby hamster kidney (BHK) cells, monkey kidney cells, Hela cells, human hepatocellular carcinoma cells, or 293 cells, etc.).

### Method for preparing bispecific fusion polypeptide or multifunctional fusion protein

The bispecific fusion polypeptide or the multifunctional fusion protein of the present disclosure may be prepared according to any well-known methods in the art,
for example, transforming a host cell with the vector;
culturing the transformed host cell; and
collecting the bispecific fusion polypeptide or the multifunctional fusion protein expressed in the host cell.

In particular, the following methods may be used.

Early methods for constructing a bispecific antibody include a chemical crosslinking method or a hybrid hybridoma or quadroma method (such as Staerz UD et al., Nature, 314: 628-31, 1985; Milstein C et al., Nature, 305: 537-540, 1983; Karpovsky B et al., J.Exp.Md., 160:1686-1701, 1984). The chemical coupling method is to link 2 different monoclonal antibodies together by chemical coupling to prepare a bispecific monoclonal antibody, for example, chemical binding of two different monoclonal antibodies, or for example, chemical binding of two antibody fragments, such as two Fab fragments. The hybrid hybridoma method is to produce a bispecific monoclonal antibody by a cell hybridization method or a triple hybridoma method. These cell hybridomas or ternary hybridomas are fused by established hybridomas or obtained by fusing the established hybridomas with lymphocytes obtained from mice. Although these technologies are used to make BiAb, various produced problems make such complexes difficult to use, such as generation of a mixed population containing different combinations of antigen-binding sites, difficulties in protein expression, need to purify the target BiAb, low yield, high production cost, etc.

Recent methods utilize genetically engineered constructs that are capable of producing a homogeneous product of a single BiAb without the need for thorough purification to remove unwanted side products. Such constructs include tandem scFv, bispecific antibodies, tandem bispecific antibodies, double variable domain antibodies, and heterodimers using motifs such as Ch1/Ck domain or DNLTM (Chames&Baty, Curr. Opin. Drug. Discov. Devel., 12: 276-83, 2009; Chames&Baty, mAbs, 1: 539-47). Related purification technologies are well-known.

Antibodies may also be produced using a single lymphocyte antibody method by cloning and expressing immunoglobulin variable region cDNA produced by single lymphocytes selected for producing specific antibodies, for example, the methods by Babcook J et al., Proc.Natl.Acad.Sci.USA.93: 7843-7848, 1996; WO 92/02551; WO 2004/051268, and WO 2004/106377.

Antigenic polypeptides for producing antibodies, e.g., for immunizing a host or for panning a phage display (yeast cell or bacterial cell surface expression) may be prepared from genetically engineered host cells comprising expression systems by methods well-known in the art, or they may be recovered from natural biological sources. For example, a nucleic acid encoding one or both polypeptide chains of a bispecific antibody may be introduced into a cultured host cell by a variety of known methods (e.g., transformation, transfection, electroporation, bombardment with nucleic acid-coated microparticles, etc.). In some embodiments, the nucleic acid encoding the bispecific antibody may be inserted into a vector suitable for expression in a host cell prior to introduction into the host cell. Typically, the vector may contain a sequence element that enables the inserted nucleic acid to be expressed at RNA and protein levels.

The bispecific antibody of the present disclosure or a portion thereof may be used to detect any or all of these antigens (e.g., in a biological sample such as serum or plasma) by a conventional immunological assay method, such as enzyme-linked immunosorbent assay (ELISA), radioimmunoassay (RIA), or tissue immunohistochemistry. The present disclosure provides a method of detecting an antigen in a biological sample, comprising: contacting the biological sample with the bispecific antibody, or an antibody portion of the present disclosure, which specifically recognizes the antigen, and detecting the antibody (or antibody portion), or a non-bound antibody (or antibody portion), bound to the antigen, thereby detecting the antigen in the biological sample. The antibody is directly or indirectly labeled with a detectable substance to facilitate detection of a bound or unbound antibody. Suitable detectable substances include various enzymes, repair groups, fluorescent substances, luminescent substances, and radioactive substances. Examples of suitable enzymes include horseradish peroxidase, alkaline phosphatase, β-galactosidase, and acetylcholinesterase; examples of suitable repair group complexes include streptavidin/biotin and avidin/biotin; examples of suitable fluorescent substances include 7-hydroxycoumarin, fluorescein, fluorescein isothiocyanate, rhodamine B, dichlorotriazinylamine fluorescein, dansyl chloride or phycoerythrin; examples of luminescent substances include 3-aminophthalicacid cyclic hydrazine; and examples of suitable radioactive substances include I¹²⁵, I¹³¹, ³⁵S, or ³H.

### Pharmaceutical composition

The bispecific fusion polypeptide or the multifunctional fusion protein or the nucleic acid encoding same of the present disclosure may be used in preparing a pharmaceutical composition or a sterile composition, e.g., the bispecific fusion polypeptide or the multifunctional fusion protein is mixed with a pharmaceutically acceptable carrier, an excipient, or a stabilizer. The pharmaceutical composition may comprise one or a combination (e.g. two or more different) of the antibody or a functional fragment thereof of the present disclosure. For example, the pharmaceutical composition of the present disclosure may comprise a combination of an antibody or an antibody fragment (or immunoconjugate) with a complementary activity that binds to different epitopes on a target antigen. Preparations of therapeutic and diagnostic agents may be prepared by mixing, for example, freeze-dried powder, slurry, an aqueous solution or a suspension, with a pharmaceutically acceptable carrier, an excipient or a stabilizer.

The bispecific fusion polypeptide or the multifunctional fusion protein in the pharmaceutical composition may be in a form that is bound to a second activator (functional molecule). The second activator may be a randomly functional molecule capable of preventing or treating a target disease and may include a compound, peptide, polypeptide, nucleic acid, carbohydrate, lipid, or inorganic particle. In the pharmaceutical composition, the bispecific fusion polypeptide or the multifunctional fusion protein may itself have a therapeutic activity; it may function to target the second activator to a specific disease region. The disease region may be those organs, tissues, or cells in which the bispecific antibody that specifically binds to an antigen is aggregated and distributed. A drug targeted to the disease region is present in a high concentration, such that a drug effect is increased compared to the injected amount. Therefore, the pharmaceutical composition may be used to treat drug-resistant tumors and may reduce side effects and adverse drug reactions due to non-specific drug distribution.

The activator comprising the bispecific fusion polypeptide or the multifunctional fusion protein in the pharmaceutical composition may be contained in microcapsules, or in a drug delivery system of a colloidal nature (such as liposomes, albumin spherules, microemulsions, nanoparticles, and nanocapsules), or in macroemulsions. The microcapsules may be prepared, for example, by a coacervation technology or an interfacial polymerization, examples include hydroxymethyl cellulose or gelatin microcapsules and poly-(methylmethacylate) microcapsules respectively.

### Medical use and therapeutic method

The present disclosure further relates to use of the bispecific fusion polypeptide or the multifunctional fusion protein in preparing a drug for treating a disease.

According to one aspect of the present disclosure, the disease may be, for example, cancer, immune disorders, metabolic diseases, and microbial infections.

The term "cancer" refers to a class of diseases characterized by an uncontrolled growth of abnormal cells *in vivo.* The "cancer" includes benign and malignant cancers as well as dormant tumors or micrometastases.

In some embodiments, microorganisms in the microbial infections may be exogenous pathogens or a population of cells bearing the exogenous pathogens, such as viruses. The present disclosure is applicable to the exogenous pathogens such as bacteria, fungi, viruses, mycoplasmas, and parasites. The pathogens that may be treated with the present disclosure may be any infectious organisms known in the art to be pathogenic in animals, including the following organisms such as: gram-negative or gram-positive cocci or bacilli, DNA viruses, and RNA viruses, including, but not limited to, DNA viruses such as papillomaviruses, parvoviruses, adenoviruses, herpesviruses, and vaccinia viruses, and RNA viruses such as arenaviruses, coronaviruses, rhinoviruses, respiratory syncytial viruses, influenza viruses, picornaviruses, paramyxoviruses, reoviruses, retroviruses, and rhabdoviruses. Antibiotic-resistant bacteria of particular interest are such as antibiotic-resistant *Streptococcus species* and *Staphlococcus species,* or bacteria that are sensitive to antibiotics but cause recurrent infections treated with antibiotics so as to finally generate resistant organisms. Such organisms may be treated with the ligand-immunogen conjugate of the present disclosure in combination with antibiotics at doses lower than those normally administered to patients to avoid generation of these antibiotic-resistant bacterial strains. The present disclosure is also applicable to any fungi, mycoplasma species, parasites or other infectious organisms that are pathogenic in animals. Examples of fungi that may be treated by the method of the present disclosure include fungi that grow as molds or yeasts, including, for example, fungi that cause diseases such as: tinea, histoplasmosis, blastomycosis, aspergillosis, cryptococcosis, sporotrichosis, coccidioidomycosis, paracoccidioidomycosis, and candidiasis. The present disclosure may be used to treat parasitic infections, including but not limited to infections caused by the following parasites: tapeworm, schistosome, roundworm, amoeba and *Plasmodium, Trypanosoma, Leishmania,* and *Toxoplasma* species. Parasites of particular interest are those that express a folate receptor and bind to folate; however, there is a large number of references for ligands exhibiting high affinity for infectious organisms. For example, penicillin and cephalosporins known for their antibiotic activity and specific binding to bacterial cell wall precursors may likewise be used as ligands for preparing the ligand-immunogen conjugates for use according to the present disclosure. The ligand-immunogen conjugate of the present disclosure may also be directed to a cell population bearing an endogenous pathogen, wherein a pathogen-specific antigen is preferentially expressed on a surface of cells bearing the pathogen and serves as a receptor for a ligand that specifically binds to the antigen.

The present disclosure further relates to a method for preventing and/or treating the disease and administering the therapeutically effective amount of the pharmaceutical composition to prevent and/or treat the disease as described above.

The method of the present disclosure may be used in human clinical medicine and veterinary medicine. Therefore, host animals bearing a pathogenic organism population and treated with the ligand-immunogen conjugate may be human, or in the case of veterinary medicine application, may be laboratory animals, agricultural animals, domestic animals, or wild animals. The present disclosure may be applicable to the host animals including, but not limited to: human; laboratory animals such as rodents (e.g., mice, rats, hamsters, etc.), rabbits, monkeys, and chimpanzees; domestic animals such as dogs, cats, and rabbits; agricultural animals, such as cattle, horses, pigs, sheep, and goats; and wild animals raised in captivity, such as bears, pandas, lions, tigers, leopards, elephants, zebras, giraffes, gorillas, dolphins, and whales.

The pharmaceutical composition may be injected into entities, including rats, mice, domestic animals, and/or human, by a variety of routes. All injection methods can be expected, for example, oral, rectal, intravenous, nasal, abdominal, subcutaneous, or topical injection. The composition may be injected by other methods known in the art.

The "therapeutically effective amount" means herein an amount sufficient to treat a disease, in view of a reasonable profit/loss ratio. The therapeutically effective amount may vary from patients for a variety of reasons, e.g., type of disease, severity, onset, age, weight, excretion rate, susceptibility to reaction, health status, and/or complications; and/or drug activity, route of injection, injection cycle, and number of injections, and/or pharmaceutical combinations; and may be appropriately selected by a person skilled in the art depending on a treatment purpose. For example, the injection amount may be randomly divided into a plurality of times such that the amount is about 0.001-100 mg/kg of adult body weight.

The bispecific fusion polypeptide or the multifunctional fusion protein of the present disclosure or the nucleic acid or the polynucleotide encoding the antibody of the present disclosure may also be administered in combination with, for example, standard cancer treatments (e.g., surgery, radiation, and chemotherapy). For example, anti-tumor therapies using these compositions of the present disclosure and/or effector cells equipped with these compositions are used in combination with chemotherapy. Non-limiting examples of antibody combined therapies of the present disclosure include surgery, chemotherapy, radiotherapy, immunotherapy, gene therapy, DNA therapy, RNA therapy, nanotherapy, viral therapy, adjuvant therapy, and a combination thereof.

The embodiment of the present disclosure will be described in detail below with reference to examples.

### Example 1 FiBody design

In some examples, FiBody was a bispecific antibody obtained recombinantly with a specific affinity between a ligand and a receptor thereof and replacing CL and CH1 on one side of the bispecific antibody, which may avoid or reduce mismatching between a light chain and a heavy chain of the bispecific antibody.

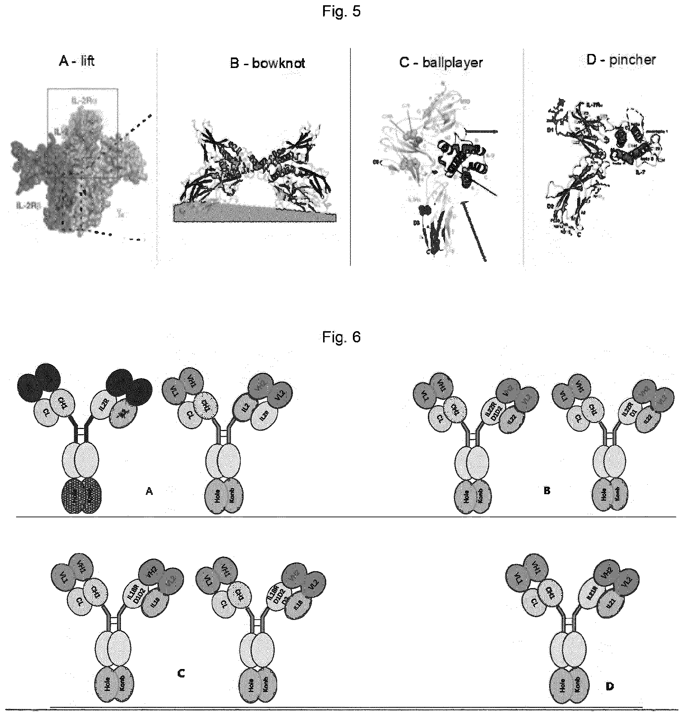

In the present example, FiBody was constructed by taking interleukins and receptors thereof as an example, and classified into four types according to steric conformantions of the interleukins and receptors thereof shown in FIG. 5:

| steric conformation | Classification No. | Interleukin and receptor thereof |
|---|---|---|
| Lift type | A | IL15/IL15R, IL2/IL2R, IL4/IL-4Rα+Rγ, IL-6/IL-6R, IL-11/IL-11R, IL-13/IL-13R1, IL-20/IL20Rα+HL20Rβ, and IL24/IL20Rα+HL20Rβ |
| Bowknot type | B | IL10/IL10R1 and IL22/IL22R |
| Ballplayer type | C | IL1β/1LR1, IL3/IL3R, IL5/IL5R, and IL18/IL18R1 |
| Pincer type | D | IL7/TL7R, IL21/IL21R, and IL23A/IL12B |

The bispecific antibodies were respectively constructed based on the 4 types of interleukins and receptors thereof.

### Example 2 Construction of FiBody based on interleukins and receptors thereof

VH targeting a first antibody was selected, linked to a receptor protein through a Linker, and linked to Fc of an antibody through a Hinge; VL targeting to the first antibody was linked to a ligand protein through a Linker to reduce or avoid mismatching of a light chain and a heavy chain; and the other end was an intact Fab structure targeting a second antibody (anti-TIGIT antibody), and the Fc making up the first antibody and the Fc making up the second antibody had a conventional KiH modification to reduce or avoid mismatching of heavy chains. or

VH targeting a first antibody was selected, linked to a ligand protein through a Linker, and linked to Fc of an antibody through a Hinge; VL targeting to a second antibody was linked to a receptor protein (IL15) through a Linker to reduce or avoid mismatching of a light chain and a heavy chain; and the other end was an intact Fab structure targeting a first antibody, and the Fc making up the first antibody and the Fc making up the second antibody had a conventional KiH modification to reduce or avoid mismatching of heavy chains.

| Molecule No. | Molecule name | Replacement mode of antibody structure | | Classification of steric conformation |
|---|---|---|---|---|
| | | Replacement of CH1 of heavy chain | Replacement of CL of light chain | |
| Molecule 1 | R0950 | IL15RA | IL15 | A |
| Molecule 2 | R0951 | ILI5RA | IL15 | A |
| Molecule 3 | R0952 | IL15 | IL15RA | A |
| Molecule 4 | R1115 | IL2RA | IL2 | A |
| Molecule 5 | R1116 | IL2 | IL2RA | A |
| Molecule 6 | R1117 | IL22RA_D1D2 | IL22 | B |
| Molecule 7 | R1118 | IL22RA_D1 | IL22 | B |
| Molecule 8 | R1119 | IL18R1_D1D2 | IL18 | C |
| Molecule 9 | R1120 | IL18R1_D10203 | IL18 | C |
| Molecule 10 | R1123 | IL21R | IL21 | D |

Some specific implementations were shown in the following table:

| **Molecule 1** | **R0950: PD-L_1_VH_IL15RA/ PD-L1_VL_IL15/TIGIT-Fab (****FIG.7****)** |
|---|---|
| 1st polypeptide | @PD-L1_VH_IL15RA_Fc-Knob (SEQ ID NO.1) |
| | EVQLLESGGGLVQPGGSLRLSCAASGFTFSSYIMMWVRQAPGKGLEWVSSIYPSGGITFYADTVKGRFTISRDNSK NTLYLQMNSLRAEDTAVYYCARIKLGTVTTVDYWGQGTLVTVSSGGGGSGGGGSGGGGSGGGGSGITCPPPMSV EHADIWVKSYSLYSRERYICNSGFKRKAGTSSLTECVLNKATNVAHWTTPSLKCIRDPALVHQRPAPPSTVTTAGVTP QPESLSPSGKEPAASSPSSNNTAATTAAIVPGSQLMPSKSPSTGTTEISSHESSHGTPSQTTAKNWELTASASHQPP GVYPQGHSDTTEPRGPTIKPCPPCKCPAPNLLGGPSVFIFPPKIKDVLMISLSPIVTCVVVDVSEDDPDVQISWFVNN VEVHTAQTQTHREDYNSTLRVVSALPIQHQDWMSGKEFKCKVNNKDLPAPIERTISKPKGSVRAPQVYVLPPPEEEM TKKQVTLWCMVTDFMPEDIYVEWTNNGKTELNYDNTEPVLDSDGSYFMYSDLRVEKKNWVERNSYSCSVVHEGLH NHHTTKSFSRTPGK |
| second polypeptide | @PD-L1_VL_IL15 (SEQ ID NO.2) |
| | QSALTQPASVSGSPGQSITISCTGTSSDVGGYNYVSWYQQHPGKAPKLMIYDVSNRPSGVSNRFSGSKSGNTASLTI SGLQAEDEADYYCSSYTSSSTRVFGTGTKVTVLGGGGSGGGGSGGGGSGGGGSGNWVNVISDLKKIEDLIQSMHI DATLYTESDVHPSCKVTAMKCFLLELQVISLESGDASIHDTVENLIILANNSLSSNGNVTESGCKECEELEEKNIKEFLQ SFVHIVQMFINTS |
| third polypeptide | @TIGIT_VH_CH1_Fc-Hole (SEQ ID NO.3) |
| | QVQLESEGGLFKPTDTLTLTCTVSGSSLSSSYMSWVRQAPGKGLEWIGIIGSNGNTYYANWAKGRFTISKTSTTVEL KITSPTTEDTATYFCARGGYRTSGMDPWGPGTLVTVSSAKTTAPSVYPLAPVCGDTTGSSVTLGCLVKGYFPEPVTL TWNSGSLSSGVHTFPAVLQSDLYTLSSSVTVTSSTWPSQSITCNVAHPASSTKVDKKIEPRGPTIKPCPPCKCPAPNL LGGPSVFIFPPKIKDVLMISLSPIVTCVVVDVSEDDPDVQISWFVNNVEVHTAQTQTHREDYNSTLRVVSALPIQHQD WMSGKEFKCKVNNKDLPAPIERTISKPKGSVRAPQVYVLPPPEEEMTKKQVTLTCRVTDFMPEDIYVEWTNNGKTEL NYKNTEPVLKSDGSYFMASKLRVEKKNWVERNSYSCSVVHEGLHNHHTTKSFSRTPGK |
| fourth polypeptide | @TIGIT_VL_CL (SEQ ID NO.4) |
| | DIVMTQTPASVEVAVGGTVTIKCQASQSISSYLNWYQQKPGQPPKLLIYDALKLASGVPSRFSGSGSGTEYTLTISGV ESADAATYYCQQEHSVGNVDNVFGGGTEVVVKRTDAAPTVSIFPPSSEQLTSGGASVVCFLNNFYPKDINVKWKID GSERQNGVLNSWTDQDSKDSTYSMSSTLTLTKDEYERHNSYTCEATHKTSTSPIVKSFNRNEC |

| **Molecule 2** | **R0951: TIGIT_VH_IL15RA/ TIGIT_VL_IL15/ PD-L1-Fab** |
|---|---|
| first polypeptide | @TIGIT_VH_IL15RA_Fc-Knob (SEQ ID NO.5) |
| | QVQLESEGGLFKPTDTLTLTCTVSGSSLSSSYMSWVRQAPGKGLEWIGIIGSNGNTYYANWAKGRFTISKTSTTVEL KITSPTTEDTATYFCARGGYRTSGMDPWGPGTLVTVSSGGGGSGGGGSGGGGSGGGGSGITCPPPMSVEHADIW VKSYSLYSRERYICNSGFKRKAGTSSLTECVLNKATNVAHWTTPSLKCIRDPALVHQRPAPPSTVTTAGVTPQPESLS PSGKEPAASSPSSNNTAATTAAIVPGSQLMPSKSPSTGTTEISSHESSHGTPSQTTAKNWELTASASHQPPGVYPQG HSDTTEPRGPTIKPCPPCKCPAPNLLGGPSVFIFPPKIKDVLMISLSPIVTCVVVDVSEDDPDVQISWFVNNVEVHTAQ TQTHREDYNSTLRVVSALPIQHQDWMSGKEFKCKVNNKDLPAPIERTISKPKGSVRAPQVYVLPPPEEEMTKKQVTL WCMVTDFMPEDIYVEWTNNGKTELNYDNTEPVLDSDGSYFMYSDLRVEKKNWVERNSYSCSVVHEGLHNHHTTK SFSRTPGK |
| second polypeptide | @TIGIT_VL_IL15 (SEQ ID NO.6) |
| | DIVMTQTPASVEVAVGGTVTIKCQASQSISSYLNWYQQKPGQPPKLLIYDALKLASGVPSRFSGSGSGTEYTLTISGV ESADAATYYCQQEHSVGNVDNVFGGGTEVVVKGGGGSGGGGSGGGGSGGGGSGNWVNVISDLKKIEDLIQSMHI DATLYTESDVHPSCKVTAMKCFLLELQVISLESGDASIHDTVENLIILANNSLSSNGNVTESGCKECEELEEKNIKEFLQ SFVHIVQMFINTS |
| third polypeptide | @PD-L1_VH_CH1_Fc-Hole (SEQ ID NO.7) |
| | EVQLLESGGGLVQPGGSLRLSCAASGFTFSSYIMMWVRQAPGKGLEWVSSIYPSGGITFYADTVKGRFTISRDNSK NTLYLQMNSLRAEDTAVYYCARIKLGTVTTVDYWGQGTLVTVSSAKTTAPSVYPLAPVCGDTTGSSVTLGCLVKGYF PEPVTLTWNSGSLSSGVHTFPAVLQSDLYTLSSSVTVTSSTWPSQSITCNVAHPASSTKVDKKIEPRGPTIKPCPPCK CPAPNLLGGPSVFIFPPKIKDVLMISLSPIVTCVVVDVSEDDPDVQISWFVNNVEVHTAQTQTHREDYNSTLRVVSALP IQHQDWMSGKEFKCKVNNKDLPAPIERTISKPKGSVRAPQVYVLPPPEEEMTKKQVTLTCRVTDFMPEDIYVEWTNN GKTELNYKNTEPVLKSDGSYFMASKLRVEKKNWVERNSYSCSVVHEGLHNHHTTKSFSRTPGK |
| fourth polypeptide | @PD-L1_VL_CL (SEQ ID NO.8) |
| | QSALTQPASVSGSPGQSITISCTGTSSDVGGYNYVSWYQQHPGKAPKLMIYDVSNRPSGVSNRFSGSKSGNTASLTI SGLQAEDEADYYCSSYTSSSTRVFGTGTKVTVLRTDAAPTVSIFPPSSEQLTSGGASVVCFLNNFYPKDINVKWKID GSERQNGVLNSWTDQDSKDSTYSMSSTLTLTKDEYERHNSYTCEATHKTSTSPIVKSFNRNEC |

| **Molecule 3** | **R0962: TIGIT_VH_IL15/ TIGIT_VL_IL15 RA / PD-L1-Fab (****FIG.7****)** |
|---|---|
| first polypeptide | @TIGIT_VH_IL15_Fc-Knob (SEQ ID NO.9) |
| | QVQLESEGGLFKPTDTLTLTCTVSGSSLSSSYMSWVRQAPGKGLEWIGIIGSNGNTYYANWAKGRFTISKTSTTVEL KITSPTTEDTATYFCARGGYRTSGMDPWGPGTLVTVSSGGGGSGGGGSGGGGSGGGGSGNWVNVISDLKKIEDLI QSMHIDATLYTESDVHPSCKVTAMKCFLLELQVISLESGDASIHDTVENLIILANNSLSSNGNVTESGCKECEELEEKNI KEFLQSFVHIVQMFINTSEPRGPTIKPCPPCKCPAPNLLGGPSVFIFPPKIKDVLMISLSPIVTCVVVDVSEDDPDVQIS WFVNNVEVHTAQTQTHREDYNSTLRVVSALPIQHQDWMSGKEFKCKVNNKDLPAPIERTISKPKGSVRAPQVYVLP PPEEEMTKKQVTLWCMVTDFMPEDIYVEWTNNGKTELNYDNTEPVLDSDGSYFMYSDLRVEKKNWVERNSYSCSV VHEGLHNHHTTKSFSRTPGK |
| second polypeptide | @TIGIT_VL_IL15RA (SEQ ID NO.10) |
| | DIVMTQTPASVEVAVGGTVTIKCQASQSISSYLNWYQQKPGQPPKLLIYDALKLASGVPSRFSGSGSGTEYTLTISGV ESADAATYYCQQEHSVGNVDNVFGGGTEVVVKGGGGSGGGGSGGGGSGGGGSGITCPPPMSVEHADIWVKSYS LYSRERYICNSGFKRKAGTSSLTECVLNKATNVAHWTTPSLKCIRDPALVHQRPAPPSTVTTAGVTPQPESLSPSGKE PAASSPSSNNTAATTAAIVPGSQLMPSKSPSTGTTEISSHESSHGTPSQTTAKNWELTASASHQPPGVYPQGHSDTT |
| third | @PD-L1 VH_CH1_Fc-Hole (SEQ ID NO.7) |
| polypeptide fourth polypeptide | @PD-L1_VL_CL (SEQ ID NO.8) |

| **Molecule 4** | **R1115: TIGIT_VH_IL2RA/ TIGIT_VL_IL2 / PD-L1-Fab** |
|---|---|
| first polypeptide | @TIGIT_VH_IL2RA_Fc-Hole (SEQ ID NO.32) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGELCDDDPPEI PHATFKAMAYKEGTMLNCECKRGFRRIKSGSLYMLCTGNSSHSSWDNQCQCTSSATRNTTKQVTPQPEEQKERKT TEMQSPMQPVDQASLPGHCREPPPWENEATERIYHFVVGQMVYYQCVQGYRALHRGPAESVCKMTHGKTRWTQ PQLICTGEPKSSDKTHTCPPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPREPQVYTLPPSREEMTKN QVSLTCRVKGFYPSDIAVEWESNGQPENNYKTTPPVLKSDGSFFLASKLTVDKSRWQQGNVFSCSVMHEALHNHY TQKSLSLSPGK |
| second polypeptide | @TIGIT_VL_IL2(SEQ ID NO.33) |
| | DIQMTQSPSSLSASVGDRVTITCRASENIYSNLAWYQQKPGKSPKLLVYAASHLPDGVPSRFSGSGSGTDYSLTISSL QPEDFATYYCQHFWGTPRTFGQGTKLEIKGGGGSGGGGSGGGGSGGGGSGAPTSSSTKKTQLQLEHLLLDLQMIL NGINNYKNPKLTRMLTFKFYMPKKATELKHLQCLEEELKPLEEVLNLAQSKNFHLRPRDLISNINVIVLELKGSETTFM CEYADETATIVEFLNRWITFCQSIISTLT |
| third polypeptide | @PD-L1_VH_CH1_Fc-Knob (SEQ ID NO.34) |
| | EVQLQESGPGLVKPSETLSLTCAVYGDSITSGYWNWIRKPPGKGLEYMGYISYTGSTYQNPSLKSRITFSRDTSKNQ YYLKLSSVTAADTATYYCARSRAWIRTYFDYWGQGTLVTVSSASTKGPSVFPLAPSSKSTSGGTAALGCLVKDYFPE PVTVSWNSGALTSGVHTFPAVLQSSGLYSLSSVVTVPSSSLGTQTYICNVNHKPSNTKVDKRVEPKSCDKTHTCPPC PAPELLGGPSVFLFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLWCLVKGFYPSDIAVEWES NGQPENNYDTTPPVLDSDGSFFLYSDLTVDKSRWQQGNVFSCSVMHEALHNHYTQKSLSLSPGK |
| fourth polypeptide | @PD-L1_VL_CL (SEQ ID NO.35) |
| | EIVLTQSPDFQSVTPKEKVTITCSVSSSISSSNLHWYQQKPDQSPKLLIYGTSNLASGVPSRFSGSGSGTDFTLTINSL EAEDAATYYCQQWSSYPLTFGQGTKLEIKRTVAAPSVFIFPPSDEQLKSGTASVVCLLNNFYPREAKVQWKVDNALQ SGNSQESVTEQDSKDSTYSLSSTLTLSKADYEKHKVYACEVTHQGLSSPVTKSFNRGEC |

| **Molecule 5** | **R1116: TIGIT_VH_IL2/ TIGIT_VL_IL2RA/ PD-L1-Fab** |
|---|---|
| first polypeptide | @TIGIT_VH_IL2_Fc-Hole (SEQ ID NO.39) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGAPTSSSTKKT QLQLEHLLLDLQMILNGINNYKNPKLTRMLTFKFYMPKKATELKHLQCLEEELKPLEEVLNLAQSKNFHLRPRDLISNIN VIVLELKGSETTFMCEYADETATIVEFLNRWITFCQSIISTLTEPKSSDKTHTCPPCPAPELLGGPSVFLFPPKPKDTLMI SRTPEVTCVVVDVSHEDPEVKFNWYVDGVEVHNAKTKPREEQYNSTYRVVSVLTVLHQDWLNGKEYKCKVSNKAL PAPIEKTISKAKGQPREPQVYTLPPSREEMTKNQVSLTCRVKGFYPSDIAVEWESNGQPENNYKTTPPVLKSDGSFF LASKLTVDKSRWQQGNVFSCSVMHEALHNHYTQKSLSLSPGK |
| second polypeptide | @TIGIT _ VL_IL2RA (SEQ ID NO.40) |
| | DIQMTQSPSSLSASVGDRVTITCRASENIYSNLAWYQQKPGKSPKLLVYAASHLPDGVPSRFSGSGSGTDYSLTISSL QPEDFATYYCQHFWGTPRTFGQGTKLEIKGGGGSGGGGSGGGGSGGGGSGELCDDDPPEIPHATFKAMAYKEGT MLNCECKRGFRRIKSGSLYMLCTGNSSHSSWDNQCQCTSSATRNTTKQVTPQPEEQKERKTTEMQSPMQPVDQA SLPGHCREPPPWENEATERIYHFVVGQMVYYQCVQGYRALHRGPAESVCKMTHGKTRWTQPQLICTG |
| third polypeptide | @PD-L1_VH_CH1_Fc-Knob (SEQ ID NO.34) |
| fourth polypeptide | @PD-L1_VL_CL (SEQ ID NO.35) |

| **Molecule 6** | **R1117: TIGIT_VH_IL22RA_D1D2/ TIGIT_VL_IL22** / **PD-L1-Fab** |
|---|---|
| first polypeptide | @TIGIT_VH_IL22RA_D1D2_Fc-Hole (SEQ ID NO.41) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGTQSTHESLKP QRVQFQSRNFHNILQWQPGRALTGNSSVYFVQYKIMFSCSMKSSHQKPSGCWQHISCNFPGCRTLAKYGQRQWK NKEDCWGTQELSCDLTSETSDIQEPYYGRVRAASAGSYSEWSMTPRFTPWWETKIDEPKSSDKTHTCPPCPAPEL LGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCRVKGFYPSDIAVEWESNGQ PENNYKTTPPVLKSDGSFFLASKLTVDKSRWQQGNVFSCSVMHEALHNHYTQKSLSLSPGK |
| second polypeptide | @TIGIT_VL_IL22(SEQ ID NO.42) |
| | DIQMTQSPSSLSASVGDRVTITCRASENIYSNLAWYQQKPGKSPKLLVYAASHLPDGVPSRFSGSGSGTDYSLTISSL QPEDFATYYCQHFWGTPRTFGQGTKLEIKGGGGSGGGGSGGGGSGGGGSGAPISSHCRLDKSNFQQPYITNRTF MLAKEASLADNNTDVRLIGEKLFHGVSMSERCYLMKQVLNFTLEEVLFPQSDRFQPYMQEVVPFLARLSNRLSTCHI EGDDLHIQRNVQKLKDTVKKLGESGEIKAIGELDLLFMSLRNACI |
| third polypeptide | @PD-L1_VH_CH1_Fc-Knob (SEQ ID NO.34) |
| fourth polypeptide | @PD-L1_VL_CL (SEQ ID NO.35) |

| **Molecule 7** | **R1118: TIGIT_VH_IL22RA_D1/ TIGIT_VL_IL22** / **PD-L1-Fab** |
|---|---|
| first polypeptide | @TIGIT_VH_IL22RA_D1_Fc-Hole (SEQ ID NO.43) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGTQSTHESLKP QRVQFQSRNFHNILQWQPGRALTGNSSVYFVQYKIMFEPKSSDKTHTCPPCPAPELLGGPSVFLFPPKPKDTLMISR TPEVTCVVVDVSHEDPEVKFNWYVDGVEVHNAKTKPREEQYNSTYRVVSVLTVLHQDWLNGKEYKCKVSNKALPA PIEKTISKAKGQPREPQVYTLPPSREEMTKNQVSLTCRVKGFYPSDIAVEWESNGQPENNYKTTPPVLKSDGSFFLA SKLTVDKSRWQQGNVFSCSVMHEALHNHYTQKSLSLSPGK |
| second polypeptide | @TIGIT_VL_IL22(SEQ ID NO.42) |
| third polypeptide | @PD-L1_VH_CH1_Fc-Knob (SEQ ID NO.34) |
| fourth polypeptide | @PD-L1_VL_CL (SEQ ID NO.35) |

| **Molecule 8** | **R1119: TIGIT_VH_IL18R1_D1D2/ TIGIT_VL_IL18/ PD-L1-Fab** |
|---|---|
| first polypeptide | @TIGIT_VH_IL18R1_D1D2_Fc-Hole : (SEQ ID NO.44) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGCTSRPHITVV EGEPFYLKHCSCSLAHEIETTTKSWYKSSGSQEHVELNPRSSSRIALHDCVLEFWPVELNDTGSYFFQMKNYTQKW KLNVIRRNKHSCFTERQVTSKIVEVKKFFQITCENSYYQTLVNSTSLYKNCKKLLLENNKNPTIKKNAEFEDQGYYSCV HFLHHNGKLFNITKTFNITIVEDRSNIVEPKSSDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNGKEYKCKVSNKALPAPIEKTISKAKGQP REPQVYTLPPSREEMTKNQVSLTCRVKGFYPSDIAVEWESNGQPENNYKTTPPVLKSDGSFFLASKLTVDKSRWQQ GNVFSCSVMHEALHNHYTQKSLSLSPGK |
| second polypeptide | @TIGIT_VL_IL18(SEQ ID NO.45) |
| | DIQMTQSPSSLSASVGDRVTITCRASENIYSNLAWYQQKPGKSPKLLVYAASHLPDGVPSRFSGSGSGTDYSLTISSL QPEDFATYYCQHFWGTPRTFGQGTKLEIKGGGGSGGGGSGGGGSGGGGSGYFGKLESKLSVIRNLNDQVLFIDQ GNRPLFEDMTDSDCRDNAPRTIFIISMYKDSQPRGMAVTISVKCEKISTLSCENKIISFKEMNPPDNIKDTKSDIIFFQR SVPGHDNKMQFESSSYEGYFLACEKERDLFKLILKKEDELGDRSIMFTVQNED |
| third polypeptide | @PD-L1_VH_CH1_Fc-Knob (SEQ ID NO.34) |
| fourth polypeptide | @PD-L1_VL_CL (SEQ ID NO.35) |

| **Molecule 9** | **R1120: TIGIT_VH_IL18R1_D1D2D3/ TIGIT_VL_IL18/ PD-L1-Fab** |
|---|---|
| first polypeptide | @TIGIT_VH_IL18R1_D1D2D3_Fc-Hole (SEQ ID NO.46) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGCTSRPHITVV EGEPFYLKHCSCSLAHEIETTTKSWYKSSGSQEHVELNPRSSSRIALHDCVLEFWPVELNDTGSYFFQMKNYTQKW KLNVIRRNKHSCFTERQVTSKIVEVKKFFQITCENSYYQTLVNSTSLYKNCKKLLLENNKNPTIKKNAEFEDQGYYSCV HFLHHNGKLFNITKTFNITIVEDRSNIVPVLLGPKLNHVAVELGKNVRLNCSALLNEEDVIYWMFGEENGSDPNIHEEK EMRIMTPEGKWHASKVLRIENIGESNLNVLYNCTVASTGGTDTKSFIEPKSSDKTHTCPPCPAPELLGGPSVFLFPPK PKDTLMISRTPEVTCVVVDVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCRVKGFYPSDIAVEWESNGQPENNYKTTPPVL KSDGSFFLASKLTVDKSRWQQGNVFSCSVMHEALHNHYTQKSLSLSPGK |
| second polypeptide | @TIGIT_VL_IL18(SEQ ID NO.45) |
| third polypeptide | @PD-L1_VH, CH1_Fc-Knob (SEQ ID NO.34) |
| fourth polypeptide | @PD-L1_VL_CL (SEQ ID NO.35) |

| **Molecule 10** | **R1123: TIGIT_VH_IL21R / TIGIT_VL_IL21/ PD-L1-Fab** |
|---|---|
| first polypeptide | @TIGIT_VH_IL21R_Fc-Hole (SEQ ID NO.47) |
| | QVQLVQSGAEVKKPGSSVKVSCKASGYSFTSYWMNWVRQAPGQGLEWIGMIRPSDSETRLNQMFKDRVTITVDKS TSTAYMELSSLRSEDTAVYYCAGIHDYGHGAYWGQGTLVTVSSGGGGSGGGGSGGGGSGGGGSGCPDLVCYTDY LQTVICILEMWNLHPSTLTLTWQDQYEELKDEATSCSLHRSAHNATHATYTCHMDVFHFMADDIFSVQITDQSGQYS QECGSFLLAESIKPAPPFDVTVTFSGQYQISWRSDYEDPAFYMLKGKLQYELQYRNRGDPWAVSPRRKLISVDSRSV SLLPLEFRKDSSYELQVRAGPMPGSSYQGTWSEWSDPVIFQTQSEELKEEPKSSDKTHTCPPCPAPELLGGPSVFL FPPKPKDTLMISRTPEVTCVVVDVSHEDPEVKFNWYVDGVEVHNAKTKPREEQYNSTYRVVSVLTVLHQDWLNGKE YKCKVSNKALPAPIEKTISKAKGQPREPQVYTLPPSREEMTKNQVSLTCRVKGFYPSDIAVEWESNGQPENNYKTTP PVLKSDGSFFLASKLTVDKSRWQQGNVFSCSVMHEALHNHYTQKSLSLSPGK |
| second polypeptide | @ TIGIT_VL_IL21(SEQ ID NO.48) |
| | DIQMTQSPSSLSASVGDRVTITCRASENIYSNLAWYQQKPGKSPKLLVYAASHLPDGVPSRFSGSGSGTDYSLTISSL QPEDFATYYCQHFWGTPRTFGQGTKLEIKGGGGSGGGGSGGGGSGGGGSGHKSSSQGQDRHMIRMRQLIDIVD QLKNYVNDLVPEFLPAPEDVETNCEWSAFSCFQKAQLKSANTGNNERIINVSIKKLKRKPPSTNAGRRQKHRLTCPS CDSYEKKPPKEFLERFKSLLQKMIHQHLSSRTHGSEDS |
| third polypeptide | @PD-L1_VH_CH1_Fc-Knob (SEQ ID NO.34) |
| fourth polypeptide | @PD-L1_VL_CL (SEQ ID NO.35) |

### Example 3 Bispecific antibodies with disulfide-bond modification

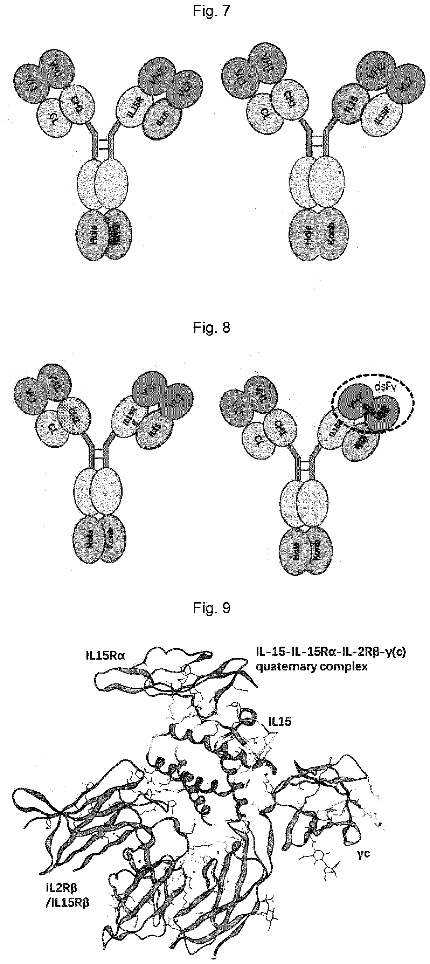

3.1 To further improve stability and extend a half-life period of bispecific antibodies, the bispecific antibodies were subjected to a disulfide-bond modification (see FIG. 8).

Disulfide-bond modification of ligand and receptor: VH targeting a second antibody (anti-TIGIT) was selected, linked to a receptor protein (IL15RA) through a Linker, and linked to Fc of an antibody through a Hinge; VL targeting to the second antibody was linked to a ligand protein (IL15) through a Linker; and the other end was an intact Fab structure targeting a first antibody, and the Fc making up the first antibody and the Fc making up the second antibody had a conventional KiH modification to avoid mismatching of heavy chains. Meanwhile, the receptor and ligand proteins were mutated to form an intermolecular disulfide bond, so as to further improve stability of molecules, specifically, for example:

| **Molecule No.** | **R0954: TIGIT_VH_IL15RA (D96+C97) /TIGIT_VL_IL15 (E87C) / PD-L1-Fab** |
|---|---|
| first polypeptide | @TIGIT_VH_IL15RA (D96+C97) _Fc-Knob (SEQ ID NO.13) |
| | QVQLESEGGLFKPTDTLTLTCTVSGSSLSSSYMSWVRQAPGKGLEWIGIIGSNGNTYYANWAKGR FTISKTSTTVELKITSPTTEDTATYFCARGGYRTSGMDPWGPGTLVTVSSGGGGSGGGGSGGGG SGGGGSGITCPPPMSVEHADIWVKSYSLYSRERYICNSGFKRKAGTSSLTECVLNKATNVAHWTT PSLKCIRDPALVHQRPAPPSTVTTAGVTPQPESLSPSGKEPAASSPSSNNTAATTAAIVPGSQLMP SKSPSTGTTEISSHESSHGTPSQTTAKNWELTASASHQPPGVYPQGHSDTTEPRGPTIKPCPPCK CPAPNLLGGPSVFIFPPKIKDVLMISLSPIVTCVVVDVSEDDPDVQISWFVNNVEVHTAQTQTHRED YNSTLRVVSALPIQHQDWMSGKEFKCKVNNKDLPAPIERTISKPKGSVRAPQVYVLPPPEEEMTK KQVTLWCMVTDFMPEDIYVEWTNNGKTELNYDNTEPVLDSDGSYFMYSDLRVEKKNWVERNSY SCSVVHEGLHNHHTTKSFSRTPGK |
| second polypeptide | @ TIGIT_VL_IL15 (E87C) (SEQ ID NO.14) |
| | DIVMTQTPASVEVAVGGTVTIKCQASQSISSYLNWYQQKPGQPPKLLIYDALKLASGVPSRFSGSG |
| | SGTEYTLTISGVESADAATYYCQQEHSVGNVDNVFGGGTEVVVKGGGGSGGGGSGGGGSGGG GSGNWVNVISDLKKIEDLIQSMHIDATLYTESDVHPSCKVTAMKCFLLELQVISLESGDASIHDTVEN LIILANNSLSSNGNVTESGCKCCEELEEKNIKEFLQSFVHIVQMFINTS |
| third polypeptide | @PD-L1_VH_CH1_Fc-Hole (SEQ ID NO.7) |
| fourth polypeptide | @PD-L1_VL_CL (SEQ ID NO.8) |

| **Molecule No.** | **R1085: TIGIT_VH_IL15RA (P67C) / TIGIT_VL_IL15 (E90C) / PD-L1-Fab** |
|---|---|
| first polypeptide | @TIGIT_VH_IL15RA (P67C) _Fc-Knob(SEQ ID NO.49) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYWGQGTLVTVSSGGGGSGGG GSGGGGSGGGGSGITCPPPMSVEHADIWVKSYSLYSRERYICNSGFKRKAGTSSLTECVLNKAT NVAHWTTPSLKCIRDCALVHQRPAPPSTVTTAGVTPQPESLSPSGKEPAASSPSSNNTAATTAAIV PGSQLMPSKSPSTGTTEISSHESSHGTPSQTTAKNWELTASASHQPPGVYPQGHSDTTEPKSSD KTHTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVYTLP PSREEMTKNQVSLTCRVKGFYPSDIAVEWESNGQPENNYKTTPPVLKSDGSFFLASKLTVDKSR WQQGNVFSCSVMHEALHNHYTQKSLSLSPGK |
| second polypeptide | @ TIGIT_VL_IL15 (E90C) (SEQ ID NO.50) |
| | DIQMTQSPSSLSASVGDRVTITCRASENIYSNLAWYQQKPGKSPKLLVYAASHLPDGVPSRFSGS GSGTDYSLTISSLQPEDFATYYCQHFWGTPRTFGQGTKLEIKGGGGSGGGGSGGGGSGGGGSG NWVNVISDLKKIEDLIQSMHIDATLYTESDVHPSCKVTAMKCFLLELQVISLESGDASIHNTVQDLIIL ANNSLSSNGNVTESGCKECECLEEKNIKEFLQSFVHIVQMFINTS |
| third polypeptide | @PD-L1_VH_CH1_Fc-Knob (SEQ ID NO.51) |
| | QVQLQESGPGLVKPSETLSLTCAVYGDSITSGYWNWIRKPPGKGLEYIGYISYTGSTYQNPSLKSR ITMSRDTSKNQYYLKLSSVTAADTAVYYCARSRAWIRTYFDYWGQGTLVTVSSASTKGPSVFPLAP SSKSTSGGTAALGCLVKDYFPEPVTVSWNSGALTSGVHTFPAVLQSSGLYSLSSVVTVPSSSLGT QTYICNVNHKPSNTKVDKRVEPKSCDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVS NKALPAPIEKTISKAKGQPREPQVYTLPPSREEMTKNQVSLWCLVKGFYPSDIAVEWESNGQPEN NYDTTPPVLDSDGSFFLYSDLTVDKSRWQQGNVFSCSVMHEALHNHYTQKSLSLSPGK |
| fourth polypeptide | @PD-L1_VL_CL (SEQ ID NO.52) |
| | DIQMTQSPSSLSASVGDRVTITCSVSSSISSSNLHWYQQKPGKAPKPWIYGTSNLASGVPSRFSG SGSGTDYTLTISSLQPEDFATYYCQQWSSYPLTFGQGTKLEIKRTVAAPSVFIFPPSDEQLKSGTAS VVCLLNNFYPREAKVQWKVDNALQSGNSQESVTEQDSKDSTYSLSSTLTLSKADYEKHKVYACE VTHQGLSSPVTKSFNRGEC |

| Molecule No. | **R1086: TIGIT_VH_IL15RA (R35C) / TIGIT_VL_IL15 (E93C) / PD-L1-Fab** |
|---|---|
| first polypeptide | @TIGIT_VH_IL15RA (R35C) _Fc-Knob(SEQ ID NO.65) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYWGQGTLVTVSSGGGGSGGG GSGGGGSGGGGSGITCPPPMSVEHADIWVKSYSLYSRERYICNSGFKCKAGTSSLTECVLNKAT NVAHWTTPSLKCIRDPALVHQRPAPPSTVTTAGVTPQPESLSPSGKEPAASSPSSNNTAATTAAIVP GSQLMPSKSPSTGTTEISSHESSHGTPSQTTAKNWELTASASHQPPGVYPQGHSDTTEPKSSDK THTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYTLPP SREEMTKNQVSLTCRVKGFYPSDIAVEWESNGQPENNYKTTPPVLKSDGSFFLASKLTVDKSRW QQGNVFSCSVMHEALHNHYTQKSLSLSPGK |
| second polypeptide | @ TIGIT_VL_IL15 (E93C) (SEQ ID NO.12) |
| | DIQMTQSPSSLSASVGDRVTITCRASENIYSNLAWYQQKPGKSPKLLVYAASHLPDGVPSRFSGS GSGTDYSLTISSLQPEDFATYYCQHFWGTPRTFGQGTKLEIKGGGGSGGGGSGGGGSGGGGSG NWVNVISDLKKIEDLIQSMHIDATLYTESDVHPSCKVTAMKCFLLELQVISLESGDASIHNTVQDLIIL ANNSLSSNGNVTESGCKECEELECKNIKEFLQSFVHIVQMFINTS |
| third polypeptide | @PD-L1_VH_CH1_Fc-Knob (SEQ ID NO.51) |
| fourth polypeptide | @PD-L1_VL_CL (SEQ ID NO.52) |

3.2 The disulfide-bond modification was designed between VH and VL to form dsFv to facilitate a covalent disulfide-bond linkage between non-covalently designed heavy and light chains of a receptor and a ligand. Disulfide-bond modification sites include, but are not limited to, the following mutation sites:

| Combination | VH | VL |
|---|---|---|
| 1 | 37C | 95C |
| 2 | 44C | 100C |
| 3 | 44C | 101C |
| 4 | 44C | 105C |
| 5 | 45C | 87C |
| 6 | 45C | 98C |
| 7 | 100C | 50C |
| 8 | 100bC | 49C |
| 9 | 98C | 46C |
| 10 | 101C | 46C |
| 11 | 105C | 43C |
| 12 | 106C | 57C |
| 13 | 108C | 43C |

Specifically, for example:

| Molecule No. | **R1081: TIGIT_VH (G44C)_IL15RA/ TIGIT_VL(Q100C)_IL15/ PD-L1-Fab** |
|---|---|
| first polypeptide | @TIGIT_VH (G44C)_IL15RA_Fc-Knob (SEQ ID NO.53) |
| | QVQLVQSGAEVKKPGSSVKVSCKASGYSFTSYWMNWVRQAPGQCLEWIGMIRPSDSETRLNQ MFKDRVTITVDKSTSTAYMELSSLRSEDTAVYYCAGIHDYGHGAYWGQGTLVTVSSGGGGSGG GGSGGGGSGGGGSGITCPPPMSVEHADIWVKSYSLYSRERYICNSGFKRKAGTSSLTECVLNK ATNVAHWTTPSLKCIRDPALVHQRPAPPSTVTTAGVTPQPESLSPSGKEPAASSPSSNNTAATTA AIVPGSQLMPSKSPSTGTTEISSHESSHGTPSQTTAKNWELTASASHQPPGVYPQGHSDTTEPK SSDKTHTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPREP QVYTLPPSREEMTKNQVSLTCRVKGFYPSDIAVEWESNGQPENNYKTTPPVLKSDGSFFLASKL TVDKSRWQQGNVFSCSVMHEALHNHYTQKSLSLSPGK |
| second polypeptide | @ TIGIT_VL (Q100C)_IL15 (SEQ ID NO.54) |
| | DIQMTQSPSSLSASVGDRVTITCRASENIYSNLAWYQQKPGKSPKLLVYAASHLPDGVPSRFSG SGSGTDYSLTISSLQPEDFATYYCQHFWGTPRTFGCGTKLEIKGGGGSGGGGSGGGGSGGGG SGNWVNVISDLKKIEDLIQSMHIDATLYTESDVHPSCKVTAMKCFLLELQVISLESGDASIHNTVQ DLIILANNSLSSNGNVTESGCKECEELEEKNIKEFLQSFVHIVQMFINTS |
| third polypeptide | @PD-L1_VH_CH1_Fc-Knob (SEQ ID NO.51) |
| fourth polypeptide | @PD-L1_VL_CL (SEQ ID NO.52) |

| **Molecule No.** | **R1082: TIGIT_VH (W108C)_IL15RA/ TIGIT_VL(S43C)_IL15/ PD-L1-Fab** |
|---|---|
| first polypeptide | @TIGIT_VH (W108C)_IL15RA_Fc-Knob (SEQ ID NO.55) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYCGQGTLVTVSSGGGGSGG GGSGGGGSGGGGSGITCPPPMSVEHADIWVKSYSLYSRERYICNSGFKRKAGTSSLTECVLNK ATNVAHWTTPSLKCIRDPALVHQRPAPPSTVTTAGVTPQPESLSPSGKEPAASSPSSNNTAATTA AIVPGSQLMPSKSPSTGTTEISSHESSHGTPSQTTAKNWELTASASHQPPGVYPQGHSDTTEPK |
| | SSDKTHTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPREP QVYTLPPSREEMTKNQVSLTCRVKGFYPSDIAVEWESNGQPENNYKTTPPVLKSDGSFFLASKL TVDKSRWQQGNVFSCSVMHEALHNHYTQKSLSLSPGK |
| second polypeptide | @ TIGIT_VL (S43C)_IL15 (SEQ ID NO.56) |
| | DIQMTQSPSSLSASVGDRVTITCRASENIYSNLAWYQQKPGKCPKLLVYAASHLPDGVPSRFSG SGSGTDYSLTISSLQPEDFATYYCQHFWGTPRTFGQGTKLEIKGGGGSGGGGSGGGGSGGGG SGNWVNVISDLKKIEDLIQSMHIDATLYTESDVHPSCKVTAMKCFLLELQVISLESGDASIHNTVQ DLIILANNSLSSNGNVTESGCKECEELEEKNIKEFLQSFVHIVQMFINTS |
| third polypeptide | @PD-L1_VH_CH1_Fc-Knob (SEQ ID NO.51) |
| fourth polypeptide | @PD-L1_VL_CL (SEQ ID NO.52) |

| **Molecule No.** | **R1083: TIGIT_VH (L45C) _IL15RA/ TIGIT_VL(F98C)_IL15/ PD-L1-Fab** |
|---|---|
| first polypeptide | @TIGIT_VH (L45C)_IL15RA_Fc-Knob (SEQ ID NO.57) |
| | QVQLVQSGAEVKKPGSSVKVSCKASGYSFTSYWMNWVRQAPGQGCEWIGMIRPSDSETRLN QMFKDRVTITVDKSTSTAYMELSSLRSEDTAVYYCAGIHDYGHGAYWGQGTLVTVSSGGGGSG GGGSGGGGSGGGGSGITCPPPMSVEHADIWVKSYSLYSRERYICNSGFKRKAGTSSLTECVLN KATNVAHWTTPSLKCIRDPALVHQRPAPPSTVTTAGVTPQPESLSPSGKEPAASSPSSNNTAATT AAIVPGSQLMPSKSPSTGTTEISSHESSHGTPSQTTAKNWELTASASHQPPGVYPQGHSDTTEP KSSDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQPRE PQVYTLPPSREEMTKNQVSLTCRVKGFYPSDIAVEWESNGQPENNYKTTPPVLKSDGSFFLASK LTVDKSRWQQGNVFSCSVMHEALHNHYTQKSLSLSPGK |
| second polypeptide | @ TIGIT_VL (F98C)_IL15 (SEQ ID NO.58) |
| | DIQMTQSPSSLSASVGDRVTITCRASENIYSNLAWYQQKPGKSPKLLVYAASHLPDGVPSRFSG SGSGTDYSLTISSLQPEDFATYYCQHFWGTPRTCGQGTKLEIKGGGGSGGGGSGGGGSGGGG SGNWVNVISDLKKIEDLIQSMHIDATLYTESDVHPSCKVTAMKCFLLELQVISLESGDASIHNTVQ DLIILANNSLSSNGNVTESGCKECEELEEKNIKEFLQSFVHIVQMFINTS |
| third polypeptide | @PD-L1_VH_CH1_Fc-Knob (SEQ ID NO.51) |
| fourth polypeptide | @PD-L1_VL_CL (SEQ ID NO.51) |

| **Molecule No.** | **R1084: PD-L1_VH (G44C)_IL15RA/ PD-L1_VL(Q101C)_IL15/TIGIT-Fab** |
|---|---|
| first polypeptide | @ PD-L1_VH (G44C)_IL15RA_Fc-Knob (SEQ ID NO.59) |
| | QVQLQESGPGLVKPSETLSLTCAVYGDSITSGYWNWIRKPPGKCLEYIGYISYTGSTYQNPSLKS RITMSRDTSKNQYYLKLSSVTAADTAVYYCARSRAWIRTYFDYWGQGTLVTVSSGGGGSGGGG SGGGGSGGGGSGITCPPPMSVEHADIWVKSYSLYSRERYICNSGFKRKAGTSSLTECVLNKAT NVAHWTTPSLKCIRDPALVHQRPAPPSTVTTAGVTPQPESLSPSGKEPAASSPSSNNTAATTAAI VPGSQLMPSKSPSTGTTEISSHESSHGTPSQTTAKNWELTASASHQPPGVYPQGHSDTTEPKS SDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQ VYTLPPSREEMTKNQVSLTCRVKGFYPSDIAVEWESNGQPENNYKTTPPVLKSDGSFFLASKLT VDKSRWQQGNVFSCSVMHEALHNHYTQKSLSLSPGK |
| second polypeptide | @ PD-L1_VL (Q101C)_IL15 (SEQ ID NO.60) |
| | DIQMTQSPSSLSASVGDRVTITCSVSSSISSSNLHWYQQKPGKAPKPWIYGTSNLASGVPSRFS GSGSGTDYTLTISSLQPEDFATYYCQQWSSYPLTFGCGTKLEIKGGGGSGGGGSGGGGSGGG GSGNWVNVISDLKKIEDLIQSMHIDATLYTESDVHPSCKVTAMKCFLLELQVISLESGDASIHNTV QDLIILANNSLSSNGNVTESGCKECEELEEKNIKEFLQSFVHIVQMFINTS |
| third polypeptide | @ TIGIT_VH_CH1_Fc-Knob (SEQ ID NO.61) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYWGQGTLVTVSSASTKGPSV FPLAPSSKSTSGGTAALGCLVKDYFPEPVTVSWNSGALTSGVHTFPAVLQSSGLYSLSSVVTVP SSSLGTQTYICNVNHKPSNTKVDKRVEPKSCDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLWCLVKGFYPSDIAVEW |
| | ESNGQPENNYDTTPPVLDSDGSFFLYSDLTVDKSRWQQGNVFSCSVMHEALHNHYTQKSLSLS PGK |
| fourth polypeptide | @ TIGIT_VL_CL (SEQ ID NO.62) |
| | DIQMTQSPSSLSASVGDRVTITCRASENIYSNLAWYQQKPGKSPKLLVYAASHLPDGVPSRFSG SGSGTDYSLTISSLQPEDFATYYCQHFWGTPRTFGQGTKLEIKRTVAAPSVFIFPPSDEQLKSGT ASVVCLLNNFYPREAKVQWKVDNALQSGNSQESVTEQDSKDSTYSLSSTLTLSKADYEKHKVY ACEVTHQGLSSPVTKSFNRGEC |

### Example 4 Mutation modification to eliminate glycosylation

Construction method of Fab-IL15/IL15RA_Fc: VH targeting a second antibody (anti-TIGIT) was specifically selected, linked to a receptor protein (IL15RA) through a Linker, and linked to Fc of an antibody through a Hinge; VL targeting to the second antibody was linked to a ligand protein (IL15) through a Linker; and the other end was an intact Fab structure targeting a first antibody, and the Fc making up the first antibody and the Fc making up the second antibody had a conventional KiH modification to avoid mismatching of heavy chains. Meanwhile, the receptor and ligand proteins were mutated to form an intermolecular disulfide bond, so as to further improve stability of molecules. Further, glycosylation sites on the receptor and ligand proteins were modified to eliminate molecular heterogeneity, specifically, for example:

| **Molecule No.** | **R1072: TIGIT_VH_IL15 RA/ TIGIT_VL_IL15/ PD-L1-Fab** |
|---|---|
| first polypeptide | @TIGIT_VH_IL15RA_Fc-Knob (SEQ ID NO.63) |
| | QVQLVQSGAEVKKPGSSVKVSCKASGYSFTSYWMNWVRQAPGQGLEWIGMIRPSDSETRLNQMFKDRVTITVDKSTSTA YMELSSLRSEDTAVYYCAGIHDYGHGAYWGQGTLVTVSSGGGGSGGGGSGGGGSGGGGSGITCPPPMSVEHADIWVKSY SLYSRERYICNSGFKRKAGTSSLTECVLNKATNVAHWTTPSLKCIRDPALVHQRPAPPSTVTTAGVTPQPESLSPSGKEPAASSP SSNNTAATTAAIVPGSQLMPSKSPSTGTTEISSHESSHGTPSQTTAKNWELTASASHQPPGVYPQGHSDTTEPKSSDKTHTC PPCPAPELLGGPSVFLFPPKPKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPSREEMTKNQVSLTCRVKGFYPSDIAVEWESNGQPEN NYKTTPPVLKSDGSFFLASKLTVDKSRWQQGNVFSCSVMHEALHNHYTQKSLSLSPGK |
| second polypeptide | @TIGIT_VL_IL15 (SEQ ID NO.64) |
| | DIQMTQSPSSLSASVGDRVTITCRASENIYSNLAWYQQKPGKSPKLLVYAASHLPDGVPSRFSGSGSGTDYSLTISSLQPEDFA TYYCQHFWGTPRTFGQGTKLEIKGGGGSGGGGSGGGGSGGGGSGNWVNVISDLKKIEDLIQSMHIDATLYTESDVHPSCK VTAMKCFLLELQVISLESGDASIHNTVQDLIILANNSLSSNGNVTESGCKECEELEEKNIKEFLQSFVHIVQMFINTS |
| third polypeptide | @PD-L1_VH_CH1_Fc-Knob (SEQ ID NO.51) |
| fourth polypeptide | @PD-L1_VL_CL (SEQ ID NO.52) |

| **Molecule No.** | **R0955: TIGIT_VH_IL15 RA/ TIGIT_VL_IL15 (N71Q, N79Q, N112Q) / PD-L1-Fab** |
|---|---|
| first polypeptide | @TIGIT_VH_IL15RA_Fc-Knob (SEQ ID NO.11) |
| | QVQLESEGGLFKPTDTLTLTCTVSGSSLSSSYMSWVRQAPGKGLEWIGIIGSNGNTYYANWAKGRFTISKTSTTVELKITSPTT EDTATYFCARGGYRTSGMDPWGPGTLVTVSSGGGGSGGGGSGGGGSGGGGSGITCPPPMSVEHADIWVKSYSLYSRERYI CNSGFKRKAGTCSLTECVLNKATNVAHWTTPSLKCIRDPALVHQRPAPPSTVTTAGVTPQPESLSPSGKEPAASSPSSNNTAA TTAAIVPGSQLMPSKSPSTGTTEISSHESSHGTPSQTTAKNWELTASASHQPPGVYPQGHSDTTEPRGPTIKPCPPCKCPAPN LLGGPSVFIFPPKIKDVLMISLSPIVTCVVVDVSEDDPDVQISWFVNNVEVHTAQTQTHREDYNSTLRVVSALPIQHQDWMS GKEFKCKVNNKDLPAPIERTISKPKGSVRAPQVYVLPPPEEEMTKKQVTLWCMVTDFMPEDIYVEWTNNGKTELNYDNTE PVLDSDGSYFMYSDLRVEKKNWVERNSYSCSVVHEGLHNHHTTKSFSRTPGK |
| second polypeptide | @TIGIT_VL_IL15 (N71Q, N79Q, N112Q) (SEQ ID N0.15) |
| | DIVMTQTPASVEVAVGGTVTIKCQASQSISSYLNWYQQKPGQPPKLLIYDALKLASGVPSRFSGSGSGTEYTLTISGVESADAA TYYCQQEHSVGNVDNVFGGGTEVVVKGGGGSGGGGSGGGGSGGGGSGNWVNVISDLKKIEDLIQSMHIDATLYTESDV HPSCKVTAMKCFLLELQVISCESGDASIHDTVENLIILAQNSLSSNGQVTESGCKECEELEEKNIKEFLQSFVHIVQMFIQTS |
| third polypeptide | @PD-L1_VH_CH1_Fc-Hole (SEQ ID NO.7) |
| fourth polypeptide | @PD-L1_VL_CL (SEQ ID NO.8) |

| **Molecule No.** | **R1109: TIGIT_VH_IL15 RA (sushi65) / TIGIT_VL_IL15/ PD-L1-Fab** |
|---|---|
| first polypeptide | @TIGIT_VH_IL15RA (sushi65) _Fc-Knob (SEQ ID NO.66) |
| | QVQLVQSGAEVKKPGSSVKVSCKASGYSFTSYWMNWVRQAPGQGLEWIGMIRPSDSETRLNQMFKDRVTITVDKSTSTA YMELSSLRSEDTAVYYCAGIHDYGHGAYWGQGTLVTVSSGGGGSGGGGSGGGGSGGGGSGITCPPPMSVEHADIWVKSY SLYSRERYICNSGFKRKAGTSSLTECVLNKATNVAHWTTPSLKCIREPKSSDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPPVLKSDGSFFLASKLTVDKSR WQQGNVFSCSVMHEALHNHYTQKSLSLSPGK |
| second polypeptide | @TIGIT_VL_IL15 (SEQ ID NO.64) |
| third polypeptide | @PD-L1_VH_CH1_Fc-Knob (SEQ ID NO.51) |

| fourth polypeptide | @PD-L1_VL_CL (SEQ ID NO.52 |
|---|---|
| **Molecule No.** | **R1110: TIGIT_VH_IL15 RA (sushi65 T2A) / TIGIT_VL_IL15 / PD-L1-Fab** |
| first polypeptide | @TIGIT_VH_IL15RA (sushi6S T2A) _Fc-Knob (SEQ ID NO.67) |
| | QVQLVQSGAEVKKPGSSVKVSCKASGYSFTSYWMNWVRQAPGQGLEWIGMIRPSDSETRLNQMFKDRVTITVDKSTSTA YMELSSLRSEDTAVYYCAGIHDYGHGAYWGQGTLVTVSSGGGGSGGGGSGGGGSGGGGSGIACPPPMSVEHADIWVKSY SLYSRERYICNSGFKRKAGTSSLTECVLNKATNVAHWTTPSLKCIREPKSSDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPPVLKSDGSFFLASKLTVDKSR WQQGNVFSCSVMHEALHNHYTQKSLSLSPGK |
| second polypeptide | @TIGIT_VL_IL15 (SEQ ID NO.64) |

| third polypeptide | @PD-L1_VH_CH1_Fc-Knob (SEQ ID NO.51) |
|---|---|
| fourth polypeptide | @PD-L1_VL_CL (SEQ ID NO.52) |
| **Molecule No.** | **R1111: TIGIT_VH_IL15 RA Csushi65 T2A, T81A, T86A) / TIGIT_VL_IL15 / PD-L1-Fab** |
| first polypeptide | @TIGIT_VH_IL15RA (sushi6S T2A, T81A, T86A) _Fc-Knob (SEQ ID NO.68) |
| | QVQLVQSGAEVKKPGSSVKVSCKASGYSFTSYWMNWVRQAPGQGLEWIGMIRPSDSETRLNQMFKDRVTITVDKSTSTA YMELSSLRSEDTAVYYCAGIHDYGHGAYWGQGTLVTVSSGGGGSGGGGSGGGGSGGGGSGIACPPPMSVEHADIWVKSY SLYSRERYICNSGFKRKAGTSSLTECVLNKATNVAHWTTPSLKCIRDPALVHQRPAPPSTVATAGVAPQPESLSPSGKEPAASSP SSNNTAATTAEPKSSDKTHTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYTLPPSREEMTKNQVSLTCRVK GFYPSDIAVEWESNGQPENNYKTTPPVLKSDGSFFLASKLTVDKSRWQQGNVFSCSVMHEALHNHYTQKSLSLSPGK |
| second polypeptide | @TIGIT_VL_IL15 (SEQ ID NO.64) |
| third polypeptide | @PD-L1_VH_CH1_Fc-Knob (SEQ ID NO.51) |
| fourth polypeptide | @PD-L1_VL_CL (SEQ ID NO.52) |

### Example 5 Modified IL15/IL15RA and bispecific antibody composed thereof with reduced affinity of IL15/IL15RA with IL2/15Rβ/γC complex

In certain applications, in order to avoid interaction of IL15/IL15RA and a bispecific antibody composed thereof with an IL2/15Rβ/γC complex, causing unwanted non-specific binding, the IL15/IL15RA and the bispecific antibody composed thereof were modified to reduce or completely lose an affinity of the IL15/IL15RA with the IL2/15Rβ/γC complex. By examining a crystal structure of an interaction interface of the IL15/IL15RA and the IL2/15Rβ/γC complex, and performing modeling using a Molecular Operating Environment (MOE; Chemical Computing Group, Montreal, Quebec, Canada) software, it was predicted that an amino acid mutation modification could be performed at an IL15/IL15RA interface so as to reduce or completely lose the affinity of the IL15/IL15RA with the IL2/15Rβ/γC complex, as depicted in FIG. 9.

Construction method of Fab-IL15/IL15RA_Fc: VH targeting a second antibody (anti-TIGIT) was specifically selected, linked to a receptor protein (IL15RA) through a Linker, and linked to Fc of an antibody through a Hinge; VL targeting to the second antibody was linked to a ligand protein (IL15) through a Linker; and the other end was an intact Fab structure targeting a first antibody, and the Fc making up the first antibody and the Fc making up the second antibody had a conventional KiH modification to avoid mismatching of heavy chains. At the same time, the ligand protein was mutated to reduce or inactivate biological functions of the receptor-ligand complex, specifically, for example:

| **Molecule No.** | **R0960: TIGIT_VH_IL15 RA/ TIGIT_VL_IL15** (**D61N, E64Q, N65D**) **/ PD-L1-Fab** |
|---|---|
| first polypeptide | @TIGIT_VH_IL15RA_Fc-Knob (SEQ ID NO.5) |
| | QVQLESEGGLFKPTDTLTLTCTVSGSSLSSSYMSWVRQAPGKGLEWIGIIGSNGNTYYANWAKGRFTISKTSTTVELKITSPTTEDTAT YFCARGGYRTSGMDPWGPGTLVTVSSGGGGSGGGGSGGGGSGGGGSGITCPPPMSVEHADIWVKSYSLYSRERYICNSGFKRKA GTSSLTECVLNKATNVAHWTTPSLKCIRDPALVHQRPAPPSTVTTAGVTPQPESLSPSGKEPAASSPSSNNTAATTAAIVPGSQLMPS KSPSTGTTEISSHESSHGTPSQTTAKNWELTASASHQPPGVYPQGHSDTTEPRGPTIKPCPPCKCPAPNLLGGPSVFIFPPKIKDVLMI SLSPIVTCVVVDVSEDDPDVQISWFVNNVEVHTAQTQTHREDYNSTLRVVSALPIQHQDWMSGKEFKCKVNNKDLPAPIERTISKP KGSVRAPQVYVLPPPEEEMTKKQVTLWCMVTDFMPEDIYVEWTNNGKTELNYDNTEPVLDSDGSYFMYSDLRVEKKNWVERNS YSCSVVHEGLHNHHTTKSFSRTPGK |
| second polypeptide | @TIGIT_VL_IL15 (D61N, E64Q, N65D) (SEQ ID NO.16) |
| | DIVMTQTPASVEVAVGGTVTIKCQASQSISSYLNWYQQKPGQPPKLLIYDALKLASGVPSRFSGSGSGTEYTLTISGVESADAATYYC QQEHSVGNVDNVFGGGTEVVVKGGGGSGGGGSGGGGSGGGGSGNWVNVISDLKKIEDLIQSMHIDATLYTESDVHPSCKVTA MKCFLLELQVISLESGDASIHNTVQDLIILANNSLSSNGNVTESGCKECEELEEKNIKEFLQSFVHIVQMFINTS |
| third polypeptide | @PD-L1_VH_CH1_Fc-Hole (SEQ ID NO.7) |
| fourth polypeptide | @PD-L1_VL_CL (SEQ ID NO.8) |

### Example 6 Construction of bispecific antibodies based on scFv and CrossMab structures as experimental control

As described above, both scFv and CrossMab were commonly used technological means for constructing bispecific antibodies, which were used as designed controls to compare with our molecules:
Construction method of bispecific antibody based on scFv structure: VH targeting a second antibody (anti-TIGIT) was specifically selected, linked to VL of the second antibody through a Linker to form an scFv structure, and linked to Fc of an antibody through a Hinge; and the other end was an intact Fab structure (this structure was similar to Y-Body of Wuhan YZY Med), and the Fc making up the first antibody and the Fc making up the second antibody had a conventional KiH modification to avoid mismatching of heavy chains. Specifically, for example:

| **Molecule No.** | **R0809: Y-Body TIGIT_scFv / PD-L1-Fab** (FIG.1-C) |
|---|---|
| scFv arm | @TIGIT_scFv_Fc-Knob (SEQ ID NO.17) |
| | QVQLESEGGLFKPTDTLTLTCTVSGSSLSSSYMSWVRQAPGKGLEWIGIIGSNGNTYYANWAKGRFTISKTSTTVELKITSPTTEDTAT YFCARGGYRTSGMDPWGPGTLVTVSSGGGGSGGGGSGGGGSDIVMTQTPASVEVAVGGTVTIKCQASQSISSYLNWYQQKPG QPPKLLIYDALKLASGVPSRFSGSGSGTEYTLTISGVESADAATYYCQQEHSVGNVDNVFGGGTEVVVKGAAAEPRGPTIKPCPPCK CPAPNLLGGPSVFIFPPKIKDVLMISLSPIVTCVVVDVSEDDPDVQISWFVNNVEVHTAQTQTHREDYNSTLRVVSALPIQHQDWM SGKEFKCKVNNKDLPAPIERTISKPKGSVRAPQVYVLPPPEEEMTKKQVTLWCMVTDFMPEDIYVEWTNNGKTELNYDNTEPVLD SDGSYFMYSDLRVEKKNWVERNSYSCSVVHEGLHNHHTTKSFSRTPGK |
| Fab arm-VH | @PD-L1_VH_CH1_Fc-Hole (SEQ ID NO.7) |
| Fab arm-VL | @PD-L1_VL_CL (SEQ ID NO.8) |

Construction method of bispecific antibody based on scFv structure: VH targeting a second antibody (anti-TIGIT) was specifically selected, linked to VL of the second antibody through a Linker to form an scFv structure, and linked to a C terminus of an intact Fc targeting a first antibody through a Linker to form a symmetric structure. Specifically, for example:

| **Molecule No.** | **R0810: Symmetric scFv PD-L1_VH_CH1_Fc_@TIGIT_scFv/PD-L1_VL_CL (**FIG. 1-D) |
|---|---|
| Heavy chain | @PD-L1_VH_CH1_Fc_@TIGIT_scFv (SFQ ID NO. 20) |
| | EVQLLESGGGLVQPGGSLRLSCAASGFTFSSYIMMWVRQAPGKGLEWVSSIYPSGGITFYADTVKG RFTISRDNSKNTLYLQMNSLRAEDTAVYYCARIKLGTVTTVDYWGQGTLVTVSSAKTTAPSVYPLAPV CGDTTGSSVTLGCLVKGYFPEPVTLTWNSGSLSSGVHTFPAVLQSDLYTLSSSVTVTSSTWPSQSIT CNVAHPASSTKVDKKIEPRGPTIKPCPPCKCPAPNLLGGPSVFIFPPKIKDVLMISLSPIVTCVVVDVSE DDPDVQISWFVNNVEVHTAQTQTHREDYNSTLRVVSALPIQHQDWMSGKEFKCKVNNKDLPAPIER TISKPKGSVRAPQVYVLPPPEEEMTKKQVTLTCMVTDFMPEDIYVEWTNNGKTELNYKNTEPVLDSD GSYFMYSKLRVEKKNWVERNSYSCSVVHEGLHNHHTTKSFSRTPGAGGGGSGGGGSGGGGSGG GGSQVQLESEGGLFKPTDTLTLTCTVSGSSLSSSYMSWVRQAPGKGLEWIGIIGSNGNTYYANWAK GRFTISKTSTTVELKITSPTTEDTATYFCARGGYRTSGMDPWGPGTLVTVSSGGGGSGGGGSGGG GSDIVMTQTPASVEVAVGGTVTIKCQASQSISSYLNWYQQKPGQPPKLLIYDALKLASGVPSRFSGS GSGTEYTLTISGVESADAATYYCQQEHSVGNVDNVFGGGTEVVVK |
| Light chain | @PD-L1_VL_CL (SEQ ID NO. 8) |

Construction method of bispecific antibody based on CrossMab structure: VH targeting a second antibody (anti-TIGIT) was specifically selected, linked to a CL domain, and linked to Fc of an antibody through a Hinge; VL targeting to the second antibody (anti-TIGIT) was linked to a CH1 domain to form a light chain; and the other end was an intact Fab structure targeting a first antibody, and the Fc making up the first antibody and the Fc making up the second antibody had a conventional KiH modification to avoid mismatching of heavy chains. Specifically, for example:

| **Molecule No.** | **R0959: CrossMab TIGIT_VH_CL/TIGIT_VL_CH1/PD-L1_Fab (**FIG. 1-B) |
|---|---|
| First polypeptide | @TIGIT_VH_CL_Fc-Knob (SEQ ID NO. 18) |
| | QVQLESEGGLFKPTDTLTLTCTVSGSSLSSSYMSWVRQAPGKGLEWIGIIGSNGNTYYANWAKG RFTISKTSTTVELKITSPTTEDTATYFCARGGYRTSGMDPWGPGTLVTVSSLRTDAAPTVSIFPPS SEQLTSGGASVVCFLNNFYPKDINVKWKIDGSERQNGVLNSWTDQDSKDSTYSMSSTLTLTKDE YERHNSYTCEATHKTSTSPIVKSFNRNECEPRGPTIKPCPPCKCPAPNLLGGPSVFIFPPKIKDVL MISLSPIVTCVVVDVSEDDPDVQISWFVNNVEVHTAQTQTHREDYNSTLRVVSALPIQHQDWMS GKEFKCKVNNKDLPAPIERTISKPKGSVRAPQVYVLPPPEEEMTKKQVTLWCMVTDFMPEDIYVE WTNNGKTELNYDNTEPVLDSDGSYFMYSDLRVEKKNWVERNSYSCSVVHEGLHNHHTTKSFS RTPGK |
| Second polypeptide | @TIGIT_VL_CH1 (SEQ ID NO. 19) |
| | DIVMTQTPASVEVAVGGTVTIKCQASQSISSYLNWYQQKPGQPPKLLIYDALKLASGVPSRFSGS GSGTEYTLTISGVESADAATYYCQQEHSVGNVDNVFGGGTEVVVKAKTTAPSVYPLAPVCGDTT GSSVTLGCLVKGYFPEPVTLTWNSGSLSSGVHTFPAVLQSDLYTLSSSVTVTSSTWPSQSITCNV AHPASSTKVDKKI |
| Third polypeptide | @PD-L1 VH CH1_Fc-Hole (SEQ ID NO. 7) |
| Fourth polypeptide | @PD-L1_VL_CL (SEQ ID NO. 8) |

### Example 7 Antibody heavy-light chain mismatching assay

Light-heavy chain mismatching was a difficult problem faced by a bispecific-antibody platform. In order to verify an anti-mismatching performance of the platform, a receptor and a ligand were specially designed to be distributed on Fab of two sides of an antibody, a mismatched light-chain structure was intentionally designed, and an expression verification was performed.

### Construction method of Fab-IL15/IL15RA_Fc mismatching:

R1042: VH targeting a first antibody (anti-PD-L1 antibody) was specifically selected, linked to a receptor protein (IL15RA) through a Linker, and linked to Fc of an antibody through a Hinge; VL targeting to a second antibody (anti-TIGIT antibody) was linked to a ligand protein (IL15) through a Linker; the other end was VH targeting the second antibody through a conventional sequence to be linked to CH1, and linked to Fc of an antibody through a Hinge; and VL targeting the first antibody was linked to CL through a conventional sequence, and both Fc had a conventional KiH modification to avoid mismatching of heavy chains. Specifically, for example:

| **Molecule No.** | **R1042: PD-L1_VH_IL15RA/TIGIT_VL_IL15/TIGIT_VH/PD-L1-VL (****FIG. 10** **left)** |
|---|---|
| First polypeptide | @PD-L1_VH_IL15RA_Fc-Knob (SEQ ID NO. 1) |
| Second polypeptide | @TIGIT_VL_IL15 (SEQ ID NO. 6) |
| Third polypeptide | @TIGIT_VH_CH1_Fc-Hole (SEQ ID NO. 3) |
| Fourth polypeptide | @PD-L1_VL_CL (SEQ ID NO. 8) |

R1043: VH targeting a second antibody (anti-TIGIT antibody) was specifically selected, linked to a receptor protein (IL15RA) through a Linker, and linked to Fc of an antibody through a Hinge; VL targeting to a first antibody (anti-PD-L1 antibody) was linked to a ligand protein (IL15) through a Linker; the other end was VH targeting the first antibody through a conventional sequence to be linked to CH1, and linked to Fc of an antibody through a Hinge; and VL targeting the second antibody was linked to CL through a conventional sequence, and both Fc had a conventional KiH modification to avoid mismatching of heavy chains.

| **Molecule No.** | **R1043: PD-L1_VH_IL15RA/TIGIT_VL_IL15/TIGIT_VH/PD-L1-VL** |
|---|---|
| First polypeptide | @TIGIT_VH_IL15RA_Fc-Knob (SEQ ID NO. 5) |
| Second polypeptide | @PD-L1_VL_IL15 (SEQ ID NO. 2) |
| Third polypeptide | @PD-L1_VH_CH1_Fc-Hole (SEQ ID NO. 7) |
| Fourth polypeptide | @TIGIT_VL_CL (SEQ ID NO. 4) |

### Construction method of Fab-IL21/IL21R_Fc mismatching:

VH targeting a second antibody (anti-PD-L1 antibody) was selected, linked to a receptor protein (IL21R) through a Linker, and linked to Fc of an antibody through a Hinge; VL targeting to a first antibody (anti-TIGIT antibody) was linked to a ligand protein (IL21) through a Linker; the other end was VL targeting the second antibody (anti-PD-L1 antibody) linked to CL; and VH targeting the first antibody (anti-TIGIT antibody) was linked to CH1, and linked to Fc of an antibody through a Hinge, and Fc on both ends had a conventional KiH modification. Specifically, for example:

| **Molecule No.** | **R1124: PD-L1_VH_IL21R/TIGIT_VL_IL21/TIGIT_VH/PD-L1-VL (****FIG. 10** **right)** |
|---|---|
| First polypeptide | @PD-L1_VH_IL21R_Fc-Knob (SEQ ID NO: 69) |
| | EVQLQESGPGLVKPSETLSLTCAVYGDSITSGYWNWIRKPPGKGLEYMGYISYTGSTYQNPSLKSRI TFSRDTSKNQYYLKLSSVTAADTATYYCARSRAWIRTYFDYWGQGTLVTVSSGGGGSGGGGSGGG GSGGGGSGCPDLVCYTDYLQTVICILEMWNLHPSTLTLTWQDQYEELKDEATSCSLHRSAHNATHAT YTCHMDVFHFMADDIFSVQITDQSGQYSQECGSFLLAESIKPAPPFDVTVTFSGQYQISWRSDYEDP AFYMLKGKLQYELQYRNRGDPWAVSPRRKLISVDSRSVSLLPLEFRKDSSYELQVRAGPMPGSSYQ GTWSEWSDPVIFQTQSEELKEEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNGKEYKCKVSNK ALPAPIEKTISKAKGQPREPQVYTLPPSREEMTKNQVSLTCRVKGFYPSDIAVEWESNGQPENNYKT TPPVLKSDGSFFLASKLTVDKSRWQQGNVFSCSVMHEALHNHYTQKSLSLSPG |
| Second polypeptide | @TIGIT_VL_IL21 (SEQ ID NO: 48) |
| Third polypeptide | @TIGIT_VH_CH1_Fc-Hole (SEQ ID NO. 70) |
| | QVQLVQSGAEVKKPGSSVKVSCKASGYSFTSYWMNWVRQAPGQGLEWIGMIRPSDSETRLNQMF KDRVTITVDKSTSTAYMELSSLRSEDTAVYYCAGIHDYGHGAYWGQGTLVTVSSASTKGPSVFPLAP SSKSTSGGTAALGCLVKDYFPEPVTVSWNSGALTSGVHTFPAVLQSSGLYSLSSVVTVPSSSLGTQT YICNVNHKPSNTKVDKRVEPKSCDKTHTCPPCPAPELLGGPSVFLFPPKPKDTLMISRTPEVTCVVV DVSHEDPEVKFNWYVDGVEVHNAKTKPREEQYNSTYRVVSVLTVLHQDWLNGKEYKCKVSNKALP APIEKTISKAKGQPREPQVYTLPPSREEMTKNQVSLWCLVKGFYPSDIAVEWESNGQPENNYDTTPP VLDSDGSFFLYSDLTVDKSRWQQGNVFSCSVMHEALHNHYTQKSLSLSPGK |
| Fourth polypeptide | @PD-L1_VL_CL (SEQ ID NO. 35) |

### Example 8 Bispecific antibodies with disulfide-bond modification

A disulfide-bond modification was designed between IL2 and IL2Rα (the amino acid position 75 in the amino acid sequence of wild-type interleukin 2 (SEQ ID NO.21) was mutated to cysteine; the N-terminal of the amino acid sequence of interleukin-2 receptor α (SEQ ID NO.22 or SEQ ID NO.23) is extended by two or three amino acids, in which the second amino acid is cysteine) to help form a covalent disulfide bond between non-covalent linked IL2 and IL2Rα.

A construction method of a bispecific antibody with a disulfide-bond modification (disulfide bond-modified IL2/IL2Rα complex) was exemplarily constructed: a heavy chain variable region (VH) of an antibody (such as TIGIT antibody) targeting a surface antigen of a tumor cell or an immune cell was linked to an IL2Rα mutant through a Linker, and linked to Fc of a hIgG1 antibody through a Hinge region; a light chain variable region (VL) of the antibody (TIGIT antibody) targeting the same antigen was linked to an IL2 mutant through a Linker; VH of an antibody (such as a PD-L1 antibody) targeting a surface antigen of an immune cell or a tumor cell was directly linked to a constant region (hlgG1) of the hlgG1 antibody; and VL of the antibody (PD-L1 antibody) targeting the same antigen was directly linked to a light-chain kappa antibody (κ-lg LC) of human immunoglobulin, so as to prepare a targeted disulfide bond-modified IL2/IL2Rα complex.

According to the above construction method, the inventor designed 2 disulfide bond-modified IL2/IL2Rα complexes, namely disulfide bond-modified IL2/IL2Rα complex 1 and disulfide bond-modified IL2/IL2Rα complex 2. IL2/IL2Rα complex 3 was the complex reported in the prior art and complex 4 was a disulfide bond-unmodified IL2/IL2Rα complex. Specific compositions and amino acid sequences of complexes 1, 2, 3, and 4 were as follows:
The sequence structure of the disulfide bond-modified IL2/IL2Rα complex 1 (sample R1262) was: TIGIT VH-IL2Rα mutant-Fc, TIGIT VL-IL2 mutant, PD-L1 VH-hlgG1, and PD-L1 VL-κ-lg LC, wherein the amino acid sequence of the TIGIT VH-IL2Rα mutant-Fc was shown in SEQ ID NO. 77, an amino acid sequence of the TIGIT VL-IL2 mutant was shown in SEQ ID NO. 78, the amino acid sequence of the PD-L1 VH-hlgG1 was shown in SEQ ID NO. 79, and the amino acid sequence of the PD-L1 VL-κ-lg LC was shown in SEQ ID NO. 80.

| **Molecule No.** | **R1262: TIGIT VH-IL2Rα mutant-Fc/TIGIT VL-IL2 mutant/PD-L1_VH-hIgG1/PD-LI VL-κ-Ig LC** |
|---|---|
| First polypeptide | @TIGIT VH-IL2Rα mutant-Fc (SEQ ID NO. 77) |
| | QVQLVQSGAEVKKPGSSVKVSCKASGYSFTSYWMNWVRQAPGQGLEWIGMIRPSDSETRLNQMFKDRV TITVDKSTSTAYMELSSLRSEDTAVYYCAGIHDYGHGAYWGQGTLVTVSSGGGGSGGGGSGGGGSGGG GCGELCDDDPPEIPHATFKAMAYKEGTMLNCECKRGFRRIKSGSLYMLCTGNSSHSSWDNQCQCTSSAT RNTTKQVTPQPEEQKERKTTEMQSPMQPVDQASLPGHCREPPPWENEATERIYHFVVGQMVYYQCVQG YRALHRGPAESVCKMTHGKTRWTQPQLICTGEPKSSDKTHTCPPCPAPELLGGPSVFLFPPKPKDTLMIS RTPEVTCVVVDVSHEDPEVKFNWYVDGVEVHNAKTKPREEQYNSTYRVVSVLTVLHQDWLNGKEYKCKV SNKALPAPIEKTISKAKGQPREPQVYTLPPSREEMTKNQVSLTCRVKGFYPSDIAVEWESNGQPENNYKTT PPVLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| | DIQMTQSPSSLSASVGDRVTITCRASENIYSNLAWYQQKPGKSPKLLVYAASHLPDGVPSRFSGSGSGTD YSLTISSLQPEDFATYYCQHFWGTPRTFGQGTKLEIKGGGGSGGGGSGGGGSGGGGSGAPTSSSTKKTQ |
| | LQLEHLLLDLQMILNGINNYKNPKLTRMLTFKFYMPKKATELKHLQCLEEELKPLEEVLNLAQCKNFHLRPR DLISNINVIVLELKGSETTFMCEYADETATIVEFLNRWITFCQSIISTLT |
| Third polypeptide | @PD-L1 VH-hIgG1 (SEQ ID NO. 79) |
| | EVQLQESGPGLVKPSETLSLTCAVYGDSITSGYWNWIRKPPGKGLEYMGYISYTGSTYQNPSLKSRITFSR DTSKNQYYLKLSSVTAADTATYYCARSRAWIRTYFDYWGQGTLVTVSSASTKGPSVFPLAPSSKSTSGGTA ALGCLVKDYFPEPVTVSWNSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQPREPQVYTLP PSREEMTKNQVSLWCLVKGFYPSDIAVEWESNGQPENNYDTTPPVLDSDGSFFLYSDLTVDKSRWQQGN VFSCSVMHEALHNHYTQKSLSLSPGK |
| Fourth polypeptide | @PD-L1_VL-κ-Ig LC (SEQ ID NO. 80) |
| | EIVLTQSPDFQSVTPKEKVTITCSVSSSISSSNLHWYQQKPDQSPKLLIYGTSNLASGVPSRFSGSGSGTDF TLTINSLEAEDAATYYCQQWSSYPLTFGQGTKLEIKRTVAAPSVFIFPPSDEQLKSGTASVVCLLNNFYPRE AKVQWKVDNALQSGNSQESVTEQDSKDSTYSLSSTLTLSKADYEKHKVYACEVTHQGLSSPVTKSFNRG EC |

The sequence structure of the disulfide bond-modified IL2/IL2Rα complex 2 was: TIGIT VH-IL2 mutant-Fc, TIGIT VL-IL2Rα mutant, PD-L1 VH-hlgG1, and PD-L1 VL-κ-Ig LC, wherein an amino acid sequence of the TIGIT VH-IL2 mutant-Fc was shown in SEQ ID NO. 81, an amino acid sequence of the TIGIT VL-IL2Rα mutant was shown in SEQ ID NO. 82, an amino acid sequence of the PD-L1 VH-hlgG1 was shown in SEQ ID NO. 79, and an amino acid sequence of the PD-L1 VL-κ-Ig LC was shown in SEQ ID NO. 80.

| **Molecule No.** | **Disulfide bond-modified IL2/IL2Rα complex 2: TIGIT VH-IL2Rα mutant-Fc/TIGIT VL-IL2 mutant/PD-L1 VH-hIgG1/PD-L1 VL-κ-Ig LC** |
|---|---|
| First polypeptide | @T IGIT VH-IL2 mutant-Fc (SEQ ID NO. 81) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYWGQGTLVTVSSGGGGSGGG GSGGGGSGGGGSGAPTSSSTKKTQLQLEHLLLDLQMILNGINNYKNPKLTRMLTFKFYMPKKAT ELKHLQCLEEELKPLEEVLNLAQCKNFHLRPRDLISNINVIVLELKGSETTFMCEYADETATIVEFLN RWITFCQSIISTLTEPKSSDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKALPAPI EKTISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @T TIGIT VL-IL2Rα mutant (SEQ ID NO. 82) |
| | DIQMTQSPSSLSASVGDRVTITCRASENIYSNLAWYQQKPGKSPKLLVYAASHLPDGVPSRFSGS GSGTDYSLTISSLQPEDFATYYCQHFWGTPRTFGQGTKLEIKGGGGSGGGGSGGGGSGGGGC GELCDDDPPEIPHATFKAMAYKEGTMLNCECKRGFRRIKSGSLYMLCTGNSSHSSWDNQCQCT SSATRNTTKQVTPQPEEQKERKTTEMQSPMQPVDQASLPGHCREPPPWENEATERIYHFVVGQ MVYYQCVQGYRALHRGPAESVCKMTHGKTRWTQPQLICTG |
| Third polypeptide | @P D-L1_VH-hIgG1 (SEQ ID NO. 79) |
| Fourth polypeptide | @P D-L1_VL-κ-Ig LC (SEQ ID NO. 80) |

The sequence structure of IL2/IL2Rα complex 3 was: TIGIT VH-IL2Rα(L42C)-Fc, TIGIT VL-IL2(F42C), PD-L1 VH-hlgG1, and PD-L1 VL-κ-Ig LC, wherein the amino acid sequence of the TIGIT VH-IL2Rα(L42C)-Fc was shown in SEQ ID NO. 83, the amino acid sequence of the TIGIT VL-IL2(F42C) was shown in SEQ ID NO. 84, the amino acid sequence of the PD-L1 VH-hlgG1 was shown in SEQ ID NO. 79, and the amino acid sequence of the PD-L1 VL-κ-Ig LC was shown in SEQ ID NO. 80. IL2Rα(L42C): the position 42 of leucine in the amino acid sequence of IL2Rα was mutated to cysteine, and IL2(F42C): the position 42 of phenylalanine in the amino acid sequence of IL2 was mutated to cysteine.

| **Molecule No.** | **Disulfide bond-modified IL2/IL2R α complex 3: TIGIT VH-IL2Rα (L42C)-Fc/TIGIT VL-IL2(F42C)/PD-L1 VH-hlgG1/PD-LI VL-κ-Ig LC** |
|---|---|
| First polypeptide | @TIGIT VH-IL2Ra(L42C)-Fc (SEQ ID NO. 83) |
| | QVQLVQSGAEVKKPGSSVKVSCKASGYSFTSYWMNWVRQAPGQGLEWIGMIRPSDSETRLNQ MFKDRVTITVDKSTSTAYMELSSLRSEDTAVYYCAGIHDYGHGAYWGQGTLVTVSSGGGGSGGG GSGGGGSGGGGSGELCDDDPPEIPHATFKAMAYKEGTMLNCECKRGFRRIKSGSCYMLCTGN SSHSSWDNQCQCTSSATRNTTKQVTPQPEEQKERKTTEMQSPMQPVDQASLPGHCREPPPW ENEATERIYHFVVGQMVYYQCVQGYRALHRGPAESVCKMTHGKTRWTQPQLICTGEPKSSDKT HTCPPCPAPELLGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQVYTLP PSREEMTKNQVSLTCRVKGFYPSDIAVEWESNGQPENNYKTTPPVLKSDGSFFLASKLTVDKSR WQQGNVFSCSVMHEALHNHYTQKSLSLSPGK |
| Second polypeptide | OTIGIT VL-IL2(F42C) (SEQ ID NO. 84) |
| | DIQMTQSPSSLSASVGDRVTITCRASENIYSNLAWYQQKPGKSPKLLVYAASHLPDGVPSRFSGS GSGTDYSLTISSLQPEDFATYYCQHFWGTPRTFGQGTKLEIKGGGGSGGGGSGGGGSGGGGS GAPTSSSTKKTQLQLEHLLLDLQMILNGINNYKNPKLTRMLTCKFYMPKKATELKHLQCLEEELKP LEEVLNLAQSKNFHLRPRDLISNINVIVLELKGSETTFMCEYADETATIVEFLNRWITFCQSIISTLT |
| Third polypeptide | @PD-L1 VH-hIgG1 (SEQ ID NO. 79) |
| Fourth polypeptide | @PD-L1 VL-κ-Ig LC (SEQ ID NO. 80) |

### Example 9 Preparation of FiBody sample

### Transient expression of proteins:

After a plasmid containing a target gene formed a cationic complex with a transfection reagent PEI, the cationic complex was introduced into a host cell Expi293, and an exogenous gene on the plasmid was transcribed and translated in the cell during the plasmid was in the cell, so as to obtain a target protein.

The Expi293 cells were cultured at 37°C in 8% carbon dioxide at 130 rpm and counted before transfection, and 2E6 cells were inoculated into a 1-L shake flask at a culture system of about 300 mL. Preparation of transfection complex for transfection: firstly, 750 µg target plasmid was added into a 50-mL centrifuge tube containing 15 mL Opti-MEM reagent, and the mixture was gently mixed uniformly and marked as tube A; 1.5 mg transfection reagent PEI was added into a 50-mL centrifuge tube containing 15 mL Opti-MEM reagent, and the mixture was gently mixed uniformly, incubated at room temperature for 5 min, and marked as tube B; and the PEI diluent of the B tube was dropwise added the into the DNA diluent of the tube A, the mixture was gently mixed uniformly, and incubated at room temperature for 15 min, the PEI-target plasmid complex was added into the Expi293 cells after the incubation was finished, and the cells were placed in a shaking table at 37°C for a continuous culture. The sample was collected until D7-D10.

### Protein purification:

The supernatant of the transient cell expression solution was collected after 9000rpm/20min centrifugation, and then filtered by 0.22µm filter membrane. A purification was performed with ProA affinity chromatography. The process was as follows: an AKTA avant 150 chromatography device was used, a chromatography column (e.g., MabSelectSuRe LX, GE) was equilibrated with at least 5 CV of an equilibration buffer (10 mM PBS), the sample was loaded onto a chromatographic column, such that the target protein was adsorbed onto the chromatographic column, and other impurities passed through to be separated. After the sample was loaded, the chromatographic column was washed again with at least 5 CV of an equilibration buffer (10 mM PBS), then the target protein was eluted with an elution buffer (20 mM NaAc, pH = 3.4), and the neutralizing buffer (1 M Tris, pH 8.0) was pre-added into a collection tube, wherein the volume of the added neutralizing buffer was determined based on the estimated content of the eluted sample, generally 10% of the elution volume.

### Example 10 Physical and chemical detection of FiBody

The samples were subjected to a one-step purification, then the purities were detected by HPLC-SEC (analytical column TOSOH, TSKgel G2000), and the expression level and purity of each sample were shown in the following table.

| Type of bispecific antibody | Number of samples | Expression level mg/L | Purity (%) |
|---|---|---|---|
| FiBody-A | R0950 | 25.1 | 89.2 |
| | R0951 | 16.1 | 90.8 |
| | R0952 | 14.6 | 84.7 |
| | R1072 | 122.1 | 91.7 |
| | R1109 | 70.2 | 98.4 |
| | R1115 | 83.9 | |
| | R1116 | 89.3 | 94.0 |
| FiBody-C | R1119 | 22.9 | 95.3 |
| | R1120 | 18.0 | 50.7 |
| FiBody-D | R1123 | 49.6 | 92.9 |
| FiBody-disulfide bond-modified | R0954 | 17.1 | 91.0 |
| | R1085 | 99.6 | 90.4 |
| | R1086 | 66.13 | 56.1 |
| FiBody-glycosylation modified | R0955 | 7.6 | 90.9 |
| | R1110 | 98.4 | 98.6 |
| | R1111 | 107.6 | 92.4 |
| FiBody-affinity modified | R0960 | 17.0 | 90.4 |
| ScFv-asymmetric (Y-Body) | R0809 | 1.5 | 36.8 |
| ScFv-symmetric | R0810 | 100 | 68.9 |
| CrossMab | R0959 | 1.4 | 41.4 |
| FiBody-mismatching | R1042 | 12.9 | 41.9 |
| | R1043 | 19.5 | 86.8 |
| | R1124 | 14.5 | 65.6 |

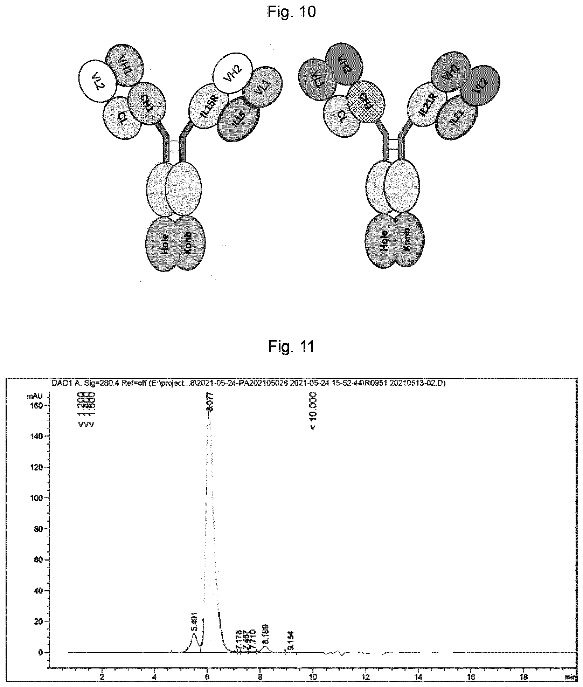

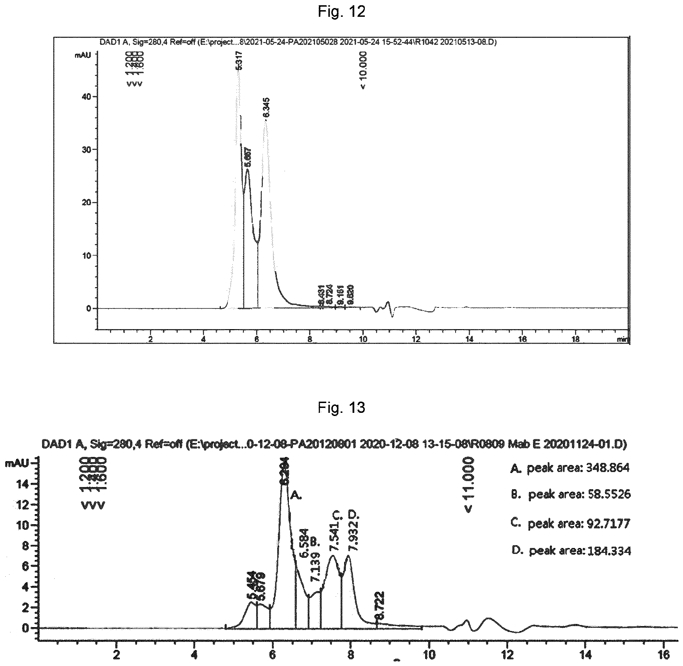

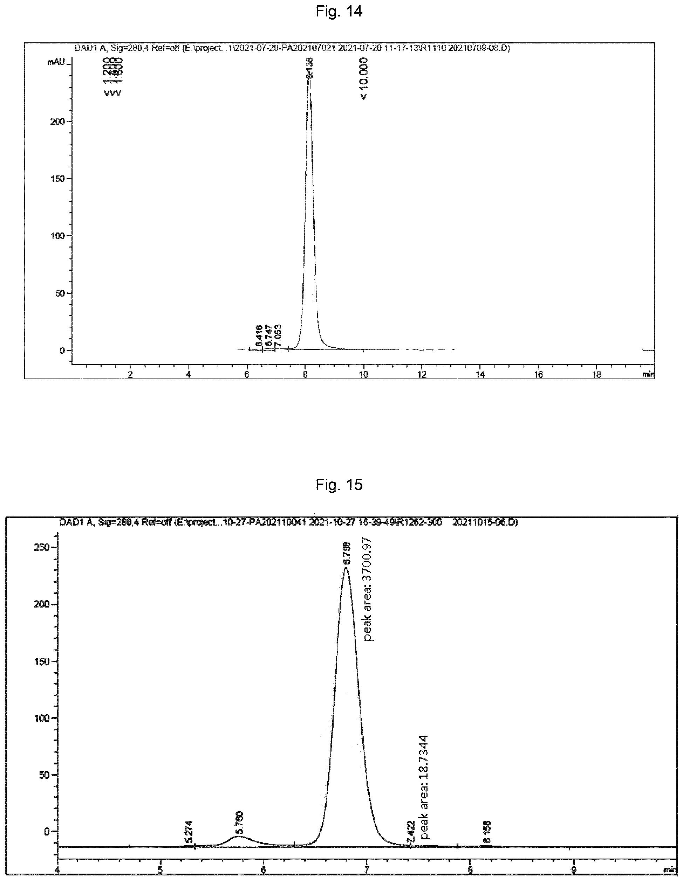

An HPLC-SEC detection result of the sample R0951 was shown in FIG. 11, an HPLC-SEC detection result of the sample R1042 was shown in FIG. 12, an HPLC-SEC detection result of the sample R0809 was shown in FIG. 13, and an HPLC-SEC detection result of the sample R1110 was shown in FIG. 14.

The results showed that bispecific antibodies (including various modified optimized antibodies) prepared by the FiBody platform had higher expression level and/or higher purity than those with asymmetric scFv (Y-Body and R0809), symmetric scFv (R0810), and CrossMab (R0959) structures.

Surprisingly, the bispecific antibodies with a mismatching pattern (samples R1042, R1043, and R1124) were also expressed and had the expression level similar to that of the normal molecule, but had a significantly lower purity.

The HPLC-SEC detection result of R1262 was shown in FIG. 15 and indicated that free light chains were successfully removed, and the purity of the disulfide bond-modified IL2/IL2Rα compound 1 prepared by the present disclosure was high.

### Example 11 Detection of antigen affinity of FiBody

### Analysis of binding activity of TIGIT end

A binding activity of a bispecific antibody molecule (TIGIT end) to CHO-TIGIT cells was detected by an FCM experimental method. Preparation of 3% BSA buffer: 4.5 g BSA was weighed into 150 mL 1XPBS and mixed uniformly, and the mixture was placed on ice for later use; antibody dilution: the tested antibody and the positive control were diluted to an initial concentration of 800 nM with 3% BSA, the subtype control was diluted to an initial concentration of 20 µg/mL at a volume of 300 µL, and 10 points were obtained by 3 times of gradient dilution (100+200); detection of binding activity: counting and plate-spreading of cells: after the R0254-3 cells were counted, the cells were distributed into 96-well V-shaped plates according to100 µL 2E+05/well; 50 µL antibody with different concentrations was firstly added into the cells, the cells were incubated at 2-8°C for 0.5 h, then 50 µL ligand was added, and the cells were incubated at 2-8°C for 0.5 h; the cells were centrifuged at 350xg for 5 min, the supernatant was removed, and 200 µL/well of 3% BSA was added; the cells were centrifuged at 350xg for 5 min, the supernatant was removed, fluorescent antibodies PE Goat anti-human IgG Fc and PE Goat anti-mouse IgG Fc (diluted at 1:500x) were prepared with 3% BSA, the antibodies were added into a corresponding 96-well plate according to 100 µL/well, and the cells were incubated at 2-8°C for 30 min; the cells were centrifuged at 350 g for 5 min, a supernatant was removed, and the cells were washed once with 3% BSA; after the cells were centrifuged at 350xg for 5 min, the supernatant was removed, and 1XPBS was added to resuspend the cells at 100 µL/well; and the resuspended cells were detected according to a standard operation procedure of a CytoFLEX flow cytometer.

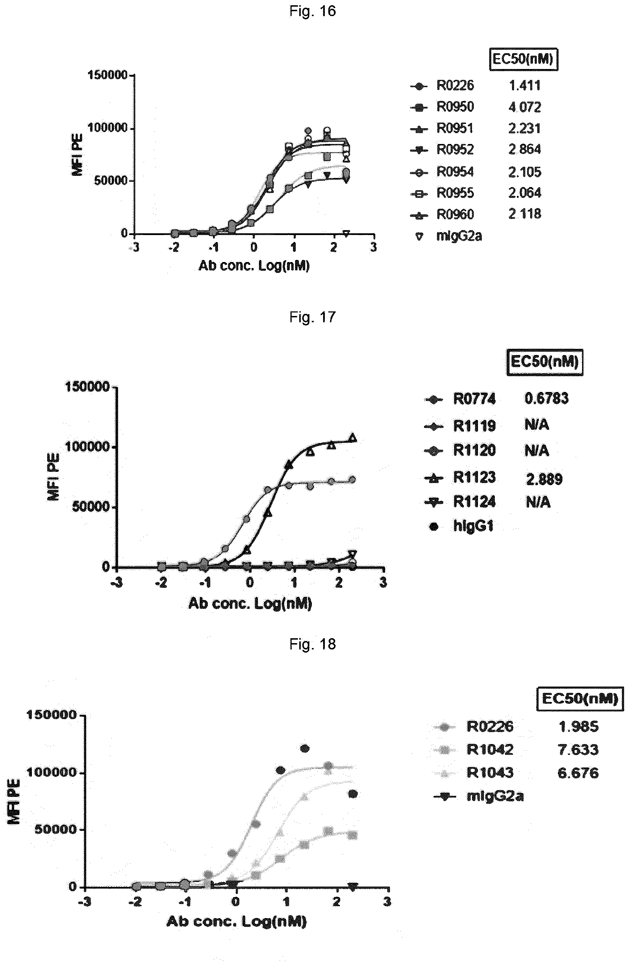

Results of R0950, R0951, R0952, R0954, R0955, R0960, R1123/R1119/R1120/R1124, R1042/R1043, and R0810 were shown in FIGs. 16-19. Surprisingly, the binding activity of R0950 (@TIGIT at Fab terminal) was lower than that of R0951-R0960 (@TIGIT was at IL15/IL15R terminal); and the binding activity of R0951-R0960 was similar to that of the positive control R0226(Tigit monoclonal antibody, OMP-313R12, WO2016191643). After CH1 and CL of A-type and D-type interleukins and receptors thereof were replaced, a binding force of the target region was not affected and the equivalent affinity was shown compared to the positive control (R0226\R0774(the VH sequence was shown in SEQ ID NO: 73, and the VL sequence was shown in SEQ ID NO: 74) and Tigit monoclonal antibody). After CH1 and CL of C-type molecules were replaced, the target region was affected, and the binding force of the target region was obviously lower than that of the positive control (R0226\R0774 and Tigit monoclonal antibody).

Modified molecules of disulfide bond-modified samples R1081 and R1085 and the glycosylated sample had an affinity result of the tigit terminal equivalent to that of the molecules before the modification.

R1042, R1043, and R1124 were mismatched test molecules, and had an obviously reduced TIGIT binding activity. R0810 was an ScFv structural molecule and had the binding activity weaker than that of the control molecule R0226.

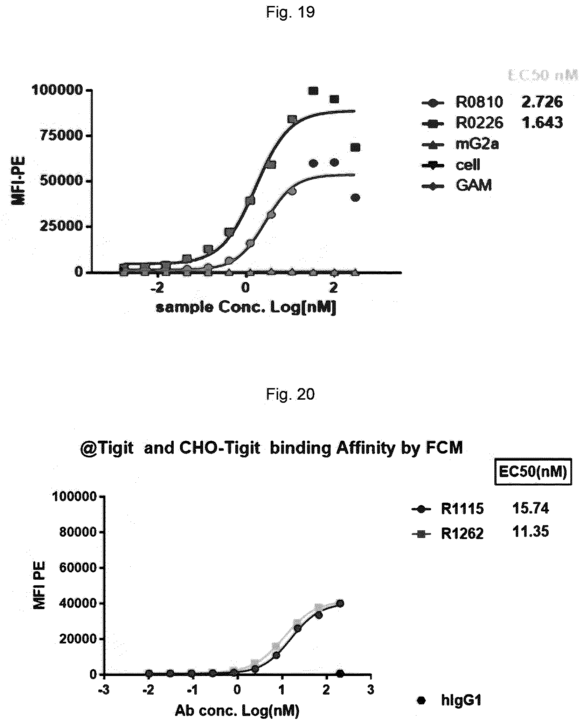

The result of R1262 was shown in FIG. 20 and showed that the disulfide bond-modified IL2/IL2Rα complex 1 (R1262) had an affinity equivalent to that of the disulfide bond-unmodified IL2/IL2Rα complex (R1115), indicating that the disulfide-bond modification did not affect the affinity of the target region.

### Analysis of binding activity of PD-L1 terminal

A binding activity of a double-antibody molecule (PD-L1 end) and CHO-PD-L1 cells was detected by an FCM experiment method. Preparation of 3% BSA buffer: 4.5 g BSA was weighed into 150 mL 1XPBS and mixed uniformly, and the mixture was placed on ice for later use; antibody dilution: the tested antibody and the positive control were diluted to an initial concentration of 800 nM with 3% BSA, the subtype control was diluted to an initial concentration of 20 µg/mL at a volume of 300 µL, and 10 points were obtained by 3 times of gradient dilution (100+200); detection of binding activity: cells were counted and spread on plates: after the R0254-3 cells were counted, the cells were distributed into 96-well V-shaped plates according to 100 µL 2E+05/well; 50 µL antibody with different concentrations was firstly added into the cells, the cells were incubated at 2-8°C for 0.5 h, then 50 µL ligand was added, and the cells were incubated at 2-8°C for 0.5 h; the cells were centrifuged at 350xg for 5 min, the supernatant was removed, and 200 µL/well of 3% BSA was added; the cells were centrifuged at 350xg for 5 min, the supernatant was removed, fluorescent antibodies PE Goat anti-human IgG Fc and PE Goat anti-mouse IgG Fc (diluted at 1:500x) were prepared with 3% BSA, the antibodies were added into a corresponding 96-well plate according to 100 µL/well, and the cells were incubated at 2-8°C for 30 min; the cells were centrifuged at 350 g for 5 min, the supernatant was removed, and the cells were washed once with 3% BSA; after the cells were centrifuged at 350xg for 5 min, the supernatant was removed, and 1XPBS was added to resuspend the cells at 100 µL/well; and the resuspended cells were detected according to a standard operation procedure of a CytoFLEX flow cytometer.

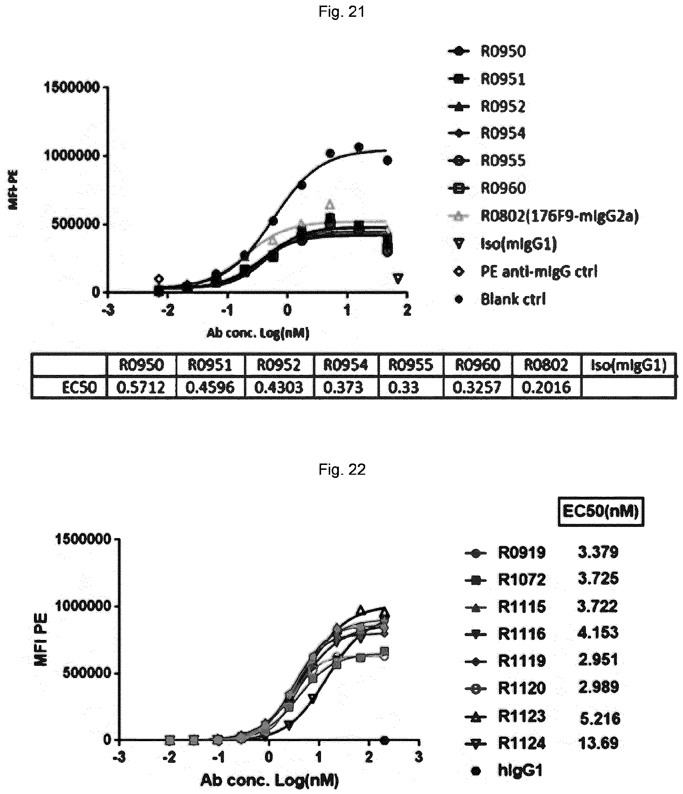

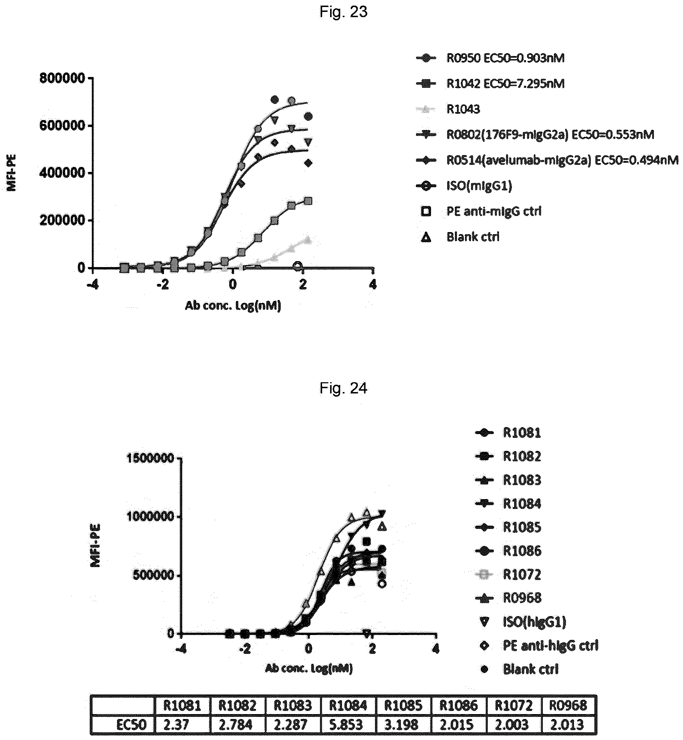

Results of R0950, R0951, R0952, R0954, R0955, R0960, R1072, R1115-R1120 and R1123-R1124; R0950, R1042 and R104; R1072 and R1081-R1086; R1072 and R1109-R1111 were shown in FIG. 21-FIG. 25. Surprisingly, the activity of @PD-L1 at an IL15 end was better than that at a Fab end. R1042, R1043, and R1124 were corresponding mismatched molecules with a significantly weaker activity, and other FiBody all showed an equivalent affinity to that of a positive antibody (PD-L1 monoclonal antibody). R0802 was a PD-L1 monoclonal antibody, 176F9. The VH sequence was shown in SEQ ID NO: 36, the VL sequence was shown in SEQ ID NO: 37, R0514 was a PD-L1 monoclonal antibody, Avelumab, R0919 was a PD-L1 monoclonal antibody, the VH sequence was shown in SEQ ID NO: 75, the VL sequence was shown in SEQ ID NO: 76, and R0968 was a PD-L1 monoclonal antibody, the VH sequence was shown in SEQ ID NO: 71, and the VL sequence was shown in SEQ ID NO: 72.

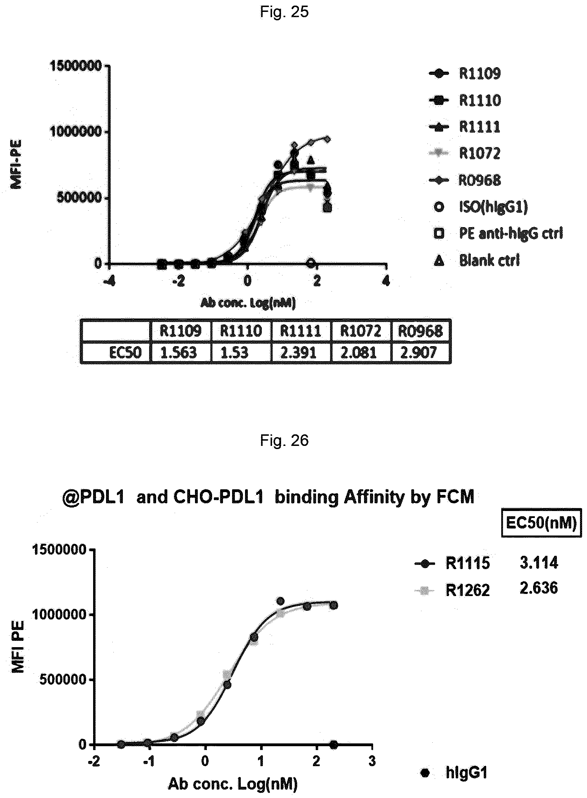

The result of R1262 was shown in FIG. 26 and showed that the disulfide bond-modified IL2/IL2Rα complex 1 (R1262) had an affinity equivalent to that of the antibody of the disulfide bond-unmodified IL2/IL2Rα complex 4 (R1115), indicating that the disulfide-bond modification did not affect the affinity of the target region.

### Analysis of binding blockade of TIGIT end

A binding activity of a bispecific-antibody molecule (TIGIT end) blockade ligand and CHO-TIGIT cells was detected by an FCM experiment method. Preparation of 3% BSA buffer: 4.5 g BSA was weighed into 150 mL 1×PBS and mixed uniformly, and the mixture was placed on ice for later use; antibody dilution: the tested antibody (R1262) and the positive control antibody (R1115) were diluted to an initial concentration of 800 nM with 3% BSA, and the subtype control antibody (hlgG1 antibody) was diluted to an initial concentration of 20 µg/mL at a volume of 300 µL with 3% BSA. 10 concentrations were obtained by 3 times of gradient dilution (100 µL+200 µL); counting and plate-spreading of cells: after the R0254-3 cells were counted, the cells were distributed into 96-well V-shaped plates according to 100 µL 2E+05/well; 50 µL of an antibody with different concentrations was firstly added into the cells, the cells were incubated at 2-8°C for 0.5 h, then 50 µL of a ligand was added, and the cells were incubated at 2-8°C for 0.5 h; the cells were centrifuged at 350×g for 5 min, the supernatant was removed, and 200 µL/well of 3% BSA was added; the cells were centrifuged at 350×g for 5 min, the supernatant was removed, fluorescent antibodies PE Goat anti-human IgG Fc and PE Goat anti-mouse IgG Fc (diluted at 1:500x) were prepared with 3% BSA, the antibodies were added into a corresponding 96-well plate according to 100 µL/well, and the cells were incubated at 2-8°C for 30 min; the cells were centrifuged at 350 g for 5 min, the supernatant was removed, and the cells were washed once with 3% BSA; after the cells were centrifuged at 350×g for 5 min, a supernatant was removed, and 1×PBS was added to resuspend the cells at 100 µL/well; and the resuspended cells were detected according to a standard operation procedure of a CytoFLEX flow cytometer.

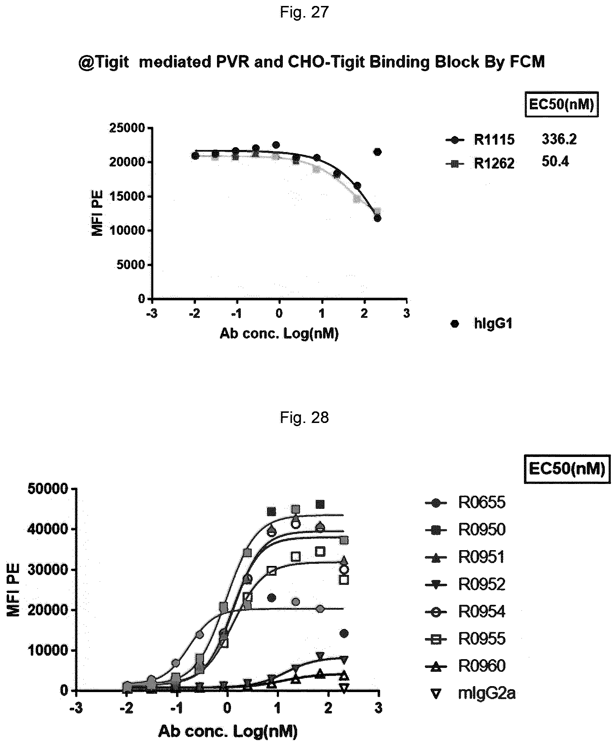

The detection result of R1262 was shown in FIG. 27 and showed that compared with the disulfide bond-unmodified IL2/IL2Rα complex 4 (R1115), the disulfide bond-modified IL2/IL2Rα complex 1 (R1262) had an equivalent blocking effect on a binding force of the ligand and the target region.

### Example 12 Binding activity of FiBody receptor and ligand complex (IL15/IL15R)

Antibody dilution: all molecules were diluted with an FACS buffer to an initial concentration 400 nM at a volume of 180 µl; 10 concentrations were obtained by 3 times of gradient dilution (60+120); counting and plate-spreading of cells: R0255-2(CHO-mTigit)/293T-IL15R-28 cells were centrifuged at 250 g for 5 min, a supernatant was removed, a cell density was adjusted to 2E+06 with an FACS buffer, and the cells were uniformly distributed into 96-well V-shaped plates at a 100 µL/tube; the diluted antibody was added into the cells at 100 µL/well and the cells were incubated at 2-8°C for 0.5 h; the 96-well plate was taken out and centrifuged at 250 g for 5 min, a supernatant was carefully removed, the FACS buffer was added at 200 µL/well, and the cells were centrifuged at 250 g for 5 min again, and a supernatant was carefully removed; a PE fluorescent secondary antibody (diluted at 1: 500) was prepared with the FACS buffer and added into a corresponding 96-well plate at 100 µL/well, and the cells were resuspended and incubated at 2-8°C for 30 min; the 96-well plate was taken out, the cells were centrifuged at 250 g for 5 min, the supernatant was carefully removed, the FACS buffer at 200 µL/well, the cells were centrifuged at 250 g again for 5 min, and the supernatant was carefully removed; and the cells were resuspended with 1xPBS at 100 µL/well and detected by FACS.

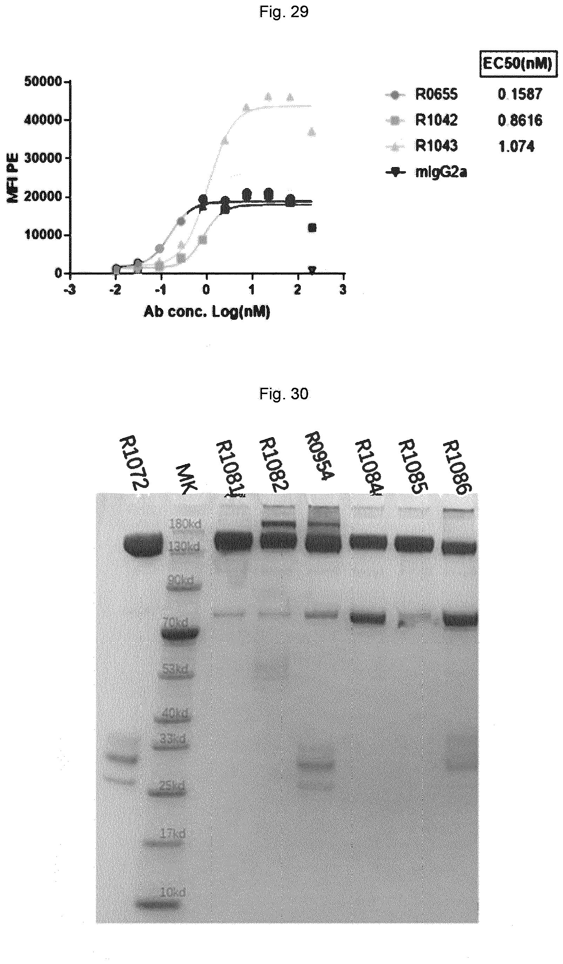

Results were shown in FIG. 28 and FIG 29: R0952 (IL15 and IL15RA rearranged) and R0960 (deactivated) molecules had a very low IL15 receptor complex binding activity; the IL15 activity of R0955 (deglycosylated) was decreased; and the activity of the R0953 and R0954 after covalent linking was similar to that of R0951, indicating that there was little effect on the structure.

The mismatched molecules R1042 and R1043 had an activity equivalent to R0951, indicating that no mismatching was generated and even a wrong Fv may also be expressed, wherein R0655 was IL15/IL15RFc fusion protein (see SEQ ID NO: 38).

### Example 13 Electrophoresis detection of disulfide bond-modified molecules

The disulfide bond-modified optimized samples (R1072, R1081, R1082, R0954, and R1084-R1086) in the example of the present disclosure were subjected to an SDS-PAGE electrophoresis detection.

The result was shown in FIG. 30. The R1072 molecule without disulfide-bond modification had a band with a molecular weight of 25 KD-35 KD, indicating that a free light chain exists; the ligand and receptor disulfide bond-modified molecules were R0954, R1085, and R1086, wherein the electrophoresis result of R0954 and R1086 showed that the non-covalent light chain still existed (a band existed between 25 KD and 35 KD), and R1085 was free of non-covalent light chain (a band did not exist between 25 KD and 35 KD), indicating that the disulfide-bond modification of R1085 was successful.

The light and heavy chain disulfide bond-modified molecules were R1081, R1082, and R1084. The electrophoresis result showed that no non-covalent light chain existed (no band existed between 25 KD and 35 KD), indicating that the disulfide-bond modification of R1081, R1082, and R1084 was successful.

The FiBody samples prepared in example 9 (disulfide bond-modified IL2/IL2Rα complexes 1 and 2, and IL2/IL2Rα complex 3 constructed in example 8) and R1115 in example 2 were subjected to an SDS-PAGE electrophoresis detection.

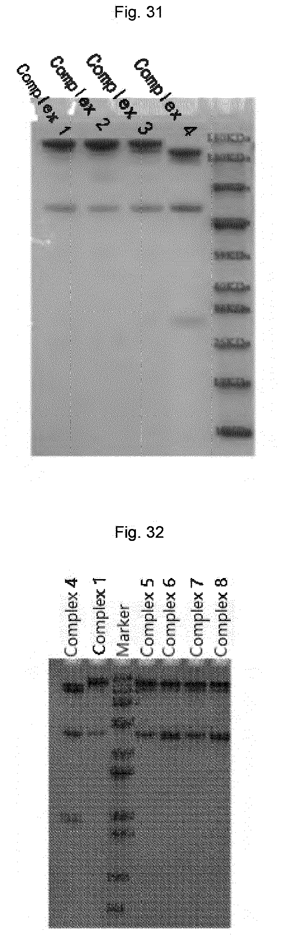

The detection result was shown in FIG. 31, wherein R1115 was a disulfide bond-unmodified molecule, and a band existed between a molecular weight of 25 KD and 35 KD, indicating that a free light chain existed. The disulfide bond-modified IL2/IL2Rα complexes 1 and 2 had no bands with a molecular weight of 25 KD-35 KD, which was the same as the IL2/IL2Rα complex 3, indicating that the free light chain was successfully removed, and the disulfide-bond modification was successful.

### Example 14 Effect of types of first and third amino acids extended from N terminus of amino acid sequence of IL2Rα mutant on formation of disulfide bond introduced after modification of IL2/IL2Rα complex

When the second antigen-binding moiety was indirectly linked to the N terminus of the IL2Rα mutant through a linker, the N terminus of the amino acid sequence of the IL2Rα mutant extended three amino acids. To further clarify an effect of types of first and third amino acids extended from the N terminus of the amino acid sequence of the IL2Rα mutant on formation of a disulfide bond introduced after modification of an IL2/IL2Rα complex, the following experiments were performed:
1. Effect of type of extended third amino acid on formation of disulfide bond introduced after modification of IL2/IL2Rα complex

The extended third amino acid was selected from any one of an aromatic amino acid (for example, phenylalanine), an amino acid with an uncharged R group (for example, serine), an amino acid with a positively charged R group (for example, lysine) or an amino acid with a negatively charged R group (for example, aspartic acid) to construct and prepare disulfide bond-modified IL2/IL2Rα complexes 5, 6, 7, and 8 (abbreviated to R1493, R1494, R1495, and R1496 successively), the complexes, together with a disulfide bond-modified IL2/IL2Rα complex 1 (the third amino acid extended from the N terminus of the amino acid sequence of the IL2Rα mutant in the IL2/IL2Rα complex 1 was glycine (non-polar fatty acid amino acid)) were used as experimental groups, and a disulfide bond-unmodified IL2/IL2Rα complex 4 was used as a control group.

The disulfide bond-modified IL2/IL2Rα complexes 5, 6, 7, and 8 had the same preparation process as the disulfide bond-modified IL2/IL2Rα complex 1 in example 8.

Sequence structures of the disulfide bond-modified IL2/IL2Rα complexes 5, 6, 7, and 8 were all as follows: TIGIT VH-IL2Rα mutant-Fc, TIGIT VL-IL2 mutant (SEQ ID NO: 78), PDL1 VH-hlgG1(SEQ ID NO: 79), and PDL1 VL-κ-Ig LC (SEQ ID NO: 80), wherein amino acid sequences of TIGIVH-IL2Rα mutant-Fc of the disulfide bond-modified IL2/IL2Rα complexes 5, 6, 7, and 8 were shown in SEQ ID NO: 85, SEQ ID NO: 86, SEQ ID NO: 87, and SEQ ID NO: 88 successively (wherein the three amino acids extended from the N terminus of the amino acid sequence of the IL2Rα mutant were shown in bold, and the extended third amino acid was underlined).

| **Molecule No.** | **R1493: TIGIT VH-IL2Rα mutant-Fc/TIGIT VL4L2 mutant/PD-L1 VH-hIgG1/PD-L1 VL-κ-Ig LC** |
|---|---|
| First polypeptide | @TIGIT **VH-IL2Rα** mutant-Fc (SEQ ID NO. 85) |
| | QVQLVQSGAEVKKPGSSVKVSCKASGYSFTSYWMNWVRQAPGQGLEWIGMIRPSDSETRLNQM FKDRVTITVDKSTSTAYMELSSLRSEDTAVYYCAGIHDYGHGAYWGQGTLVTVSSGGGGSGGGGS GGGGSGGGFCGELCDDDPPEIPHATFKAMAYKEGTMLNCECKRGFRRIKSGSLYMLCTGNSSHS SWDNQCQCTSSATRNTTKQVTPQPEEQKERKTTEMQSPMQPVDQASLPGHCREPPPWENEATE RIYHFVVGQMVYYQCVQGYRALHRGPAESVCKMTHGKTRWTQPQLICTGEPKSSDKTHTCPPCP APELLGGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEMTKN QVSLTCRVKGFYPSDIAVEWESNGQPENNYKTTPPVLKSDGSFFLASKLTVDKSRWQQGNVFSCS VMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | **R1494: TIGIT VH-IL2Rα mutant-Fc/TIGIT VL-IL2 mutant/PD-L1 VH-hIgG1/PD-L1 VL-κ-Ig LC** |
|---|---|
| First polypeptide | OTIGIT **VH-IL2Rα** mutant-Fc (SEQ ID NO. 86) |
| | QVQLVQSGAEVKKPGSSVKVSCKASGYSFTSYWMNWVRQAPGQGLEWIGMIRPSDSETRLNQM FKDRVTITVDKSTSTAYMELSSLRSEDTAVYYCAGIHDYGHGAYWGQGTLVTVSSGGGGSGGGGS GGGGSGGGSCGELCDDDPPEIPHATFKAMAYKEGTMLNCECKRGFRRIKSGSLYMLCTGNSSHS SWDNQCQCTSSATRNTTKQVTPQPEEQKERKTTEMQSPMQPVDQASLPGHCREPPPWENEATE RIYHFVVGQMVYYQCVQGYRALHRGPAESVCKMTHGKTRWTQPQLICTGEPKSSDKTHTCPPCP APELLGGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEMTKN QVSLTCRVKGFYPSDIAVEWESNGQPENNYKTTPPVLKSDGSFFLASKLTVDKSRWQQGNVFSCS VMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | **R1495: TIGIT VH-IL2Rα mutant-Fc/TIGIT VL-IL2 mutant/PD-L1 VH-hIgG1/PD-L1 VL-κ-Ig LC** |
|---|---|
| First polypeptide | @TIGIT VIH-IL2Rα mutant-Fc (SEQ ID NO. 87) |
| | QVQLVQSGAEVKKPGSSVKVSCKASGYSFTSYWMNWVRQAPGQGLEWIGMIRPSDSETRLNQM FKDRVTITVDKSTSTAYMELSSLRSEDTAVYYCAGIHDYGHAYWGQGTLVTVSSGGGGSGGGGS GGGGSGGGKCGELCDDDPPEIPHATFKAMAYKEGTMLNCECKRGFRRIKSGSLYMLCTGNSSHS SWDNQCQCTSSATRNTTKQVTPQPEEQKERKTTEMQSPMQPVDQASLPGHCREPPPWENEATE RIYHFVVGQMVYYQCVQGYRALHRGPAESVCKMTHGKTRWTQPQLICTGEPKSSDKTHTCPPCP APELLGGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEMTKN QVSLTCRVKGFYPSDIAVEWESNGQPENNYKTTPPVLKSDGSFFLASKLTVDKSRWQQGNVFSCS VMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | **RI496: TIGIT VH-IL2Rα mutant-Fc/TIGIT VL-IL2 mutant/PD-L1 VH-hIgG1/PD-L1 VL-κ-Ig LC** |
|---|---|
| First polypeptide | @TIGIT **VH-IL2Rα** mutant-Fc (SEQ ID NO. 88) |
| | QVQLVQSGAEVKKPGSSVKVSCKASGYSFTSYWMNWVRQAPGQGLEWIGMIRPSDSETRLNQM FKDRVTITVDKSTSTAYMELSSLRSEDTAVYYCAGIHDYGHGAYWGQGTLVTVSSGGGGSGGGGS GGGGSGGGDCGELCDDDPPEIPHATFKAMAYKEGTMLNCECKRGFRRIKSGSLYMLCTGNSSHS SWDNQCQCTSSATRNTTKQVTPQPEEQKERKTTEMQSPMQPVDQASLPGHCREPPPWENEATE RIYHFVVGQMVYYQCVQGYRALHRGPAESVCKMTHGKTRWTQPQLICTGEPKSSDKTHTCPPCP APELLGGPSVFLFPPKPKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPREPQVYTLPPSREEMTKN QVSLTCRVKGFYPSDIAVEWESNGQPENNYKTTPPVLKSDGSFFLASKLTVDKSRWQQGNVFSCS VMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

The obtained disulfide bond-modified IL2/IL2Rα complexes 5, 6, 7, and 8 and the complex 1 were subjected to an SDS-PAGE electrophoresis detection, and the disulfide bond-unmodified IL2/IL2Rα complex 4 was used as a control group to investigate the effect of the type of the third amino acid extended from the N terminus of the amino acid sequence of the IL2Rα mutant on formation of the disulfide bond introduced after the modification of the IL2/IL2Rα complex. The experimental result was shown in FIG. 32. It may be seen that compared with the control group, the free light chains were successfully removed from the disulfide bond-modified IL2/IL2Rα complexes 5, 6, 7, and 8, and the disulfide-bond modification was successful, indicating that the extended third amino acid selected from the non-polar fatty acid amino acid, the aromatic amino acid, the amino acid with an uncharged R group, the amino acid with a positively charged R group or the amino acid with a negatively charged R group all did not affect the formation of the disulfide bond introduced after the modification of the IL2/IL2Rα complex.

### 2. Effect of combined types of extended first and third amino acids on formation of disulfide bond after modification of IL2/IL2Rα complex

The extended first and third amino acids were selected from any combination of a non-polar fatty acid amino acid (for example, glycine) an aromatic amino acid (for example, phenylalanine), an amino acid with an uncharged R group (for example, serine), an amino acid with a positively charged R group (for example, lysine) or an amino acid with a negatively charged R group (for example, aspartic acid) to construct and prepare disulfide bond-modified IL2/IL2Rα complexes 9, 10, 11, 12, and 13 (abbreviated to R1662, R1663, R1664, R1665, and R1666 successively), the complexes, together with the disulfide bond-modified IL2/IL2Rα complex 1 were used as experimental groups, and the disulfide bond-unmodified IL2/IL2Rα complex 4 was used as a control group.

The disulfide bond-modified IL2/IL2Rα complexes 9, 10, 11, 12, and 13 had the same preparation process as the disulfide bond-modified IL2/IL2Rα complex 1 in example 8.

Sequence structures of the disulfide bond-modified IL2/IL2Rα complexes 9, 10, 11, 12, and 13 were all as follows: TIGIT VH-IL2Rα mutant-Fc, TIGIT VL-IL2 mutant (SEQ ID NO: 78), PDL1 VH-hlgG1(SEQ ID NO: 79), and PDL1 VL-κ-Ig LC (SEQ ID NO: 80), wherein amino acid sequences of TIGIVH-IL2Rα mutant-Fc of the disulfide bond-modified IL2/IL2Rα complexes 9, 10, 11, 12, and 13 were shown in SEQ ID NO: 89, SEQ ID NO: 90, SEQ ID NO: 91, SEQ ID NO: 92, and SEQ ID NO: 93 successively (wherein the three amino acids extending from the N terminus of the amino acid sequence of the IL2Rα mutant were shown in bold, and the extended first and third amino acids were underlined).

| **Molecule No.** | **R1662: TIGIT VH-IL2Rα mutant-Fc/TIGIT VL-IL2 mutant/PD-L1 VH-hIgG1/PD-L1 VL-κ-Ig LC** |
|---|---|
| First polypeptide | @TIGIT VH-IL2Rα mutant-Fc (SEQ ID NO. 89) |
| | QVQLVQSGAEVKKPGSSVKVSCKASGYSFTSYWMNWVRQAPGQGLEWIGMIRPSDSETRL NQMFKDRVTITVDKSTSTAYMELSSLRSEDTAVYYCAGIHDYGHGAYWGQGTLVTVSSGGG GSGGGGSGGGGSGGGFCSELCDDDPPEIPHATFKAMAYKEGTMLNCECKRGFRRIKSGSL YMLCTGNSSHSSWDNQCQCTSSATRNTTKQVTPQPEEQKERKTTEMQSPMQPVDQASLP GHCREPPPWENEATERIYHFVVGQMVYYQCVQGYRALHRGPAESVCKMTHGKTRWTQPQ LICTGEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | **R1663: TIGIT VH-IL2Rα mutant-Fc/TIGIT VL-IL2 mutant/PD-L1 VH-hIgG1/PD-L1 VL-κ-Ig** LC |
|---|---|
| First polypeptide | @TIGIT **VH-IL2Rα** mutant-Fc (SEQ ID NO. 90) |
| | QVQLVQSGAEVKKPGSSVKVSCKASGYSFTSYWMNWVRQAPGQGLEWIGMIRPSDSETRL NQMFKDRVTITVDKSTSTAYMELSSLRSEDTAVYYCAGIHDYGHGAYWGQGTLVTVSSGGG GSGGGGSGGGGSGGGKCDELCDDDPPEIPHATFKAMAYKEGTMLNCECKRGFRRIKSGSL YMLCTGNSSHSSWDNQCQCTSSATRNTTKQVTPQPEEQKERKTTEMQSPMQPVDQASLP GHCREPPPWENEATERIYHFVVGQMVYYQCVQGYRALHRGPAESVCKMTHGKTRWTQPQ LICTGEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule** No. | **R1664: TIGIT VH-IL2Rα mutant-Fc/TIGIT VL-IL2 mutant/PD-L1 VH-hIgG1/PD-L1 VL-κ-Ig LC** |
|---|---|
| First polypeptide | @TIGIT VH-IL2Rα mutant (SEQ ID NO. 91) |
| | QVQLVQSGAEVKKPGSSVKVSCKASGYSFTSYWMNWVRQAPGQGLEWIGMIRPSDSETRL NQMFKDRVTITVDKSTSTAYMELSSLRSEDTAVYYCAGIHDYGHGAYWGQGTLVTVSSGGG GSGGGGSGGGGSGGGSCDELCDDDPPEIPHATFKAMAYKEGTMLNCECKRGFRRIKSGSL YMLCTGNSSHSSWDNQCQCTSSATRNTTKQVTPQPEEQKERKTTEMQSPMQPVDQASLP GHCREPPPWENEATERIYHFVVGQMVYYQCVQGYRALHRGPAESVCKMTHGKTRWTQPQ LICTGEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | R1665: TIGIT VH-IL2Rα mutant-Fc/TIGIT VL-IL2 mutant/PD-L1 VH-hIgG1/PD-L1 VL-κ-Ig **LC** |
|---|---|
| First polypeptide | @TIGIT VH-IL2Rα mutant (SEQ ID NO. 92) |
| | QVQLVQSGAEVKKPGSSVKVSCKASGYSFTSYWMNWVRQAPGQGLEWIGMIRPSDSETRL NQMFKDRVTITVDKSTSTAYMELSSLRSEDTAVYYCAGIHDYGHGAYWGQGTLVTVSSGGG GSGGGGSGGGGSGGGDCFELCDDDPPEIPHATFKAMAYKEGTMLNCECKRGFRRIKSGSL YMLCTGNSSHSSWDNQCQCTSSATRNTTKQVTPQPEEQKERKTTEMQSPMQPVDQASLP GHCREPPPWENEATERIYHFVVGQMVYYQCVQGYRALHRGPAESVCKMTHGKTRWTQPQ LICTGEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

| **Molecule No.** | **R1666: TIGIT VH-IL2Rα mutant-Fc/TIGIT VL-IL2 mutant/PD-L1 VH-hIgG1/PD-L1 VL-κ-Ig LC** |
|---|---|
| First polypeptide | @TIGIT VH-IL2Rα mutant-Fc (SEQ ID NO. 93) |
| | QVQLVQSGAEVKKPGSSVKVSCKASGYSFTSYWMNWVRQAPGQGLEWIGMIRPSDSETRL NQMFKDRVTITVDKSTSTAYMELSSLRSEDTAVYYCAGIHDYGHGAYWGQGTLVTVSSGGG GSGGGGSGGGGSGGGSCKELCDDDPPEIPHATFKAMAYKEGTMLNCECKRGFRRIKSGSL YMLCTGNSSHSSWDNQCQCTSSATRNTTKQVTPQPEEQKERKTTEMQSPMQPVDQASLP GHCREPPPWENEATERIYHFVVGQMVYYQCVQGYRALHRGPAESVCKMTHGKTRWTQPQ LICTGEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPSREEMTKNQVSLTCRVKGFYPSDIAVEWESNGQPENNYKTTPP VLKSDGSFFLASKLTVDKSRWQQGNVFSCSVMHEALHNHYTQKSLSLSPGK |
| Second polypeptide | @TIGIT VL-IL2 mutant (SEQ ID NO. 78) |
| Third polypeptide | @PD-L1 VH-hIgG1 (SEQ ID NO. 79) |
| Fourth polypeptide | @PD-L1 VL-κ-Ig LC (SEQ ID NO. 80) |

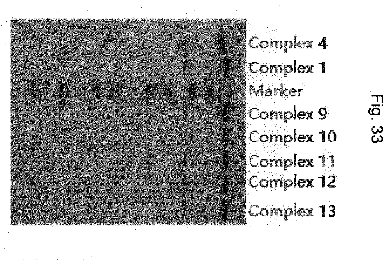

The obtained disulfide bond-modified IL2/IL2Rα complexes 9, 10, 11, 12, and 13 and the complex 1 were subjected to an SDS-PAGE electrophoresis detection, and the disulfide bond-unmodified IL2/IL2Rα complex 4 was used as a control group to investigate the effect of the combined types of the first and third amino acids extended from the N terminus of the amino acid sequence of the IL2Rα mutant on formation of the disulfide bond introduced after the modification of the IL2/IL2Rα complex. The experimental result was shown in FIG. 33. It may be seen that compared with the control group, the free light chains were successfully removed from the disulfide bond-modified IL2/IL2Rα complexes 9, 10, 11, 12, and 13, and the disulfide-bond modification was successful, indicating that the extended first and third amino acids selected from any combination of the non-polar fatty acid amino acid, the aromatic amino acid, the amino acid with an uncharged R group, the amino acid with a positively charged R group or the amino acid with a negatively charged R group all did not affect the formation of the disulfide bond introduced after the modification of the IL2/IL2Rα complex.

The technical characteristics of the above examples may be combined arbitrarily. For a concise description, all possible combinations of all technical characteristics of the examples may not be described; however, these combinations of the technical characteristics should be construed as disclosed in the description as long as no contradiction occurs.

The above embodiments only express several embodiments of the present disclosure and the description is relatively specific and in detail, but they should not therefore be construed as limiting the scope of the present disclosure. It should be noted that a person of ordinary skill in the art may further make several variations and improvements without departing from the conception of the present disclosure. These variations and improvements all fall within the protection scope of the present disclosure. Therefore, the scope of protection of the patent of the present disclosure shall be in accordance with the appended claims.
Sequence 36: @PD-L1: VH (SEQ ID NO: 36) 
EVQLLESGGGLVQPGGSLRLSCAASGFTFSSYIMMWVRQAPGKGLEWVSSIYPSGGITFYAD
TVKGRFTISRDNSKNTLYLQMNSLRAEDTAVYYCARIKLGTVTTVDYWGQGTLVTVSS.
Sequence 37: @PD-L1: VL (SEQ ID NO: 37) 
QSALTQPASVSGSPGQSITISCTGTSSDVGGYNYVSWYQQHPGKAPKLMIYDVSNRPSGVSN
RFSGSKSGNTASLTISGLQAEDEADYYCSSYTSSSTRVFGTGTKVTVL.
Amino acid sequence of R0655 (SEQ ID NO: 38) 
EPRGPTIKPCPPCKCPAPNLLGGPSVFIFPPKIKDVLMISLSPIVTCVVVDVSEDDPDVQISWFV
NNVEVHTAQTQTHREDYNSTLRVVSALPIQHQDWMSGKEFKCKVNNKDLPAPIERTISKPKGSVR
APQVYVLPPPEEEMTKKQVTLTCMVTDFMPEDIYVEWTNNGKTELNYKNTEPVLDSDGSYFMYS
KLRVEKKNWVERNSYSCSVVHEGLHNHHTTKSFSRTPGAGGGGSGGGGSGGGGSGGGGSGIT
CPPPMSVEHADIWVKSYSLYSRERYICNSGFKRKAGTSSLTECVLNKATNVAHWTTPSLKCIRDPA
LVHQRPAPPSTVTTAGVTPQPESLSPSGKEPAASSPSSNNTAATTAAIVPGSQLMPSKSPSTGTTEI
SSHESSHGTPSQTTAKNWELTASASHQPPGVYPQGHSDTTSGGSGGGGSGGGSGGGGSLQNW
VNVISDLKKIEDLIQSMHIDATLYTESDVHPSCKVTAMKCFLLELQVISLESGDASIHDTVENLIILAND
SLSSNGNVTESGCKECEELEEKNIKEFLQSFVHIVQMFINTS.
Amino acid sequence of R0968PD-L1 VH (SEQ ID NO: 71) 
QVQLQESGPGLVKPSETLSLTCAVYGDSITSGYWNWIRKPPGKCLEYIGYISYTGSTYQNPSLK
SRITMSRDTSKNQYYLKLSSVTAADTAVYYCARSRAWIRTYFDYWGQGTLVTVSS.
Amino acid sequence of R0968PD-L1 VL (SEQ ID NO: 72) 
DIQMTQSPSSLSASVGDRVTITCSVSSSISSSNLHWYQQKPGKAPKPWIYGTSNLASGVPSRF
SGSGSGTDYTLTISSLQPEDFATYYCQQWSSYPLTFGCGTKLEIK.
Amino acid sequence of R0774TIGIT VH (SEQ ID NO: 73) 
QVQLVQSGAEVKKPGSSVKVSCKASGYSFTSYWMNWVRQAPGQGLEWIGMIRPSDSETRLN
QMFKDRVTITVDKSTSTAYMELSSLRSEDTAVYYCAGIHDYGHGAYWGQGTLVTVSS.
Amino acid sequence of R0774TIGIT VL (SEQ ID NO: 74) 
DIQMTQSPSSLSASVGDRVTITCRASENIYSNLAWYQQKPGKSPKLLVYAASHLPDGVPSRFS
GSGSGTDYSLTISSLQPEDFATYYCQHFWGTPRTFGQGTKLEIK.
Amino acid sequence of R0919PD-L1 VH (SEQ ID NO: 75) 
EVQLQESGPGLVKPSETLSLTCAVYGDSITSGYWNWIRKPPGKGLEYMGYISYTGSTYQNPSL
KSRITFSRDTSKNQYYLKLSSVTAADTATYYCARSRAWIRTYFDYWGQGTLVTVSS.
Amino acid sequence of R0919PD-L1 VL (SEQ ID NO: 76)

EIVLTQSPDFQSVTPKEKVTITCSVSSSISSSNLHWYQQKPDQSPKLLIYGTSNLASGVPSRFS
GSGSGTDFTLTINSLEAEDAATYYCQQWSSYPLTFGQGTKLEIK.

## Claims

1. A bispecific fusion polypeptide, comprising a first antigen-binding moiety which comprises:
a first polypeptide comprising from a first heavy chain variable domain VH1 of a first antibody from the N terminus to the C terminus, which is operably linked to a first conjugate fragment; and
a second polypeptide comprising a first light chain variable domain VL1 of the first antibody from the N terminus to the C terminus, which is operably linked to a second conjugate fragment, wherein the first conjugate fragment and the second conjugate fragment are capable of specifically binding; and
the first conjugate fragment is a receptor and the second conjugate fragment is a ligand; or the first conjugate fragment is a ligand and the second conjugate fragment is a receptor.

2. The bispecific fusion polypeptide according to claim 1, further comprising a second antigen-binding moiety, wherein the second antigen-binding moiety is different from the first antigen-binding moiety, and
the second antigen-binding moiety comprises:
a third polypeptide comprising a second heavy chain variable domain VH2 of a second antibody from the N terminus to the C terminus, which is operably linked to a third conjugate fragment; and
a fourth polypeptide comprising a second light chain variable domain VL2 of the second antibody from the N terminus to the C terminus, which is operably linked to a fourth conjugate fragment, wherein the third conjugate fragment and the fourth conjugate fragment are capable of specifically binding; the third conjugate fragment is a receptor and the fourth conjugate fragment is a ligand; or the third conjugate fragment is a ligand and the fourth conjugate fragment is a receptor; and
the third conjugate fragment and/or the fourth conjugate fragment, and the first conjugate fragment and/or the second conjugate fragment are selected from different receptors and ligands.

3. The bispecific fusion polypeptide according to claim 1, further comprising a second antigen-binding moiety, wherein the second antigen-binding moiety is different from the first antigen-binding moiety, and
the second antigen-binding moiety comprises:
a third polypeptide comprising from the N terminus to the C terminus a second heavy chain variable domain VH2 of a second antibody from the N terminus to the C terminus, which is operably linked to an antibody heavy chain constant region CH1; and
a fourth polypeptide comprising from the N terminus to the C terminus a second light chain variable domain VL2 of the second antibody from the N terminus to the C terminus, which is operably linked to an antibody light chain constant region CL.

4. The bispecific fusion polypeptide according to any one of the preceding claims, wherein at least one non-natural interchain bond is comprised between the receptor and the ligand, and the non-natural interchain bond is capable of enhancing a specific binding force between the receptor and the ligand.

5. The bispecific fusion polypeptide according to claim 4, wherein the non-natural interchain bond is formed between a first mutant residue comprised by the receptor and a second mutant residue comprised by the ligand.

6. The bispecific fusion polypeptide according to claim 4, wherein the non-natural interchain bond is formed between a first mutant residue comprised by the first heavy chain variable domain VH1 and a second mutant residue comprised by the first light chain variable domain VL1.

7. The bispecific fusion polypeptide according to claim 5 or 6, wherein at least one of the first mutant residue and the second mutant residue is a cysteine residue.

8. The bispecific fusion polypeptide according to claim 7, wherein the non-natural interchain bond is a disulfide bond.

9. The bispecific fusion polypeptide according to any one of the preceding claims, wherein at least one natural glycosylation site is not present in the receptor and/or the ligand.

10. The bispecific fusion polypeptide according to any one of the preceding claims, wherein the receptor and the ligand thereof are selected from an interleukin and a receptor thereof.

11. The bispecific fusion polypeptide according to claim 10, wherein the interleukin and the receptor thereof have a lift-type steric conformantion and are selected from IL15/IL15R, IL2/IL2R, IL4/IL-4Rα+Rγ, IL-6/IL-6R, IL-11/IL-11R, IL-13/IL-13R1, IL-20/IL20Rα+IL20Rβ, and/or IL24/IL20Rα+IL20Rβ.

12. The bispecific fusion polypeptide according to claim 10, wherein the interleukin and the receptor thereof have a pincer-type steric conformantion and are selected from IL7/IL7R, IL21/IL21R, and IL23A/IL12B.

13. The bispecific fusion polypeptide according to claim 11, wherein the ligand and the receptor are selected from IL15 and IL15Rα.

14. The bispecific fusion polypeptide according to claim 13, wherein the E at position 90 of the IL15 is mutated into C and the P at position 67 of the IL15Rα is mutated into C.

15. The bispecific fusion polypeptide according to claim 13, wherein a non-natural disulfide bond is present between the first heavy chain variable domain VH1 and the first light chain variable domain VL1, and preferably, the first heavy chain variable domain VH1 and the first light chain variable domain VL1 comprise any one of the following mutation combinations:
| Combination | VH | VL |
|---|---|---|
| 1 | 37C | 95C |
| 2 | 44C | 100C |
| 3 | 44C | 101C |
| 4 | 44C | 105C |
| 5 | 45C | 87C |
| 6 | 45C | 98C |
| 7 | 100C | 50C |
| 8 | 100bC | 49C |
| 9 | 98C | 46C |
| 10 | 101C | 46C |
| 11 | 105C | 43C |
| 12 | 106C | 57C |
| 13 | 108C | 43C |

16. The bispecific fusion polypeptide according to any one of claims 13-15, wherein the D at position 61 of the IL15 is mutated into N, the E at position 64 is mutated into Q, and/or the N at position 65 is mutated into D.

17. The bispecific fusion polypeptide according to any one of claims 13-16, wherein at least one N-glycosylation site of the IL15 is not present; preferably, the N-glycosylation site is selected from N71, N79, and/or N112; and preferably, the IL15 comprises the following amino acid mutations: N71Q, N79Q, and/or N112Q.

18. The bispecific fusion polypeptide according to any one of claims 13-17, wherein at least one O-glycosylation site of the IL15Rα is not present; preferably, the O-glycosylation site is selected from T2, T81, and/or T86; and preferably, the IL15Rα comprises the following amino acid mutations: T2A, T81A, and/or T86A.

19. The bispecific fusion polypeptide according to claim 11, wherein the ligand and the receptor are selected from IL2 and IL2Rα.

20. The bispecific fusion polypeptide according to claim 19, wherein S at position 75 of the IL2 is mutated into C and the N terminus of the IL2Rα is extended by two or three amino acids;
when the N terminus of the IL2Rα is extended by two amino acids, the second extended amino acid is cysteine, and the first extended amino acid is any one of a non-polar fatty acid amino acid, an aromatic amino acid, an amino acid with an uncharged R group, an amino acid with a positively charged R group or an amino acid with a negatively charged R group; and
when the N terminus of the IL2Rα is extended by three amino acids, the second extended amino acid is cysteine, and the first and third extended amino acids are any one of a non-polar fatty acid amino acid, an aromatic amino acid, an amino acid with an uncharged R group, an amino acid with a positively charged R group or an amino acid with a negatively charged R group.

21. The bispecific fusion polypeptide according to any one of the preceding claims, comprising an antibody Fc constant region.

22. The bispecific fusion polypeptide according to claim 21, wherein the antibody Fc constant region is a heterodimer.

23. The bispecific fusion polypeptide according to claim 21 or 22, wherein the antibody Fc constant region is a heterodimer associated by KiH, a hydrophobic interaction, an electrostatic interaction, a hydrophilic interaction, and/or an increased flexibility.

24. The bispecific fusion polypeptide according to any one of claim 21 or 22, wherein the antibody Fc constant region comprises CH2, CH3, and optionally CH4, and the CH2, the CH3, and/or the optionally CH4 are replaced by the receptor and the ligand thereof.

25. The bispecific fusion polypeptide according to any one of the above, wherein the first antigen-binding moiety and the second antigen-binding moiety bind to different antigens or bind to different epitopes of the same antigen;
preferably, the first antigen-binding moiety targets an immune cell and the second antigen-binding moiety targets a tumor cell;
preferably, the first antigen-binding moiety and the second antigen-binding moiety both target a tumor cell; and
preferably, the first antigen-binding moiety and the second antigen-binding moiety both target an immune cell.

26. An isolated nucleic acid, encoding the bispecific fusion polypeptide according to any one of claims 1-25.

27. A vector comprising the nucleic acid according to claim 26.

28. A host cell comprising the nucleic acid according to claim 26 or the vector according to claim 27.

29. A pharmaceutical composition, comprising the bispecific fusion polypeptide according to any one of claims 1-25, and a pharmaceutically acceptable carrier, an excipient, or a stabilizer.

30. Use of the bispecific fusion polypeptide according to any one of claims 1-25 in the preparation of drugs for the treatment of diseases.
